# EUROPÄISCHE PATENTSCHRIFT

(11) **EP 3 080 252 B1**
(45) Veröffentlichungstag und Bekanntmachung des Hinweises auf die Patenterteilung: **07.07.2021**
(21) Anmeldenummer: 14830951.1
(22) Anmeldetag: 10.12.2014
(51) Int. Cl.: C12N 9/04, C12P 7/42, C12P 7/64, C12N 1/19, C12R 1/73

(54) **HEFESTÄMME UND VERFAHREN ZUR PRODUKTION VON -HYDROXYFETTSÄUREN UND DICARBONSÄUREN**
YEAST STRAINS AND METHODS FOR THE PRODUCTION OF -HYDROXY FATTY ACIDS AND DICARBOXYLIC ACIDS
SOUCHES DE LEVURE ET PROCÉDÉ DE PRODUCTION D'ACIDES GRAS -HYDROXYLÉS ET D'ACIDES DICARBOXYLIQUES

(30) Priorität: 12.12.2013 DE 102013022175
(43) Veröffentlichungstag der Anmeldung: 19.10.2016
(60) Teilanmeldung: 21162092.7
(73) Patentinhaber: Provivi, Inc., Santa Monica, California 90404 (US)
(72) Erfinder: GATTER, Michael, 01129 Dresden (DE); MATTHÄUS, Falk, 01187 Dresden (DE); BARTH, Gerold, 01189 Dresden (DE)
(74) Vertreter: Appleyard Lees IP LLP
(86) Internationale Anmeldenummer: PCT/EP2014/077237
(87) Internationale Veröffentlichungsnummer: WO 2015/086684

(56) Entgegenhaltungen:
- WO-A1-2006/064131
- WO-A2-2011/008232
- US-B2- 7 238 482
- KEMP G D ET AL: "LIGHT SENSITIVITY OF THE N ALKANE-INDUCED FATTY ALCOHOL OXIDASE FROM CANDIDA-TROPICALIS AND YARROWIA-LIPOLYTICA", APPLIED MICROBIOLOGY AND BIOTECHNOLOGY, Bd. 32, Nr. 4, 1990, Seiten 461-464, XP002737635, ISSN: 0175-7598
- GATTER MICHAEL ET AL: "A newly identified fatty alcohol oxidase gene is mainly responsible for the oxidation of long-chain omega-hydroxy fatty acids in Yarrowia lipolytica", FEMS YEAST RESEARCH, Bd. 14, Nr. 6, September 2014 (2014-09), Seiten 858-872, XP8175574,
- None

### SEQUENCE LISTING

<110> Technische Universität Dresden
<120> Hefestämme und Verfahren zur Produktion von Omega-Hydroxyfettsäuren und Dicarbonsäuren
<130> 00017P0263DEWO
<140> DE 10 2013 022 175.7
   <141> 2013-12-12
<160> 101
<170> PatentIn version 3.5
<210> 1
   <211> 2974
   <212> DNA
   <213> Artificial Sequence
<220>
   <223> pJET1.2/blunt (blunt end cloning verctor)
<400> 1
   atcttgctga aaaactcgag ccatccggaa gatctggcgg ccgctctccc tatagtgagt      60
   cgtattacgc cggatggata tggtgttcag gcacaagtgt taaagcagtt gattttattc     120
   actatgatga aaaaaacaat gaatggaacc tgctccaagt taaaaataga gataataccg     180
   aaaactcatc gagtagtaag attagagata atacaacaat aaaaaaatgg tttagaactt     240
   actcacagcg tgatgctact aattgggaca attttccaga tgaagtatca tctaagaatt     300
   taaatgaaga agacttcaga gcttttgtta aaaattattt ggcaaaaata atataattcg     360
   gctgcagggg cggcctcgtg atacgcctat ttttataggt taatgtcatg ataataatgg     420
   tttcttagac gtcaggtggc acttttcggg gaaatgtgcg cggaacccct atttgtttat     480
   ttttctaaat acattcaaat atgtatccgc tcatgagaca ataaccctga taaatgcttc     540
   aataatattg aaaaaggaag agtatgagta ttcaacattt ccgtgtcgcc cttattccct     600
   tttttgcggc attttgcctt cctgtttttg ctcacccaga aacgctggtg aaagtaaaag     660
   atgctgaaga tcagttgggt gcacgagtgg gttacatcga actggatctc aacagcggta     720
   agatccttga gagttttcgc cccgaagaac gttttccaat gatgagcact tttaaagttc     780
   tgctatgtgg cgcggtatta tcccgtattg acgccgggca agagcaactc ggtcgccgca     840
   tacactattc tcagaatgac ttggttgagt actcaccagt cacagaaaag catcttacgg     900
   atggcatgac agtaagagaa ttatgcagtg ctgccataac catgagtgat aacactgcgg     960
   ccaacttact tctgacaacg atcggaggac cgaaggagct aaccgctttt ttgcacaaca    1020
   tgggggatca tgtaactcgc cttgatcgtt gggaaccgga gctgaatgaa gccataccaa    1080
   acgacgagcg tgacaccacg atgcctgtag caatggcaac aacgttgcgc aaactattaa    1140
   ctggcgaact acttactcta gcttcccggc aacaattaat agactggatg gaggcggata    1200
   aagttgcagg accacttctg cgctcggccc ttccggctgg ctggtttatt gctgataaat    1260
   ctggagccgg tgagcgtggg tctcgcggta tcattgcagc actggggcca gatggtaagc    1320
   cctcccgtat cgtagttatc tacacgacgg ggagtcaggc aactatggat gaacgaaata    1380
   gacagatcgc tgagataggt gcctcactga ttaagcattg gtaactgtca gaccaagttt    1440
   actcatatat actttagatt gatttaaaac ttcattttta atttaaaagg atctaggtga    1500
   agatcctttt tgataatctc atgaccaaaa tcccttaacg tgagttttcg ttccactgag    1560
   cgtcagaccc cgtagaaaag atcaaaggat cttcttgaga tccttttttt ctgcgcgtaa    1620
   tctgctgctt gcaaacaaaa aaaccaccgc taccagcggt ggtttgtttg ccggatcaag    1680
   agctaccaac tctttttccg aaggtaactg gcttcagcag agcgcagata ccaaatactg    1740
   tccttctagt gtagccgtag ttaggccacc acttcaagaa ctctgtagca ccgcctacat    1800
   acctcgctct gctaatcctg ttaccagtgg ctgctgccag tggcgataag tcgtgtctta    1860
   ccgggttgga ctcaagacga tagttaccgg ataaggcgca gcggtcgggc tgaacggggg    1920
   gttcgtgcac acagcccagc ttggagcgaa cgacctacac cgaactgaga tacctacagc    1980
   gtgagctatg agaaagcgcc acgcttcccg aagggagaaa ggcggacagg tatccggtaa    2040
   gcggcagggt cggaacagga gagcgcacga gggagcttcc agggggaaac gcctggtatc    2100
   tttatagtcc tgtcgggttt cgccacctct gacttgagcg tcgatttttg tgatgctcgt    2160
   caggggggcg gagcctatgg aaaaacgcca gcaacgcggc ctttttacgg ttcctggcct    2220
   tttgctggcc ttttgctcac atgttctttc ctgcgttatc ccctgattct gtggataacc    2280
   gtattaccgc ctttgagtga gctgataccg ctcgccgcag ccgaacgacc gagcgcagcg    2340
   agtcagtgag cgaggaagcg gaagagcgcc caatacgcaa accgcctctc cccgcgcgtt    2400
   ggccgattca ttaatgcagc tggcacgaca ggtttcccga ctggaaagca attggcagtg    2460
   agcgcaacgc aattaatgtg agttagctca ctcattaggc accccaggct ttacacttta    2520
   tgcttccggc tcgtataatg tgtggaatta tgagcggata ataatttcac acaggaggtt    2580
   taaactttaa acatgtcaaa agagacgtct tttgttaaga atgctgagga acttgcaaag    2640
   caaaaaatgg atgctattaa ccctgaactt tcttcaaaat ttaaattttt aataaaattc    2700
   ctgtctcagt ttcctgaagc ttgctctaaa cctcgttcaa aaaaaatgca gaataaagtt    2760
   ggtcaagagg aacatattga atatttagct cgtagttttc atgagagtcg attgccaaga    2820
   aaacccacgc cacctacaac ggttcctgat gaggtggtta gcatagttct taatataagt    2880
   tttaatatac agcctgaaaa tcttgagaga ataaaagaag aacatcgatt ttccatggca    2940
   gctgagaata ttgtaggaga tcttctagaa agat                                2974
<210> 2
   <211> 2725
   <212> DNA
   <213> Artificial Sequence
<220>
   <223> pUCBM21 (cloning vector)
<400> 2
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac catgattacg ccaagcttcc 240
   atgggatatc gcatgcctgc agagctctag agtcgacggg cccggtaccg cggccgcact 300
   taagttacgc gtggatcccc gggaattcac tggccgtcgt tttacaacgt cgtgactggg 360
   aaaaccctgg cgttacccaa cttaatcgcc ttgcagcaca tccccctttc gccagctggc 420
   gtaatagcga agaggcccgc accgatcgcc cttcccaaca gttgcgcagc ctgaatggcg 480
   aatggcgcct gatgcggtat tttctcctta cgcatctgtg cggtatttca caccgcatat 540
   ggtgcactct cagtacaatc tgctctgatg ccgcatagtt aagccagccc cgacacccgc 600
   caacacccgc tgacgcgccc tgacgggctt gtctgctccc ggcatccgct tacagacaag 660
   ctgtgaccgt ctccgggagc tgcatgtgtc agaggttttc accgtcatca ccgaaacgcg 720
   cgagacgaaa gggcctcgtg atacgcctat ttttataggt taatgtcatg ataataatgg 780
   tttcttagac gtcaggtggc acttttcggg gaaatgtgcg cggaacccct atttgtttat 840
   ttttctaaat acattcaaat atgtatccgc tcatgagaca ataaccctga taaatgcttc 900
   aataatattg aaaaaggaag agtatgagta ttcaacattt ccgtgtcgcc cttattccct 960
   tttttgcggc attttgcctt cctgtttttg ctcacccaga aacgctggtg aaagtaaaag 1020
   atgctgaaga tcagttgggt gcacgagtgg gttacatcga actggatctc aacagcggta 1080
   agatccttga gagttttcgc cccgaagaac gttttccaat gatgagcact tttaaagttc 1140
   tgctatgtgg cgcggtatta tcccgtattg acgccgggca agagcaactc ggtcgccgca 1200
   tacactattc tcagaatgac ttggttgagt actcaccagt cacagaaaag catcttacgg 1260
   atggcatgac agtaagagaa ttatgcagtg ctgccataac catgagtgat aacactgcgg 1320
   ccaacttact tctgacaacg atcggaggac cgaaggagct aaccgctttt ttgcacaaca 1380
   tgggggatca tgtaactcgc cttgatcgtt gggaaccgga gctgaatgaa gccataccaa 1440
   acgacgagcg tgacaccacg atgcctgtag caatggcaac aacgttgcgc aaactattaa 1500
   ctggcgaact acttactcta gcttcccggc aacaattaat agactggatg gaggcggata 1560
   aagttgcagg accacttctg cgctcggccc ttccggctgg ctggtttatt gctgataaat 1620
   ctggagcccg tgagcgtggg tctcgcggta tcattgcagc actggggcca gatggtaagc 1680
   cctcccgtat cgtagttatc tacacgacgg ggagtcaggc aactatggat gaacgaaata 1740
   gacagatcgc tgagataggt gcctcactga ttaagcattg gtaactgtca gaccaagttt 1800
   actcatatat actttagatt gatttaaaac ttcattttta atttaaaagg atctaggtga 1860
   agatcctttt tgataatctc atgaccaaaa tcccttaacg tgagttttcg ttccactgag 1920
   cgtcagaccc cgtagaaaag atcaaaggat cttcttgaga tccttttttt ctgcgcgtaa 1980
   tctgctgctt gcaaacaaaa aaaccaccgc taccagcggt ggtttgtttg ccggatcaag 2040
   agctaccaac tctttttccg aaggtaactg gcttcagcag agcgcagata ccaaatactg 2100
   tccttctagt gtagccgtag ttaggccacc acttcaagaa ctctgtagca ccgcctacat 2160
   acctcgctct gctaatcctg ttaccagtgg ctgctgccag tggcgataag tcgtgtctta 2220
   ccgggttgga ctcaagacga tagttaccgg ataaggcgca gcggtcgggc tgaacggggg 2280
   gttcgtgcac acagcccagc ttggagcgaa cgacctacac cgaactgaga tacctacagc 2340
   gtgagctatg agaaagcgcc acgcttcccg aagggagaaa ggcggacagg tatccggtaa 2400
   gcggcagggt cggaacagga gagcgcacga gggagcttcc agggggaaac gcctggtatc 2460
   tttatagtcc tgtcgggttt cgccacctct gacttgagcg tcgatttttg tgatgctcgt 2520
   caggggggcg gagcctatgg aaaaacgcca gcaacgcggc ctttttacgg ttcctggcct 2580
   tttgctggcc ttttgctcac atgttctttc ctgcgttatc ccctgattct gtggataacc 2640
   gtattaccgc ctttgagtga gctgataccg ctcgccgcag ccgaacgacc gagcgcagcg 2700
   agtcagtgag cgaggaagcg gaaga 2725
<210> 3
   <211> 9203
   <212> DNA
   <213> Artificial Sequence
<220>
   <223> pUC-Lys2-DK2 (source of URA blaster)
<220>
   <221> CDS
   <222> (5937)..(6797)
<400> 3
   catgcgatat cccatggaag cttggcgtaa tcatggtcat agctgtttcc tgtgtgaaat      60
   tgttatccgc tcacaattcc acacaacata cgagccggaa gcataaagtg taaagcctgg     120
   ggtgcctaat gagtgagcta actcacatta attgcgttgc gctcactgcc cgctttccag     180
   tcgggaaacc tgtcgtgcca gctgcattaa tgaatcggcc aacgcgcggg gagaggcggt     240
   ttgcgtattg ggcgctcttc cgcttcctcg ctcactgact cgctgcgctc ggtcgttcgg     300
   ctgcggcgag cggtatcagc tcactcaaag gcggtaatac ggttatccac agaatcaggg 360
   gataacgcag gaaagaacat gtgagcaaaa ggccagcaaa aggccaggaa ccgtaaaaag 420
   gccgcgttgc tggcgttttt ccataggctc cgcccccctg acgagcatca caaaaatcga 480
   cgctcaagtc agaggtggcg aaacccgaca ggactataaa gataccaggc gtttccccct 540
   ggaagctccc tcgtgcgctc tcctgttccg accctgccgc ttaccggata cctgtccgcc 600
   tttctccctt cgggaagcgt ggcgctttct catagctcac gctgtaggta tctcagttcg 660
   gtgtaggtcg ttcgctccaa gctgggctgt gtgcacgaac cccccgttca gcccgaccgc 720
   tgcgccttat ccggtaacta tcgtcttgag tccaacccgg taagacacga cttatcgcca 780
   ctggcagcag ccactggtaa caggattagc agagcgaggt atgtaggcgg tgctacagag 840
   ttcttgaagt ggtggcctaa ctacggctac actagaagga cagtatttgg tatctgcgct 900
   ctgctgaagc cagttacctt cggaaaaaga gttggtagct cttgatccgg caaacaaacc 960
   accgctggta gcggtggttt ttttgtttgc aagcagcaga ttacgcgcag aaaaaaagga 1020
   tctcaagaag atcctttgat cttttctacg gggtctgacg ctcagtggaa cgaaaactca 1080
   cgttaaggga ttttggtcat gagattatca aaaaggatct tcacctagat ccttttaaat 1140
   taaaaatgaa gttttaaatc aatctaaagt atatatgagt aaacttggtc tgacagttac 1200
   caatgcttaa tcagtgaggc acctatctca gcgatctgtc tatttcgttc atccatagtt 1260
   gcctgactcc ccgtcgtgta gataactacg atacgggagg gcttaccatc tggccccagt 1320
   gctgcaatga taccgcgaga cccacgctca cgggctccag atttatcagc aataaaccag 1380
   ccagccggaa gggccgagcg cagaagtggt cctgcaactt tatccgcctc catccagtct 1440
   attaattgtt gccgggaagc tagagtaagt agttcgccag ttaatagttt gcgcaacgtt 1500
   gttgccattg ctacaggcat cgtggtgtca cgctcgtcgt ttggtatggc ttcattcagc 1560
   tccggttccc aacgatcaag gcgagttaca tgatccccca tgttgtgcaa aaaagcggtt 1620
   agctccttcg gtcctccgat cgttgtcaga agtaagttgg ccgcagtgtt atcactcatg 1680
   gttatggcag cactgcataa ttctcttact gtcatgccat ccgtaagatg cttttctgtg 1740
   actggtgagt actcaaccaa gtcattctga gaatagtgta tgcggcgacc gagttgctct 1800
   tgcccggcgt caatacggga taataccgcg ccacatagca gaactttaaa agtgctcatc 1860
   attggaaaac gttcttcggg gcgaaaactc tcaaggatct taccgctgtt gagatccagt 1920
   tcgatgtaac ccactcgtgc acccaactga tcttcagcat cttttacttt caccagcgtt 1980
   tctgggtgag caaaaacagg aaggcaaaat gccgcaaaaa agggaataag ggcgacacgg 2040
   aaatgttgaa tactcatact cttccttttt caatattatt gaagcattta tcagggttat 2100
   tgtctcatga gcggatacat atttgaatgt atttagaaaa ataaacaaat aggggttccg 2160
   cgcacatttc cccgaaaagt gccacctgac gtctaagaaa ccattattat catgacatta 2220
   acctataaaa ataggcgtat cacgaggccc tttcgtctcg cgcgtttcgg tgatgacggt 2280
   gaaaacctct gacacatgca gctcccggag acggtcacag cttgtctgta agcggatgcc 2340
   gggagcagac aagcccgtca gggcgcgtca gcgggtgttg gcgggtgtcg gggctggctt 2400
   aactatgcgg catcagagca gattgtactg agagtgcacc atatgcggtg tgaaataccg 2460
   cacagatgcg taaggagaaa ataccgcatc aggcgccaac gccgagaagg agtggaacga 2520
   gctcttcgcc aagtacggta aggagtatcc caaggagcac tctgagatca tccgacgatt 2580
   caagcgagag ctgcccgagg gatgggagaa ggctctgcct acctacaccc ccgccgacaa 2640
   tgccgttgct tctcgaaagc tgtccgagat tgtcctcacc aagatccacg aggtcctccc 2700
   cgagcttgtt ggtggttccg ccgatctgac cggctcaaac ctgacccgat ggaaggacgc 2760
   tgttgatttc cagcctcctg tcacccacct tggtgactac tccggccgat atatccgata 2820
   cggtgttcga gagcacggca tgggcgctat catgaacggt atgaacgctt acggaggtat 2880
   catcccctac ggaggtactt tccttaactt cgtctcctac gccgctggtg ccgtccgact 2940
   gtctgccctg tctggccacc acgttatctg ggttgctacc catgactcca ttggtctggg 3000
   tgaggatggc cctacccatc agcccattga gactgtcgcc tggctccgag ccacccccaa 3060
   cctctctgtg tggcgacctg ccgacggtaa cgagacctcc gctgcttact acaaggccat 3120
   caccaactac cacactccct ctgtcctgtc tctgacccga cagaacctgc ctcagcttga 3180
   gggctcttcc atcgagaagg cctccaaggg tggttaccag ctcatctccg aggacaaggg 3240
   tgacatctac cttgtgtcca ctggttctga ggttgccatc tgtgttgctg ccgccaagct 3300
   cctcaaggag aagaagggta tcactgccgg tgtcatctct ctgcccgact ggttcacctt 3360
   cgagcagcag tctctcgagt accgaaagtc tgttttcccc gatggcatcc ccatgctttc 3420
   cgtcgaggtc tactccgact ttggctggtc tcgatactct caccagcagt ttggtctgga 3480
   ccgattcggt gcttctgctc ccttccagca ggtctacgat gcctttgagt tcaatgccga 3540
   gggtgtcgcc aagcgagctg aggccaccat taactactac aagggccaga ctgtcaagtc 3600
   tcctattcag cgagccttcg accccattga cgtcaacacc cgacccggcc acggtgtcta 3660
   agtgggttgc ccgtctgata taagtattga tatagagtat atttaaatgg aaccccatga 3720
   gtttgtagaa ccttgcgtga caactggcat gtatgtgatg tcactgatag ttgtattgcc 3780
   gtatgtaact attacgagtt ctggggatga gattcccatg cttccacttg ctcatcagca 3840
   aacgctatca aatttacgct caatttttga gcactggcta caatacaaac catctcaaac 3900
   gaaaaatgct gtaattattg cgacacctca catttagtca tatatatcat aattaattaa 3960
   atcatacaaa aatagtctgc tcacagacaa gcaataatca ccattacacg gtaacaaata 4020
   acaaaatggc aatgcttcgc atcgccttat ggtcaaggca ctccctacgg cactgcttac 4080
   ttgttaccac cacgtccacc gacactcttc agcttatcaa gggtctgctc agacagctcc     4140
   accttcggca aagcaagctc aaccttggac tggggggcat ccaggaagcc gacagccacc      4200
   aggtaagcca ggtagatacc catttgagcc tcgtcaacgc ccttaccagc ggacaaatcg     4260
   actccggtcc actcagcgtc tcgagagaga gcagatcgag cattggagtc atccagctcg     4320
   ggggccttgg tagactgggg aaggtcatcg agcacaaagt gtagcagagg atacagggca     4380
   gagtcctgcg agtcctcaac aacaaacttc tccagagcca gacgccaggt gacgtagtcc     4440
   tcgacagaaa ctttgtaacc gtacttctgg agggtctgca ggaattcgtt gaaccgtgtt     4500
   cgaggctggg aagtcacatg ggcgacaacc acgccggaag gctgcttggg ccagaaagag     4560
   gcggcagtaa caacccgagc cacgtgatcg acgggcacca tgttgaccga gttgtggatg     4620
   ttgggaatct cacccagctg gatgcatccc ttgatcattc gaaccaggaa atcgtccgtg     4680
   ttggtggctc cggtcttgga gtctccgaca acgtaaccag gtcgaatgat ggttccggtg     4740
   agtccccggg ctccggcctc tcggatcacg tgctctgcga cccacttgga ctgtccgtag     4800
   ccgttgccga gacccacaga agaacccatg agatcgtcgg actcgggaac gccagccttg     4860
   ccctcctcca ccagatcggc agacagcttg ttgaagtgct cggtgtcgac ggcagaggtg     4920
   gaggacacaa agttgaagat ctactgggct gcttcctaat gcaggagtcg cataagggag     4980
   agcgtcgacc gatgcccttg agagccttca acccagtcag ctccttccgg tgggcgcggg     5040
   gcatgactat cgtcgccgca cttatgactg tcttctttat catgcaactc gtaggacagg     5100
   tgccggcagc gctctgggtc attttcggcg aggaccgctt tcgctggagc gcgacgatga     5160
   tcggcctgtc gcttgcggta ttcggaatct tgcacgccct cgctcaagcc ttcgtcactg     5220
   gtcccgccac caaacgtttc ggcgagaagc aggccattat cgccggcatg gcggccgacg     5280
   cgctgggcta cgtcttgctg gcgttcgcga cgcgaggctg gatggccttc cccattatga     5340
   ttcttctcgc ttccggcggc atcgggatgc ccgcgttgca ggccatgctg tccaggcagg     5400
   tagatgacga ccatcaggga cagcttcaag gatcgctcgc ggctcttacc agcctaactt     5460
   cgatcactgg accgctgatc gtcacggcga tttatgccgc ctcggcgagc acatggaacg     5520
   ggttggcatg gattgtaggc gccgccctat accttgtctg cctcctgtct gactcgtcat     5580
   tgccgccttt ggagtacgac tccaactatg agtgtgcttg gatcactttg acgatacatt     5640
   cttcgttgga ggctgtgggt ctgacagctg cgttttcggc gcggttggcc gacaacaata     5700
   tcagctgcaa cgtcattgct ggctttcatc atgatcacat ttttgtcggc aaaggcgacg     5760
   cccagagagc cattgacgtt ctttctaatt tggaccgata gccgtatagt ccagtctatc     5820
   tataagttca actaactcgt aactattacc ataacatata cttcactgcc ccagataagg     5880
   ttccgataaa aagttctgca gactaaattt atttcagtct cctcttcacc accaaa atg     5939
                                                                 Met
                                                                 1
   ccc tcc tac gaa gct cga gct aac gtc cac aag tcc gcc ttt gcc gct        5987
   Pro Ser Tyr Glu Ala Arg Ala Asn Val His Lys Ser Ala Phe Ala Ala
               5                   10                  15
   cga gtg ctc aag ctc gtg gca gcc aag aaa acc aac ctg tgt gct tct        6035
   Arg Val Leu Lys Leu Val Ala Ala Lys Lys Thr Asn Leu Cys Ala Ser
           20                  25                  30
   ctg gat gtt acc acc acc aag gag ctc att gag ctt gcc gat aag gtc        6083
   Leu Asp Val Thr Thr Thr Lys Glu Leu Ile Glu Leu Ala Asp Lys Val
       35                  40                  45
   gga cct tat gtg tgc atg atc aag acc cat atc gac atc att gac gac        6131
   Gly Pro Tyr Val Cys Met Ile Lys Thr His Ile Asp Ile Ile Asp Asp
   50                  55                  60                  65
   ttc acc tac gcc ggc act gtg ctc ccc ctc aag gaa ctt gct ctt aag        6179
   Phe Thr Tyr Ala Gly Thr Val Leu Pro Leu Lys Glu Leu Ala Leu Lys
                   70                  75                  80
   cac ggt ttc ttc ctg ttc gag gac aga aag ttc gca gat att ggc aac        6227
   His Gly Phe Phe Leu Phe Glu Asp Arg Lys Phe Ala Asp Ile Gly Asn
               85                  90                  95
   act gtc aag cac cag tac aag aac ggt gtc tac cga atc gcc gag tgg        6275
   Thr Val Lys His Gln Tyr Lys Asn Gly Val Tyr Arg Ile Ala Glu Trp
           100                 105                 110
   tcc gat atc acc aac gcc cac ggt gta ccc gga acc gga atc att gct        6323
   Ser Asp Ile Thr Asn Ala His Gly Val Pro Gly Thr Gly Ile Ile Ala
       115                 120                 125
   ggc ctg cga gct ggt gcc gag gaa act gtc tct gaa cag aag aag gag        6371
   Gly Leu Arg Ala Gly Ala Glu Glu Thr Val Ser Glu Gln Lys Lys Glu
   130                 135                 140                 145
   gac gtc tct gac tac gag aac tcc cag tac aag gag ttc ctg gtc ccc        6419
   Asp Val Ser Asp Tyr Glu Asn Ser Gln Tyr Lys Glu Phe Leu Val Pro
                   150                 155                 160
   tct ccc aac gag aag ctg gcc aga ggt ctg ctc atg ctg gcc gag ctg        6467
   Ser Pro Asn Glu Lys Leu Ala Arg Gly Leu Leu Met Leu Ala Glu Leu
               165                 170                 175
   tct tgc aag ggc tct ctg gcc act ggc gag tac tcc aag cag acc att        6515
   Ser Cys Lys Gly Ser Leu Ala Thr Gly Glu Tyr Ser Lys Gln Thr Ile
           180                 185                 190
   gag ctt gcc cga tcc gac ccc gag ttt gtg gtt ggc ttc att gcc cag        6563
   Glu Leu Ala Arg Ser Asp Pro Glu Phe Val Val Gly Phe Ile Ala Gln
       195                 200                 205
   aac cga cct aag ggc gac tct gag gac tgg ctt att ctg acc ccc ggg        6611
   Asn Arg Pro Lys Gly Asp Ser Glu Asp Trp Leu Ile Leu Thr Pro Gly
   210                 215                 220                 225
   gtg ggt ctt gac gac aag gga gac gct ctc gga cag cag tac cga act        6659
   Val Gly Leu Asp Asp Lys Gly Asp Ala Leu Gly Gln Gln Tyr Arg Thr
                   230                 235                 240
   gtt gag gat gtc atg tct acc gga acg gat atc ata att gtc ggc cga        6707
   Val Glu Asp Val Met Ser Thr Gly Thr Asp Ile Ile Ile Val Gly Arg
                   245                 250                 255
   ggt ctg tac ggc cag aac cga gat cct att gag gag gcc aag cga tac      6755
   Gly Leu Tyr Gly Gln Asn Arg Asp Pro Ile Glu Glu Ala Lys Arg Tyr
               260                 265                 270
   cag aag gct ggc tgg gag gct tac cag aag att aac tgt tag              6797
   Gln Lys Ala Gly Trp Glu Ala Tyr Gln Lys Ile Asn Cys
           275                 280                 285
   aggttagact atggatatgt catttaactg tgtatataga gagcgtgcaa gtatggagcg    6857
   cttgttcagc ttgtatgatg gtcagacgac ctgtctgatc gagtatgtat gatactgcac    6917
   aacctgtgta tccgcatgat ctgtccaatg gggcatgttg ttgtgtttct cgatacggag    6977
   atgctgggta caagtagcta atacgattga actacttata cttatatgag gcttgaagaa    7037
   agctgacttg tgtatgactt attctcaact acatccccag tcacaatacc accagaattc    7097
   tactgggctg cttcctaatg caggagtcgc ataagggaga gcgtcgaccg atgcccttga    7157
   gagccttcaa cccagtcagc tccttccggt gggcgcgggg catgactatc gtcgccgcac    7217
   ttatgactgt cttctttatc atgcaactcg taggacaggt gccggcagcg ctctgggtca    7277
   ttttcggcga ggaccgcttt cgctggagcg cgacgatgat cggcctgtcg cttgcggtat    7337
   tcggaatctt gcacgccctc gctcaagcct tcgtcactgg tcccgccacc aaacgtttcg    7397
   gcgagaagca ggccattatc gccggcatgg cggccgacgc gctgggctac gtcttgctgg    7457
   cgttcgcgac gcgaggctgg atggccttcc ccattatgat tcttctcgct tccggcggca    7517
   tcgggatgcc cgcgttgcag gccatgctgt ccaggcaggt agatgacgac catcagggac    7577
   agcttcaagg atcgctcgcg gctcttacca gcctaacttc gatcactgga ccgctgatcg    7637
   tcacggcgat ttatgccgcc tcggcgagca catggaacgg gttggcatgg attgtaggcg    7697
   ccgccctata ccttgtctgc ctggatctcc agttctgtac agccgatccc ggggaccctt    7757
   gtagaactct ctccaggcct cacccttgtt gaccttctcg tcctcctgga tccgagacac   7817
   acgtcctcgt cgcccgagag acggtagacg agaatggcgt agagagccag agctgcagag    7877
   aaatcagtag cgcctgctac accgctgttc ttggcaatct gatcgagctc ggccacggtc    7937
   ttggagctaa gagcctccgt caattgtgct tcgactacac tgccttcagc ggggcggacg    7997
   aaatcgaacg ggaggactga gagtgtgggg ttggtaaggg cggttgccca ttgtgttact    8057
   gtattagtac ggtacatagg gccgaaggtc agagcggacc taataaatag ctgacacaga    8117
   ataagtaccc tttctttgtc caggtcatga gtctcggtat cacgtcttaa aagagtcctg    8177
   ccgttgtcca tgagtatagt atagcagaca acatagtatt catactgcga tgacggcggg    8237
   aattggaggc tacagaactc gaagcggggt catatagaca atcgcatttg ccaacgagtg    8297
   tctgattaag gcttctccgt gtgactgctc attgtgtcaa tgctatactg tagtcatagc    8357
   tacattgaca gaaagacaac gacagacagt gataaagtaa ccagatcctc aatcaaaaga    8417
   gccgcaatga tccatttctc atatcgcata ccatccactc tgtttgtgcc gcatcctcca     8477
   caacgatttg ctcatggcac tgttctggtc agatcagaca tcggtagaca caatccagat     8537
   gacagttcac agacgattga tctcaatttt aagcctgacc aagcgatctc acgaacgatt     8597
   ccttcaattt cccatggata ctcacacgac attgtcggta gttttttttt tttgtagaga     8657
   caagttttca aacagatgtc aaaaccgaaa gttgcaagct ttcgatttgg aaggttatat     8717
   cgagacaggt gcactgagtc atactgcagt ggagtgcagc tgatgggggg tgatcaaaga     8777
   aaatggtcac gtgattttac tgacctatag atattatcac tgcaaatgca gatgacataa     8837
   taaatcatac cacgagctcc gcaccccaac tataccctaa ccctacagtc ttctgaaaaa     8897
   cctgatctgt gaacacagac cccaacccgg acaaggcttg aaccgctact cgtacagtac     8957
   atctccaagg tgggttgatg tgcagagcga tgtcattaat gacaggctgt ggttggctgt     9017
   ggtatcaaat gataaagtga taaaaatttg agacacggtt acagattatg agcaacaagg     9077
   aggaatcagg gagatgaatg ggagtaagtt gtggtctcaa tgagtggaac agttgacttt     9137
   tatacagact aatcatagct tcttgcagtc tcattattat gggctctgta acacattcta     9197
   ttgaag                                                                9203
<210> 4
   <211> 286
   <212> PRT
   <213> Artificial Sequence
<220>
   <223> Synthetic Construct
<400> 4
   Met Pro Ser Tyr Glu Ala Arg Ala Asn Val His Lys Ser Ala Phe Ala
   1               5                   10                  15
   Ala Arg Val Leu Lys Leu Val Ala Ala Lys Lys Thr Asn Leu Cys Ala
               20                  25                  30
   Ser Leu Asp Val Thr Thr Thr Lys Glu Leu Ile Glu Leu Ala Asp Lys
           35                  40                  45
   Val Gly Pro Tyr Val Cys Met Ile Lys Thr His Ile Asp Ile Ile Asp
       50                  55                  60
   Asp Phe Thr Tyr Ala Gly Thr Val Leu Pro Leu Lys Glu Leu Ala Leu
   65                  70                  75                  80
   Lys His Gly Phe Phe Leu Phe Glu Asp Arg Lys Phe Ala Asp Ile Gly
                   85                  90                  95
   Asn Thr Val Lys His Gln Tyr Lys Asn Gly Val Tyr Arg Ile Ala Glu
               100                 105                 110
   Trp Ser Asp Ile Thr Asn Ala His Gly Val Pro Gly Thr Gly Ile Ile
           115                 120                 125
   Ala Gly Leu Arg Ala Gly Ala Glu Glu Thr Val Ser Glu Gln Lys Lys
       130                 135                 140
   Glu Asp Val Ser Asp Tyr Glu Asn Ser Gln Tyr Lys Glu Phe Leu Val
   145                 150                 155                 160
   Pro Ser Pro Asn Glu Lys Leu Ala Arg Gly Leu Leu Met Leu Ala Glu
                   165                 170                 175
   Leu Ser Cys Lys Gly Ser Leu Ala Thr Gly Glu Tyr Ser Lys Gln Thr
               180                 185                 190
   Ile Glu Leu Ala Arg Ser Asp Pro Glu Phe Val Val Gly Phe Ile Ala
           195                 200                 205
   Gln Asn Arg Pro Lys Gly Asp Ser Glu Asp Trp Leu Ile Leu Thr Pro
       210                 215                 220
   Gly Val Gly Leu Asp Asp Lys Gly Asp Ala Leu Gly Gln Gln Tyr Arg
   225                 230                 235                 240
   Thr Val Glu Asp Val Met Ser Thr Gly Thr Asp Ile Ile Ile Val Gly
                   245                 250                 255
   Arg Gly Leu Tyr Gly Gln Asn Arg Asp Pro Ile Glu Glu Ala Lys Arg
               260                 265                 270
   Tyr Gln Lys Ala Gly Trp Glu Ala Tyr Gln Lys Ile Asn Cys
           275                 280                 285
<210> 5
   <211> 10839
   <212> DNA
   <213> Artificial Sequence
<220>
   <223> pINTB-HMG1 (cloning vector)
<220>
   <221> CDS
   <222> (207)..(3206)
   <223> 3-methyl-glutaryl-CoA reductase (HMG1) gene
<210> 6
   <211> 999
   <212> PRT
   <213> Artificial Sequence
<220>
   <223> Synthetic Construct
<400> 6
   Met Leu Gln Ala Ala Ile Gly Lys Ile Val Gly Phe Ala Val Asn Arg
   1               5                   10                  15
   Pro Ile His Thr Val Val Leu Thr Ser Ile Val Ala Ser Thr Ala Tyr
               20                  25                  30
   Leu Ala Ile Leu Asp Ile Ala Ile Pro Gly Phe Glu Gly Thr Gln Pro
           35                  40                  45
   Ile Ser Tyr Tyr His Pro Ala Ala Lys Ser Tyr Asp Asn Pro Ala Asp
       50                  55                  60
   Trp Thr His Ile Ala Glu Ala Asp Ile Pro Ser Asp Ala Tyr Arg Leu
   65                  70                  75                  80
   Ala Phe Ala Gln Ile Arg Val Ser Asp Val Gln Gly Gly Glu Ala Pro
                   85                  90                  95
   Thr Ile Pro Gly Ala Val Ala Val Ser Asp Leu Asp His Arg Ile Val
               100                 105                 110
   Met Asp Tyr Lys Gln Trp Ala Pro Trp Thr Ala Ser Asn Glu Gln Ile
           115                 120                 125
   Ala Ser Glu Asn His Ile Trp Lys His Ser Phe Lys Asp His Val Ala
       130                 135                 140
   Phe Ser Trp Ile Lys Trp Phe Arg Trp Ala Tyr Leu Arg Leu Ser Thr
   145                 150                 155                 160
   Leu Ile Gln Gly Ala Asp Asn Phe Asp Ile Ala Val Val Ala Leu Gly
   165 170 175
   Tyr Leu Ala Met His Tyr Thr Phe Phe Ser Leu Phe Arg Ser Met Arg
   180 185 190
   Lys Val Gly Ser His Phe Trp Leu Ala Ser Met Ala Leu Val Ser Ser
   195 200 205
   Thr Phe Ala Phe Leu Leu Ala Val Val Ala Ser Ser Ser Leu Gly Tyr
   210 215 220
   Arg Pro Ser Met Ile Thr Met Ser Glu Gly Leu Pro Phe Leu Val Val
   225 230 235 240
   Ala Ile Gly Phe Asp Arg Lys Val Asn Leu Ala Ser Glu Val Leu Thr
   245 250 255
   Ser Lys Ser Ser Gln Leu Ala Pro Met Val Gln Val Ile Thr Lys Ile
   260 265 270
   Ala Ser Lys Ala Leu Phe Glu Tyr Ser Leu Glu Val Ala Ala Leu Phe
   275 280 285
   Ala Gly Ala Tyr Thr Gly Val Pro Arg Leu Ser Gln Phe Cys Phe Leu
   290 295 300
   Ser Ala Trp Ile Leu Ile Phe Asp Tyr Met Phe Leu Leu Thr Phe Tyr
   305 310 315 320
   Ser Ala Val Leu Ala Ile Lys Phe Glu Ile Asn His Ile Lys Arg Asn
   325 330 335
   Arg Met Ile Gln Asp Ala Leu Lys Glu Asp Gly Val Ser Ala Ala Val
   340 345 350
   Ala Glu Lys Val Ala Asp Ser Ser Pro Asp Ala Lys Leu Asp Arg Lys
   355 360 365
   Ser Asp Val Ser Leu Phe Gly Ala Ser Gly Ala Ile Ala Val Phe Lys
   370 375 380
   Ile Phe Met Val Leu Gly Phe Leu Gly Leu Asn Leu Ile Asn Leu Thr
   385 390 395 400
   Ala Ile Pro His Leu Gly Lys Ala Ala Ala Ala Ala Gln Ser Val Thr
   405 410 415
   Pro Ile Thr Leu Ser Pro Glu Leu Leu His Ala Ile Pro Ala Ser Val
               420                 425                 430
   Pro Val Val Val Thr Phe Val Pro Ser Val Val Tyr Glu His Ser Gln
           435                 440                 445
   Leu Ile Leu Gln Leu Glu Asp Ala Leu Thr Thr Phe Leu Ala Ala Cys
       450                 455                 460
   Ser Lys Thr Ile Gly Asp Pro Val Ile Ser Lys Tyr Ile Phe Leu Cys
   465                 470                 475                 480
   Leu Met Val Ser Thr Ala Leu Asn Val Tyr Leu Phe Gly Ala Thr Arg
                   485                 490                 495
   Glu Val Val Arg Thr Gln Ser Val Lys Val Val Glu Lys His Val Pro
               500                 505                 510
   Ile Val Ile Glu Lys Pro Ser Glu Lys Glu Glu Asp Thr Ser Ser Glu
           515                 520                 525
   Asp Ser Ile Glu Leu Thr Val Gly Lys Gln Pro Lys Pro Val Thr Glu
       530                 535                 540
   Thr Arg Ser Leu Asp Asp Leu Glu Ala Ile Met Lys Ala Gly Lys Thr
   545                 550                 555                 560
   Lys Leu Leu Glu Asp His Glu Val Val Lys Leu Ser Leu Glu Gly Lys
                   565                 570                 575
   Leu Pro Leu Tyr Ala Leu Glu Lys Gln Leu Gly Asp Asn Thr Arg Ala
               580                 585                 590
   Val Gly Ile Arg Arg Ser Ile Ile Ser Gln Gln Ser Asn Thr Lys Thr
           595                 600                 605
   Leu Glu Thr Ser Lys Leu Pro Tyr Leu His Tyr Asp Tyr Asp Arg Val
       610                 615                 620
   Phe Gly Ala Cys Cys Glu Asn Val Ile Gly Tyr Met Pro Leu Pro Val
   625                 630                 635                 640
   Gly Val Ala Gly Pro Met Asn Ile Asp Gly Lys Asn Tyr His Ile Pro
                   645                 650                 655
   Met Ala Thr Thr Glu Gly Cys Leu Val Ala Ser Thr Met Arg Gly Cys
               660                 665                 670
   Lys Ala Ile Asn Ala Gly Gly Gly Val Thr Thr Val Leu Thr Gln Asp
           675                 680                 685
   Gly Met Thr Arg Gly Pro Cys Val Ser Phe Pro Ser Leu Lys Arg Ala
       690                 695                 700
   Gly Ala Ala Lys Ile Trp Leu Asp Ser Glu Glu Gly Leu Lys Ser Met
   705                 710                 715                 720
   Arg Lys Ala Phe Asn Ser Thr Ser Arg Phe Ala Arg Leu Gln Ser Leu
                   725                 730                 735
   His Ser Thr Leu Ala Gly Asn Leu Leu Phe Ile Arg Phe Arg Thr Thr
               740                 745                 750
   Thr Gly Asp Ala Met Gly Met Asn Met Ile Ser Lys Gly Val Glu His
           755                 760                 765
   Ser Leu Ala Val Met Val Lys Glu Tyr Gly Phe Pro Asp Met Asp Ile
       770                 775                 780
   Val Ser Val Ser Gly Asn Tyr Cys Thr Asp Lys Lys Pro Ala Ala Ile
   785                 790                 795                 800
   Asn Trp Ile Glu Gly Arg Gly Lys Ser Val Val Ala Glu Ala Thr Ile
                   805                 810                 815
   Pro Ala His Ile Val Lys Ser Val Leu Lys Ser Glu Val Asp Ala Leu
               820                 825                 830
   Val Glu Leu Asn Ile Ser Lys Asn Leu Ile Gly Ser Ala Met Ala Gly
           835                 840                 845
   Ser Val Gly Gly Phe Asn Ala His Ala Ala Asn Leu Val Thr Ala Ile
       850                 855                 860
   Tyr Leu Ala Thr Gly Gln Asp Pro Ala Gln Asn Val Glu Ser Ser Asn
   865                 870                 875                 880
   Cys Ile Thr Leu Met Ser Asn Val Asp Gly Asn Leu Leu Ile Ser Val
                   885                 890                 895
   Ser Met Pro Ser Ile Glu Val Gly Thr Ile Gly Gly Gly Thr Ile Leu
               900                 905                 910
   Glu Pro Gln Gly Ala Met Leu Glu Met Leu Gly Val Arg Gly Pro His
           915                 920                 925
   Ile Glu Thr Pro Gly Ala Asn Ala Gln Gln Leu Ala Arg Ile Ile Ala
       930                 935                 940
   Ser Gly Val Leu Ala Ala Glu Leu Ser Leu Cys Ser Ala Leu Ala Ala
   945                 950                 955                 960
   Gly His Leu Val Gln Ser His Met Thr His Asn Arg Ser Gln Ala Pro
                   965                 970                 975
   Thr Pro Ala Lys Gln Ser Gln Ala Asp Leu Gln Arg Leu Gln Asn Gly
               980                 985                 990
   Ser Asn Ile Cys Ile Arg Ser
           995
<210> 7
   <211> 20
   <212> DNA
   <213> Yarrowia lipolytica
<400> 7
   tccagaagcg ctacaaagag 20
<210> 8
   <211> 38
   <212> DNA
   <213> Artificial Sequence
<220>
   <223> primer
<400> 8
   attaccctgt tatccctatg aaggttgcag tcgtagtc 38
<210> 9
   <211> 38
   <212> DNA
   <213> Artificial Sequence
<220>
   <223> primer
<400> 9
   tagggataac agggtaattg cgatctcgat gagtgatg 38
<210> 10
   <211> 20
   <212> DNA
   <213> Yarrowia lipolytica
<400> 10
   gcccagaaga ttggaatgac 20
<210> 11
   <211> 31
   <212> DNA
   <213> Artificial Sequence
<220>
   <223> primer
<400> 11
   atataccgcg ggattccgcc aagtgagact g 31
<210> 12
   <211> 38
   <212> DNA
   <213> Artificial Sequence
<220>
   <223> primer
<400> 12
   attaccctgt tatccctacg tcgaggaagt aggtcatc 38
<210> 13
   <211> 38
   <212> DNA
   <213> Artificial Sequence
<220>
   <223> primer
<400> 13
   tagggataac agggtaatgc gagcttgatg aggaatag 38
<210> 14
   <211> 31
   <212> DNA
   <213> Artificial Sequence
<220>
   <223> primer
<400> 14
   atataccgcg gcctgacgcc aatttgaaga g 31
<210> 15
   <211> 31
   <212> DNA
   <213> Artificial Sequence
<220>
   <223> primer
<400> 15
   atataccgcg gctgggctgt tcggtcgata g 31
<210> 16
   <211> 38
   <212> DNA
   <213> Artificial Sequence
<220>
   <223> primer
<400> 16
   tagggataac agggtaatag gacgcacaac gccatcac 38
<210> 17
   <211> 36
   <212> DNA
   <213> Artificial Sequence
<220>
   <223> primer
<400> 17
   attaccctgt tatccctacg ctcccattgg aaacta 36
<210> 18
   <211> 31
   <212> DNA
   <213> Artificial Sequence
<220>
   <223> primer
<400> 18
   atataccgcg gtctcttcgc tgtggtctag g 31
<210> 19
   <211> 31
   <212> DNA
   <213> Artificial Sequence
<220>
   <223> primer
<400> 19
   atataccgcg gtccaccgtt ctccttcata c 31
<210> 20
   <211> 38
   <212> DNA
   <213> Artificial Sequence
<220>
   <223> primer
<400> 20
   tagggataac agggtaatat gtctctaggg tcgaagtc 38
<210> 21
   <211> 38
   <212> DNA
   <213> Artificial Sequence
<220>
   <223> primer
<400> 21
   attaccctgt tatccctatg gcaagcctca ctactacg 38
<210> 22
   <211> 31
   <212> DNA
   <213> Artificial Sequence
<220>
   <223> primer
<400> 22
   atataccgcg gtgcggcgga actactgtat c 31
<210> 23
   <211> 31
   <212> DNA
   <213> Artificial Sequence
<220>
   <223> primer
<400> 23
   atataccgcg ggggattctc cgggttattt g 31
<210> 24
   <211> 38
   <212> DNA
   <213> Artificial Sequence
<220>
   <223> primer
<400> 24
   tagggataac agggtaatac gtctcggacc ttgaattg 38
<210> 25
   <211> 38
   <212> DNA
   <213> Artificial Sequence
<220>
   <223> primer
<400> 25
   attaccctgt tatccctacc ttcaacctgt ccgacttc 38
<210> 26
   <211> 31
   <212> DNA
   <213> Artificial Sequence
<220>
   <223> primer
<400> 26
   atataccgcg ggaagcggtc ctcgttgtat g 31
<210> 27
   <211> 20
   <212> DNA
   <213> Yarrowia lipolytica
<400> 27
   gtgtagcaac tcggatacag 20
<210> 28
   <211> 38
   <212> DNA
   <213> Artificial Sequence
<220>
   <223> primer
<400> 28
   tagggataac agggtaatgg tccataagca gagtgttc 38
<210> 29
   <211> 38
   <212> DNA
   <213> Artificial Sequence
<220>
   <223> primer
<400> 29
   attaccctgt tatccctaac cctcgacctc cttattac 38
<210> 30
   <211> 20
   <212> DNA
   <213> Yarrowia lipolytica
<400> 30
   ctcttcttga ctggcatagc 20
<210> 31
   <211> 31
   <212> DNA
   <213> Artificial Sequence
<220>
   <223> primer
<400> 31
   atataaagct tgcggctcgg cacgtgatct g 31
<210> 32
   <211> 32
   <212> DNA
   <213> Artificial Sequence
<220>
   <223> primer
<400> 32
   atataggatc catcgtgcgt acgtcgctag tg 32
<210> 33
   <211> 31
   <212> DNA
   <213> Artificial Sequence
<220>
   <223> primer
<400> 33
   atataggatc ccgaccggca cgatcaattg g 31
<210> 34
   <211> 32
   <212> DNA
   <213> Artificial Sequence
<220>
   <223> primer
<400> 34
   atatacatat gggtgcatct cagcccgacc tc 32
<210> 35
   <211> 20
   <212> DNA
   <213> Yarrowia lipolytica
<400> 35
   tcccgaaaca cagaacttcc 20
<210> 36
   <211> 32
   <212> DNA
   <213> Artificial Sequence
<220>
   <223> primer
<400> 36
   atataaagct tggtggacgt tccggcagac ag 32
<210> 37
   <211> 32
   <212> DNA
   <213> Artificial Sequence
<220>
   <223> primer
<400> 37
   atataggatc cctcccaggc atctccacac tc 32
<210> 38
   <211> 31
   <212> DNA
   <213> Artificial Sequence
<220>
   <223> primer
<400> 38
   atataggatc ccacttacag gcttagcaag g 31
<210> 39
   <211> 32
   <212> DNA
   <213> Artificial Sequence
<220>
   <223> primer
<400> 39
   atatagcggc cgcggaaatc acgcttgatt cg 32
<210> 40
   <211> 20
   <212> DNA
   <213> Yarrowia lipolytica
<400> 40
   taggcgctgg tacagaagag 20
<210> 41
   <211> 31
   <212> DNA
   <213> Artificial Sequence
<220>
   <223> primer
<400> 41
   atataaagct tgagtacagt aggtggtact c 31
<210> 42
   <211> 31
   <212> DNA
   <213> Artificial Sequence
<220>
   <223> primer
<400> 42
   atataggatc cagtggtggt ggtggtggta g 31
<210> 43
   <211> 31
   <212> DNA
   <213> Artificial Sequence
<220>
   <223> primer
<400> 43
   atataggatc ctttacgtgc aacaggagga g 31
<210> 44
   <211> 31
   <212> DNA
   <213> Artificial Sequence
<220>
   <223> primer
<400> 44
   atatacatat ggcctgtctt gagttctttg g 31
<210> 45
   <211> 20
   <212> DNA
   <213> Yarrowia lipolytica
<400> 45
   agggtcgtag ataacgagtc 20
<210> 46
   <211> 32
   <212> DNA
   <213> Artificial Sequence
<220>
   <223> primer
<400> 46
   atataaagct tcacgtggct gctgggccaa cc 32
<210> 47
   <211> 31
   <212> DNA
   <213> Artificial Sequence
<220>
   <223> primer
<400> 47
   atataggatc ccgcacggta tcggagcatc g 31
<210> 48
   <211> 31
   <212> DNA
   <213> Artificial Sequence
<220>
   <223> primer
<400> 48
   atataggatc ccgcggctat tgacgctgag g 31
<210> 49
   <211> 34
   <212> DNA
   <213> Artificial Sequence
<220>
   <223> primer
<400> 49
   atatacatat gcccgtcagc tccatcgacg agtg 34
<210> 50
   <211> 23
   <212> DNA
   <213> Artificial Sequence
<220>
   <223> primer
<400> 50
   aggtgtactg tagccaccct gac 23
<210> 51
   <211> 31
   <212> DNA
   <213> Artificial Sequence
<220>
   <223> primer
<400> 51
   atataaagct tccggccagc cgctggcaac g 31
<210> 52
   <211> 31
   <212> DNA
   <213> Artificial Sequence
<220>
   <223> primer
<400> 52
   atataggatc cacacgacag ctgcacctga c 31
<210> 53
   <211> 31
   <212> DNA
   <213> Artificial Sequence
<220>
   <223> primer
<400> 53
   atataggatc ccagccatga gccaggcatt g 31
<210> 54
   <211> 33
   <212> DNA
   <213> Artificial Sequence
<220>
   <223> primer
<400> 54
   atatacatat gggcgccagc cacatttgcc ctc 33
<210> 55
   <211> 20
   <212> DNA
   <213> Yarrowia lipolytica
<400> 55
   agcgatacag cagttgactc 20
<210> 56
   <211> 20
   <212> DNA
   <213> Yarrowia lipolytica
<400> 56
   tcagccgtct acttgtagag 20
<210> 57
   <211> 31
   <212> DNA
   <213> Artificial Sequence
<220>
   <223> primer
<400> 57
   atataggatc cgtggctcgg atactcctga c 31
<210> 58
   <211> 31
   <212> DNA
   <213> Artificial Sequence
<220>
   <223> primer
<400> 58
   atataggatc cagccggagg tcagatcaag c 31
<210> 59
   <211> 32
   <212> DNA
   <213> Artificial Sequence
<220>
   <223> primer
<400> 59
   atatacatat ggcgcaatag ttcgccggcc tg 32
<210> 60
   <211> 20
   <212> DNA
   <213> Yarrowia lipolytica
<400> 60
   ctcgtgttgt gcctttcttg 20
<210> 61
   <211> 34
   <212> DNA
   <213> Artificial Sequence
<220>
   <223> primer
<400> 61
   atataaagct tgcgcgacaa cccatagcga tggc 34
<210> 62
   <211> 31
   <212> DNA
   <213> Artificial Sequence
<220>
   <223> primer
<400> 62
   atataggatc cgataagagg gcgctctgac c 31
<210> 63
   <211> 30
   <212> DNA
   <213> Artificial Sequence
<220>
   <223> primer
<400> 63
   atataggatc cggcgtgaca tcgagtttgg 30
<210> 64
   <211> 31
   <212> DNA
   <213> Artificial Sequence
<220>
   <223> primer
<400> 64
   atatacatat gctacgtctc gccgcagagg g 31
<210> 65
   <211> 20
   <212> DNA
   <213> Yarrowia lipolytica
<400> 65
   agcgagaggt tatacggaag 20
<210> 66
   <211> 20
   <212> DNA
   <213> Yarrowia lipolytica
<400> 66
   ctcctacagc ctctcaagac 20
<210> 67
   <211> 20
   <212> DNA
   <213> Yarrowia lipolytica
<400> 67
   gtctacaaga cagcccagag 20
<210> 68
   <211> 20
   <212> DNA
   <213> Yarrowia lipolytica
<400> 68
   ccgcttgaga agagcaatac 20
<210> 69
   <211> 31
   <212> DNA
   <213> Artificial Sequence
<220>
   <223> primer
<400> 69
   atataaagct tcgccacctg tccacgtctc g 31
<210> 70
   <211> 31
   <212> DNA
   <213> Artificial Sequence
<220>
   <223> primer
<400> 70
   atataggatc cgcgaagcga cgtgtggtga g 31
<210> 71
   <211> 31
   <212> DNA
   <213> Artificial Sequence
<220>
   <223> primer
<400> 71
   atataggatc cgctgagcac gcgagtacac c 31
<210> 72
   <211> 31
   <212> DNA
   <213> Artificial Sequence
<220>
   <223> primer
<400> 72
   atatagaatt cgatctgtcg tacaactaag g 31
<210> 73
   <211> 20
   <212> DNA
   <213> Yarrowia lipolytica
<400> 73
   cagaagttac gacgccaagg 20
<210> 74
   <211> 31
   <212> DNA
   <213> Artificial Sequence
<220>
   <223> primer
<400> 74
   atataaagct tccgtaataa atgcccgtct c 31
<210> 75
   <211> 31
   <212> DNA
   <213> Artificial Sequence
<220>
   <223> primer
<400> 75
   atataggatc caggtcgatt tcggcgctgt g 31
<210> 76
   <211> 31
   <212> DNA
   <213> Artificial Sequence
<220>
   <223> primer
<400> 76
   atataggatc cggttaggca aatagctaat g 31
<210> 77
   <211> 31
   <212> DNA
   <213> Artificial Sequence
<220>
   <223> primer
<400> 77
   atatagaatt cgcctggagc gagtttctga g 31
<210> 78
   <211> 20
   <212> DNA
   <213> Yarrowia lipolytica
<400> 78
   tcgcaagggc catagaggtg 20
<210> 79
   <211> 31
   <212> DNA
   <213> Artificial Sequence
<220>
   <223> primer
<400> 79
   atataaagct tccttgcaca tctccgatga c 31
<210> 80
   <211> 31
   <212> DNA
   <213> Artificial Sequence
<220>
   <223> primer
<400> 80
   atataggatc cggtgttacg ccaccacgtt g 31
<210> 81
   <211> 31
   <212> DNA
   <213> Artificial Sequence
<220>
   <223> primer
<400> 81
   atataggatc cgggacctgc gatacgaatg c 31
<210> 82
   <211> 31
   <212> DNA
   <213> Artificial Sequence
<220>
   <223> primer
<400> 82
   atatagaatt cgcttgcgca gccggtgtat c 31
<210> 83
   <211> 20
   <212> DNA
   <213> Yarrowia lipolytica
<400> 83
   ggcgttgtgg agctatcacc 20
<210> 84
   <211> 31
   <212> DNA
   <213> Artificial Sequence
<220>
   <223> primer
<400> 84
   atataaagct tgcgtgcggt gcgtgcgtat g 31
<210> 85
   <211> 31
   <212> DNA
   <213> Artificial Sequence
<220>
   <223> primer
<400> 85
   atataggatc ccagcaccac gaaattatac g 31
<210> 86
   <211> 31
   <212> DNA
   <213> Artificial Sequence
<220>
   <223> primer
<400> 86
   atataggatc cgtgcgctta catgtggacc g 31
<210> 87
   <211> 31
   <212> DNA
   <213> Artificial Sequence
<220>
   <223> primer
<400> 87
   atatagaatt ccaggcagct tcttgccaat g 31
<210> 88
   <211> 20
   <212> DNA
   <213> Yarrowia lipolytica
<400> 88
   gagataggag gttcccatac 20
<210> 89
   <211> 20
   <212> DNA
   <213> Yarrowia lipolytica
<400> 89
   ctacgcttgt tcagactttg 20
<210> 90
   <211> 46
   <212> DNA
   <213> Artificial Sequence
<220>
   <223> primer
<400> 90
   gtgtgcttgt cgtcagacat tttgaatgat tcttatactc agaagg 46
<210> 91
   <211> 46
   <212> DNA
   <213> Artificial Sequence
<220>
   <223> primer
<400> 91
   ccttctgagt ataagaatca ttcaaaatgt ctgacgacaa gcacac 46
<210> 92
   <211> 26
   <212> DNA
   <213> Artificial Sequence
<220>
   <223> primer
<400> 92
   gcatgcttag attcgaggtc ggagat 26
<210> 93
   <211> 26
   <212> DNA
   <213> Artificial Sequence
<220>
   <223> primer
<400> 93
   ggtaccacgc acggatagtt tatcca 26
<210> 94
   <211> 31
   <212> DNA
   <213> Artificial Sequence
<220>
   <223> primer
<400> 94
   ggtacccaaa gtcaactaat gtcaagtaaa g 31
<210> 95
   <211> 17
   <212> DNA
   <213> Yarrowia lipolytica
<400> 95
   cctccaacgt gactttc 17
<210> 96
   <211> 17
   <212> DNA
   <213> Yarrowia lipolytica
<400> 96
   agagacctcc cacaaag 17
<210> 97
   <211> 46
   <212> DNA
   <213> Artificial Sequence
<220>
   <223> primer
<400> 97
   agagagtcga gtagagccat tttgaatgat tcttatactc agaagg 46
<210> 98
   <211> 46
   <212> DNA
   <213> Artificial Sequence
<220>
   <223> primer
<400> 98
   ccttctgagt ataagaatca ttcaaaatgg ctctactcga ctctct 46
<210> 99
   <211> 35
   <212> DNA
   <213> Yarrowia lipolytica
<400> 99
   atatagcatg cctaccacac atcttcctgg tagac 35
<210> 100
   <211> 18
   <212> DNA
   <213> Yarrowia lipolytica
<400> 100
   ctcaagatac ggcattgg 18
<210> 101
   <211> 18
   <212> DNA
   <213> Yarrowia lipolytica
<400> 101
   tccttggcta gacgaatg 18
1

## Beschreibung

Die Erfindung betrifft verschiedene Hefestämme der Art *Yarrowia lipolytica* sowie zugehörige Verfahren zur biokatalytischen Herstellung von ω-Hydroxyfettsäuren oder Dicarbonsäuren mithilfe dieser Stämme.

Bei Dicarbonsäuren handelt es sich um Carbonsäuren mit zwei Carboxy-Gruppen (allgemeine Struktur: HOOC-(CH₂)ₙ-COOH), die vielfältige Anwendungen in der chemischen Industrie, z.B. zur Herstellung von Duftstoffen, Klebstoffen, Nylon und anderen Polyamiden, Harzen, Korrosionsinhibitoren oder Schmierstoffen, finden. Insbesondere die biotechnologische Produktion langkettiger Dicarbonsäuren ist von besonderem Interesse, da bei der chemischen Synthese der selbigen eine Reihe an unerwünschten Nebenprodukten gebildet wird.

ω-Hydroxyfettsäuren enthalten am C_{ω}-Atom (C-Atom mit der weitest möglichen Entfernung zur Carboxygruppe) eine Hydroxylgruppe (allgemeine Struktur: HOOC-(CH₂)ₙ-CH₂OH). ω-Hydroxyfettsäuren sind ebenfalls von großer Bedeutung in der chemischen Industrie, da sie u.a. in Schmiermitteln, Klebstoffen, Kosmetika und Krebstherapeutika Anwendung finden. Weiterhin könnten ω-Hydroxyfettsäuren in Zukunft eine große Bedeutung erlangen, da sie als Monomere zur Synthese von Biokunststoffen genutzt werden können.

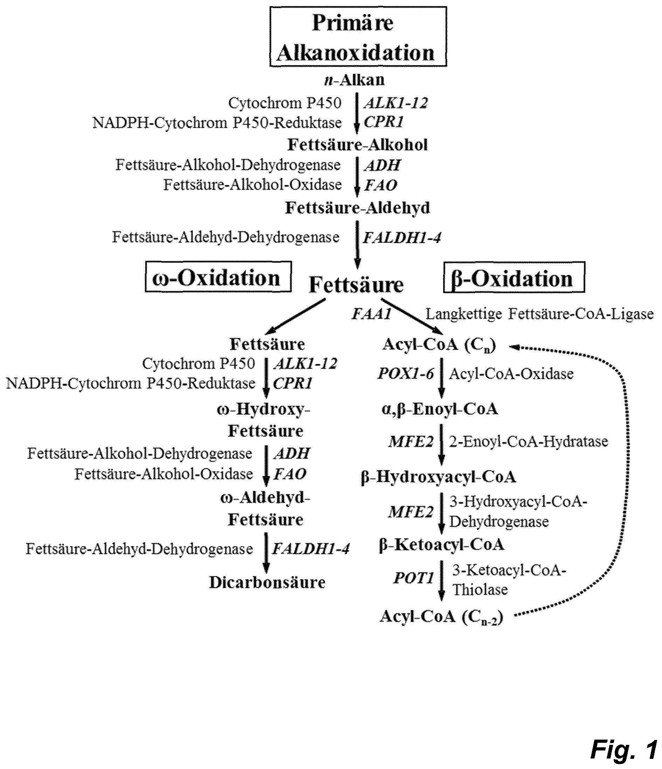

Zur Gewinnung von ω-Hydroxyfettsäuren und Dicarbonsäuren aus *n*-Alkanen und Fettsäuren soll im Rahmen dieser Erfindung die nicht-konventionelle Hefe *Yarrowia* (*Y*.) *lipolytica* genutzt werden. Die Hefe *Y. lipolytica* wurde als Wirtsorganismus für die Biokonversion ausgewählt, weil sie in der Lage ist eine Vielzahl an Substraten als Kohlenstoffquelle zu nutzen. Hierzu zählen neben Glucose, Glycerol, Proteinen, Alkoholen und Acetat auch eine Vielzahl an hydrophoben Substraten wie Pflanzenöle, Fette, Fettsäuren und *n*-Alkane (Barth G & Gaillardin C (1997) FEMS Microbiol Rev 19: 219-237.). Die hydrophoben Substrate werden durch die Hefen emulgiert und mithilfe spezialisierter Membrantransporter in das Zellinnere aufgenommen (Fickers P, Benetti PH, Wache Y, Marty A, Mauersberger S, Smit MS & Nicaud JM (2005) FEMS Yeast Res 5: 527-543.). In die Zelle aufgenommene n-Alkane werden im Rahmen der primären (monoterminalen) Alkanoxidation schrittweise zur Fettsäure mit gleicher Kettenlänge umgesetzt (Fig. 1). Die Fettsäuren werden anschließend im Rahmen der β-Oxidation zu Acetyl-CoA abgebaut, das in den Tricarbonsäure- und den Glyoxylatzyklus einfließt. Parallel zur β-Oxidation von Fettsäuren in den Peroxisomen findet die ω-Oxidation im Endoplasmatischen Retikulum statt (vgl. Fig. 1). Diese diterminale Oxidation von Fettsäuren findet natürlicher Weise jedoch in einem weitaus geringeren Umfang als die β-Oxidation statt.

Verschiedene Hefen, wie z.B. *Candida* (*C*.) *tropicalis* und *Y. lipolytica* sind in der Lage, langkettige Alkane oder Fettsäuren zu α,ω-Dicarbonsäuren umzusetzen. Damit die hydrophoben Substrate überhaupt zur jeweiligen Dicarbonsäure umgesetzt werden können, muss deren Verstoffwechselung im Rahmen der β-Oxidation unterbunden werden. Dies kann z.B. durch die Deletion der für die Acyl-CoA-Oxidase kodierenden POX-Gene geschehen. Ist die β-Oxidation ausgeschaltet, führt dies zu einer gesteigerten ω-Oxidation der Fettsäuren (Smit et al. (2005) Biotechnol. Lett. 27: 859-864.).

Die mikrobielle Produktion langkettiger Dicarbonsäuren wurde bereits mithilfe der Hefe *C. tropicalis* durchgeführt:
- Picataggio S, Rohrer T, Deanda K, Lanning D, Reynolds R, Mielenz J & Eirich LD (1992) Biotechnology (N Y) 10: 894-898.

Die Produktion der Dicarbonsäuren wurde hierbei vor allem durch die Deletion der Gene *POX4* und *POX5* erreicht. Es bestehen eine Reihe von Patenten, die entsprechende C. tropicalis-Produktionsstämme sowie zugehörige Produktionsverfahren schützen (z.B. US4339536A, EP296506A2 und WO200017380A1).

In einer Patentschrift von 2013 wird beschrieben, wie Hefen allgemein (in an sich bekannter Weise) zur Produktion erhöhter Mengen an Dicarbonsäuren veranlasst werden können. In den Ausführungsbeispielen wird hier jedoch im Speziellen nur auf *C. tropicalis* eingegangen (WO201306730A2, WO201306733A2).

Ebenso wurde die mikrobielle Produktion langkettiger ω-Hydroxyfettsäuren bereits mithilfe der Hefe *C. tropicalis* durchgeführt:
- Lu W, Ness JE, Xie W, Zhang X, Minshull J & Gross RA (2010) J Am Chem Soc 132: 15451-15455.

Hierfür wurden zusätzlich zu den Genen *POX4* und *POX5* sechs Cytochrom P450-, vier Alkohol-Oxidase- und sechs Alkohol-Dehydrogenase-Gene deletiert. Auch diese Stämme und Produktionsverfahren sind bereits patentrechtlich geschützt (WO2011008232A2).

Des Weiteren wurde bereits beschrieben, wie *Y. lipolytica* zur Bildung von Dicarbonsäuren veranlasst werden kann:
- Smit MS, Mokgoro MM, Setati E & Nicaud JM (2005) Biotechnol Lett 27: 859-864.

Hierbei wurden alle derzeit bekannten Acyl-CoA-Oxidase-Gene (*POX1, POX2, POX3, POX4, POX5 und POX6)* sowie das Acyl-CoA-Diacylglycerol-Acyltransferase-Gen (*DGA1*) und das Lecithin-Cholesterol-Acyltransferase-Gen (*LRO1*) deletiert. Weiterhin wurde das NADPH-Cytochrom P450-Reduktase-Gen (*CPR1*) überexprimiert (WO200664131A8).

Neben der biotechnologischen Produktion von ω-Hydroxyfettsäuren und Dicarbonsäuren mithilfe von gentechnisch veränderten Hefen unter Ausnutzung der intrazellulären ω-Oxidation von Fettsäuren gibt es auch weitere biotechnologische Verfahren, die auf anderen enzymatischen Umsetzungen beruhen. Ein Beispiel hierfür stellt die Verwendung der Baeyer-Villiger-Monooxygenase, die die Umwandlung eines Ketons zum Ester katalysiert, dar (WO2013151393A1).

Aufgabe der vorliegenden Erfindung ist die Bereitstellung neuartiger Hefestämme der Art *Y. lipolytica* die zur mikrobiellen Produktion von ω-Hydroxyfettsäuren oder Dicarbonsäuren, in größeren Mengen als bisher bekannt, geeignet sind sowie eines Verfahren zur Kultivierung ebendieser Stämme.

Die Aufgabe wird durch einen Hefestamm gemäß Anspruch 1 sowie durch Verfahren gemäß den Anspruchs 8 gelöst. Vorteilhafte Ausgestaltungen sind in den abhängigen Ansprüchen angegeben. Somit stellt die Erfindung folgendes bereit:
Einen Hefestamm der Art Yarrowia lipolytica, aufweisend eine Verringerung der Aktivität von Fao1p, kodiert von Gen FA01: YALIOB14014g, wobei der Hefestamm weiterhin eine Verringerung der Aktivitäten der Acyl-CoA-Oxidasen Gene POX1, POX2, POX3, POX4, POX5 und POX6 aufweist, wobei die Aktivitäten der Fao1p, POX1, POX2, POX3, POX4, POX5 und POX6 Proteine durch
(i) partielle oder komplette Deletion der kodierenden Gene fuer Fao1p, POX1, POX2, POX3, POX4, POX5 und POX6 oder
(ii) Expression von nicht-funktionalen Genen fuer Fao1p, POX1, POX2, POX3, POX4, POX5 und POX6 oder
(iii) Inhibierung oder Verminderung der Aktivität der exprimierten Gene fuer Fao1p, POX1, POX2, POX3, POX4, POX5 und POX6 durch Insertion, Substitution oder Punkmutation in dem für das Protein kodierenden Gen
   verringert werden.

Gemäß der Anmeldung wird zur mikrobiellen Produktion von *ω*-Hydroxyfettsäuren aus hydrophoben Substraten ein Hefestamm der Art *Y. lipolytica* bereitgestellt, der eine Verringerung der Aktivitäten der Acyl-CoA-Oxidasen (namentlich *POX1*: YALI0E32835g, *POX2*: YALI0F10857g, *POX3*: YALI0D24750g, *POX4*: YALI0E27654g, *POX5*: YALI0C23859g, *POX6*: YALI0E06567g) aufweist, wobei in diesem Hefestamm zusätzlich die Aktivitäten relevanter (Fett-)Alkohol-Dehydrogenasen (=(Fettsäure-)Alkohol-Dehydrogenasen, namentlich ausgewählt aus *FADH:* YALI0F09603g, *ADH1*: YALI0D25630g, *ADH2*: YALI0E17787g, *ADH3*: YALI0A16379g, *ADH4*: YALI0E15818g, *ADH5*: YALI0D02167g, *ADH6*: YALI0A15147g und/oder *ADH7*: YALI0E07766g) reduziert sind und die Aktivitäten relevanter (Fett-)Alkohol-Oxidasen (=(Fettsäure-)Alkohol-Oxidasen, namentlich *FAO1*: YALI0B14014g) reduziert sind, was zu einer gesteigerten Bildung von ω-Hydroxyfettsäuren, vorzugsweise lineare ω-Hydroxyfettsäuren gemäß der allgemeinen Struktur: HOOC-(CH₂)ₙ-CH₂OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 18, vorzugsweise im Bereich von 8 bis 16, aus einem hydrophoben Substrat, insbesondere einem entsprechenden *n*-Alkan oder einer Fettsäure (und anderen hydrophoben Substraten) führt.

Besonders bevorzugt ist die ω-Hydroxyfettsäure eine lineare ω-Hydroxyfettsäure gemäß der allgemeinen Struktur: HOOC-(CH₂)ₙ-CH₂OH mit n im Bereich von 8 bis 16.

Die Bildung von ω-Hydroxyfettsäuren bzw. Dicarbonsäuren ist im Sinne der vorliegenden Erfindung gesteigert, wenn diese gegenüber Hefezellen ohne die entsprechende gentechnische Veränderung um mindestens 10 Mol.-%, besonders bevorzugt mindestens 20 Mol.-% erhöht ist.

Der Erfindung liegt die jüngst gewonnene Erkenntnis zugrunde, dass das Gen, welches für die Fett-Alkohol-Oxidase *FAO1*: YALI0B14014g in dem Hefestamm der Art *Y. lipolytica* kodiert, identifiziert werden konnte.

Die Erfinder haben zudem nun überraschend gefunden, dass die Deletion des für das Enzym Fao1p kodierenden Gens die weitere ω-Oxidation der ω-Hydroxyfettsäuren zum Fettsäurealdehyd, bzw. zur Dicarbonsäure im Rahmen der ω-Oxidation von Fettsäuren vermindert.

Besonders vorteilhaft unterbindet oder verringert die Verringerung der Aktivität der Produkte von *FADH, ADH1, ADH2, ADH3, ADH4, ADH5, ADH6, ADH7* und/oder *FAO1* die weitere Oxidation der ω-Hydroxyfettsäuren zum Fettsäurealdehyd, bzw. zur Dicarbonsäure im Rahmen der ω-Oxidation von Fettsäuren.

Das Fett-Alkohol-Oxidase-Gen wurde in *Y. lipolytica* bisher noch nicht beschrieben. Dessen Nutzung im Rahmen von biotechnologischen Anwendungsfeldern stellt ebenfalls einen Teil dieser Erfindung dar.

Unter einer Verringerung der Aktivität eines Proteins ist vorzugsweise zu verstehen, dass die Aktivität des Proteins pro Zelle verringert wird. Besonders bevorzugt ist darunter das Fehlen der Aktivität des Proteins in der Hefezelle zu verstehen.
Die Verringerung der Aktivität von Proteinen kann durch verschiedene, dem Fachmann bekannte Verfahren erlangt werden, wie bspw.:
(i) durch die Inhibierung oder Verminderung der Expression der dafür kodierenden Gene
(ii) durch partielle oder komplette Deletion der dafür kodierenden Gene
(iii) durch Expression von nicht-funktionalen Genen
(iv) durch Inhibierung oder Verminderung der Aktivität der exprimierten Gene.

Die Inhibierung oder Verminderung der Expression eines für ein Protein kodierenden Gens kann beispielsweise durch Inhibierung oder Verminderung der Transkription des kodierenden Gens oder der Translation der gebildeten mRNA erfolgen. Die Deletion der kodierenden Gene kann beispielsweise durch einen Ausbau der Gene mittels Deletionskassetten durchgeführt werden. Die Expression eines dysfunktionalen oder aktivitätsreduzierten Genproduktes kann beispielsweise durch Insertion, Substitution oder Punktmutation in dem für das Protein kodierenden Gen bewerkstelligt werden.

Bei der Aktivitätsverringerung wird die Deletion eines kodierenden Gens bevorzugt.

Vorzugsweise weist der erfindungsgemäße Hefestamm zur Produktion von ω-Hydroxyfettsäuren somit eine Deletion der Gene für die Acyl-CoA-Oxidasen (*POX1, POX2, POX3, POX4, POX5* und *POX6*) sowie für die (Fett-)Alkohol-Dehydrogenasen (*FADH, ADH1, ADH2, ADH3, ADH4, ADH5, ADH6, ADH7*) und/oder für die (Fett-)Alkohol-Oxidasen (*FAO1*) auf. Weiterhin ist die Bildung der Fettkörperchen verringert, vorzugsweise durch die Verringerung der Aktivität der Genprodukte von *PAH1* und/oder *SCT1.*

Die Deletion der Gene *POX1, POX2, POX3, POX4, POX5* und *POX6* unterbindet die β-Oxidation und verhindert somit den Abbau von Fettsäuren (zu Acetyl-CoA, bzw. Propionyl-CoA).

Bevorzugt sind in dem erfindungsgemäßen Hefestamm zur mikrobiellen Herstellung von ω-Hydroxyfettsäuren zusätzlich die Aktivitäten einzelner oder aller Cytochrom P450-Genprodukte, insbesondere von *ALK1*: YALI0E25982g, *ALK2*: YALI0F01320g, *ALK3:* YALI0A20130g, *ALK4*: YALI0B13816g, *ALK5*: YALI0B13838g, *ALK6*: YALI0B01848g, *ALK7:* YALI0A15488g, *ALK8*: YALI0C12122g, *ALK9*: YALI0B06248g, *ALK10*: YALI0B20702g, *ALK11:* YALI0C10054g und/oder *ALK12:* YALI0A20130g erhöht. Vorteilhaft führt die Erhöhung einzelner oder aller Cytochrom P450-Genprodukte zu einer gesteigerten Bildung von ω-Hydroxyfettsäuren aus dem entsprechenden hydrophoben Substrat, insbesondere *n*-Alkanen und Fettsäuren.

Es kann vorteilhaft vorgesehen sein, dass in dem erfindungsgemäßen Hefestamm zur mikrobiellen Herstellung von ω-Hydroxyfettsäuren zusätzlich die Aktivität der NADPH-Cytochrom P450-Reduktase (*CPR1:* YALI0D04422g) erhöht ist, was zu einer gesteigerten Bildung von ω-Hydroxyfettsäuren aus dem entsprechenden hydrophoben Substrat, insbesondere *n*-Alkanen und Fettsäuren führt.

Bevorzugt ist in dem erfindungsgemäßen Hefestamm zur mikrobiellen Herstellung von ω-Hydroxyfettsäuren zusätzlich die Aktivität der Phosphatidsäure-Dephosphohydrolase (*PAH1:* YALI0D27016p) verringert. Vorteilhaft führt dies zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren.

Nach einer bevorzugten Ausgestaltung der vorliegenden Erfindung ist in dem erfindungsgemäßen Hefestamm zur mikrobiellen Herstellung von ω-Hydroxyfettsäuren zusätzlich die Aktivität der Glycerol-3-phosphat-Acyltransferase *(SCT1:* YALI0C00209g) verringert, was vorteilhaft zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren (und anderen hydrophoben Substraten) führt.

Es kann ferner auch vorgesehen sein, dass in dem Hefestamm zur mikrobiellen Herstellung von ω-Hydroxyfettsäuren zusätzlich die Aktivität der Phospholipid-Diacylglycerol-Acyltransferase (*LRO1*: YALI0E16797g) verringert ist, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Ferner wird offenbart dass in dem erfindungsgemäßen Hefestamm zur mikrobiellen Herstellung von ω-Hydroxyfettsäuren zusätzlich die Aktivität der Diacylglycerol-Acyltransferase (*DGA1:* YALI0E32769g) verringert ist, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Ferner wird offenbart dass in dem erfindungsgemäßen Hefestamm zur mikrobiellen Herstellung von ω-Hydroxyfettsäuren zusätzlich die Aktivität der Glycerol-3-phosphat-Dehydrogenase (*GUT2*: YALI0B13970g) erhöht ist, was zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Besonders vorteilhaft wurde nun gefunden, dass die zusätzlich Erhöhung der Aktivitäten relevanter Triacylglycerol-Lipasen (*TGL3*/*TGL4*: YALI0F10010g, YALI0D16379g, YALI0D17534g) in dem erfindungsgemäßen Hefestamm zur mikrobiellen Herstellung von ω-Hydroxyfettsäuren, zu einer Reduzierung der Größe der Fettkörperchen und somit zu einer gesteigerten Bildung von ω-Hydroxyfettsäuren aus dem entsprechenden hydrophoben Substrat, insbesondere *n*-Alkanen und Fettsäuren führt.

Besonders vorteilhaft hat sich die die Verringerung der Aktivität des Citrat- und Oxalacetat-Transporters (*YHM*2: YALI0B10736g) und/oder die Verringerung der Aktivität des Citrat-Transport-Proteins (*CTP1*: YALI0F26323g) in dem erfindungsgemäßen Hefestamm zur mikrobiellen Herstellung von ω-Hydroxyfettsäuren herausgestellt, da dadurch eine geringere Mengen an organischen Säuren produziert und/oder ins Kulturmedium sekretiert wird.

Optional kann vorgesehen sein, dass in dem erfindungsgemäßen Hefestamm zur mikrobiellen Herstellung von ω-Hydroxyfettsäuren zusätzlich der Transport von ω-Hydroxyfettsäuren aus der Hefezelle bzw. aus dem Endoplasmatisches Retikulum ins Cytoplasma, z.B. durch die erhöhte Aktivität spezifischer Membrantransporter, gesteigert ist, was zu einer gesteigerten Bildung von ω-Hydroxyfettsäuren aus dem entsprechenden hydrophoben Substrat, insbesondere *n*-Alkanen und Fettsäuren führt.

Bevorzugt ist in dem erfindungsgemäßen Hefestamm zur mikrobiellen Herstellung von ω-Hydroxyfettsäuren zusätzlich der Transport der hydrophoben Substrate in die Hefezelle bzw. die jeweiligen Organellen (Peroxisomen, Endoplasmatisches Retikulum), z.B. durch die erhöhte Aktivität spezifischer Membrantransporter, gesteigert ist, was zu einer gesteigerten Bildung von ω-Hydroxyfettsäuren aus dem entsprechenden hydrophoben Substrat, insbesondere *n*-Alkanen und Fettsäuren führt.

Die Erfindung betrifft auch die Verwendung des erfindungsgemäßen Hefestamm zur mikrobiellen Herstellung von ω-Hydroxyfettsäuren der allgemeinen Struktur: HOOC-(CH2)n-OH, HOOC-(CₙH₂ₙ₋₂)-CH₂OH, COOC-(CₙH₂ₙ₋₄)-CH2OH oder HOOC-(CₙH₂ₙ₋₆)-CH₂OH, mit n im Bereich von 6 bis 18, aus einem hydrophoben Substrat.

Ferner wird ein Hefestamm der Art *Y. lipolytica* offenbart zur mikrobiellen Produktion von Dicarbonsäuren aus *n*-Alkanen und Fettsäuren (und anderen hydrophoben Substraten) der eine Verringerung der Aktivitäten der Acyl-CoA-Oxidasen (namentlich *POX1*: YALI0E32835g, *POX2*: YALI0F10857g, *POX3*: YALI0D24750g, *POX4*: YALI0E27654g, *POX5*: YALI0C23859g, *POX6*: YALI0E06567g) aufweist und dadurch gekennzeichnet ist, dass in diesem Hefestamm zusätzlich die Aktivitäten relevanter (Fett-)Alkohol-Dehydrogenasen (namentlich ausgewählt aus *FADH*: YALI0F09603g, *ADH1*: YALI0D25630g, *ADH2*: YALI0E17787g, *ADH3*: YALI0A16379g, *ADH4:* YALI0E15818g, *ADH5:* YALI0D02167g, *ADH6:* YALI0A15147g und/oder *ADH7*: YALI0E07766g) und die Aktivitäten relevanter (Fett-)Alkohol-Oxidasen (namentlich *FAO1*: YALI0B14014g) erhöht sind, was zu einer gesteigerten Bildung von Dicarbonsäuren, vorzugsweise gemäß der allgemeinen Struktur: HOOC-(CH2)n-COOH, HOOC-(CₙH₂ₙ₋₂)-COOH, COOC-(CₙH₂ₙ₋₄)-COOH oder HOOC-(CₙH₂ₙ₋₆)-COOH, mit n im Bereich von 6 bis 18, vorzugsweise im Bereich von 8 bis 16, aus *n*-Alkanen und Fettsäuren (und anderen hydrophoben Substraten) führt.

Der offenbarte Hefestamm zur Produktion von Dicarbonsäuren weist vorzugsweise eine Deletion der Gene *POX1, POX2, POX3, POX4, POX5* und *POX6* auf. Außerdem weist er eine erhöhte Aktivität der Produkte von *FADH, ADH1, ADH2, ADH3, ADH4, ADH5, ADH6, ADH7* und/oder *FAO1* auf. Weiterhin ist die Bildung der Fettkörperchen verringert, vorzugsweise durch die Verringerung der Aktivität der Genprodukte von *PAH1* und/oder *SCT1.*

Besonders vorteilhaft verstärkt die Aktivitätssteigerung der Genprodukte von *FADH, ADH1, ADH2, ADH3, ADH4, ADH5, ADH6, ADH7* und/oder *FAO1* die weitere Oxidation der ω-Hydroxyfettsäuren zum Fettsäurealdehyd, bzw. zur Dicarbonsäure im Rahmen der ω-Oxidation von Fettsäuren, so dass die gewünschte Dicarbonsäuren verstärkt gebildet wird.

Unter einer erhöhten Aktivität eines Proteins ist vorzugsweise zu verstehen, dass die Aktivität pro Zelle erhöht ist, was durch die Steigerung der Proteinmenge oder durch die Steigerung der proteineigenen Aktivität erzielt werden kann. Die Steigerung der Proteinmenge ist dadurch gekennzeichnet, dass mehr Protein gebildet wird, als bei einem unveränderten Stamm unter gleichen Bedingungen. Die Steigerung der proteineigenen Aktivität ist dadurch gekennzeichnet, dass die gleiche Menge an Protein in einem veränderten Stamm eine höhere Aktivität aufweist als in einem unveränderten Stamm.

Eine erhöhte Aktivität der Proteine kann auf verschiedene Weise erzeugt werden:
(i) durch Einbringen des relevanten Gens in mehrfacher, vorzugsweise 8 bis 12facher, Kopie in das Genom des Organismus (Gen-Dosis-Effekt)
(ii) durch Austausch des natürlichen Promotors des relevanten Gens durch einen stärkeren als den natürlichen Promotor, vorzugsweise einen Promotor, der mindestens 50% der Aktivität des Promotors des Translationselongationsfaktor 1 alpha-Gens (*TEF1: YALI0C09141g*) besitzt, höchstvorzugsweise einen Promotor, der mindestens die Aktivität des *TEF1*-Promotors besitzt. Dabei können Promotoren gewählt werden, die entweder während der gesamten Kultivierung oder nur in einer gewünschten Wachstumsphase aktiv sind (Gen-Regulations-Effekt).

Bei der Aktivitätserhöhung wird das zusätzliche Einbringen des relevanten Gens unter Kontrolle eines starken Promotors besonders bevorzugt, vorzugsweise der *TEF1-*Promotor aus *Yarrowia lipolytica.*

Erfindungsgemäß sind die Hefestämme der Art *Y. lipolytica* zur Herstellung von ω-Hydroxyfettsäuren bzw. von Dicarbonsäuren nicht zum Abbau von Fettsäuren im Rahmen der β-Oxidation befähigt. Dies kann beispielsweise durch folgende Maßnahmen gewährleistet werden:
(i) Verringerung der Aktivität der Acyl-CoA-Oxidasen (*POX1*: YALI0E32835g, *POX2*: YALI0F10857g, *POX3*: YALI0D24750g, *POX4*: YALI0E27654g, *POX5*: YALI0C23859g, *POX6*: YALI0E06567g)
(ii) Verhinderung der Peroxisomenbiogenese, z.B. durch Verringerung der Aktivität der Peroxine (z.B. *PEX10*: YALI0C01023g)

Für die Produktion von ω-Hydroxyfettsäuren muss zusätzlich zur Verhinderung der β-Oxidation die Oxidation der ω-Hydroxyfettsäuren zum Fettsäure-Aldehyd, bzw. zur Dicarbonsäure im Rahmen der ω-Oxidation verhindert oder verringert werden. Dies kann folgendermaßen gewährleistet werden:
(i) Verringerung der Aktivität relevanter (Fett-)Alkohol-Dehydrogenasen (*FADH:* YALI0F09603g, *ADH1:* YALI0D25630g, *ADH2*: YALI0E17787g, *ADH3*: YALI0A16379g, *ADH4:* YALI0E15818g, *ADH5:* YALI0D02167g, *ADH6*: YALI0A15147g, *ADH7*: YALI0E07766g)
(ii) Verringerung der Aktivität relevanter (Fett-)Alkohol-Oxidasen (*FAO1*: YALI0B14014g)

Bevorzugt wird die Produktausbeute ferner durch die direkte Beeinflussung relevanter Stoffwechselwege erhöht, insbesondere sind diese:
(i) die Erhöhung der Aktivität der Cytochrom P450-Genprodukte (*ALK1*: YALI0E25982g, *ALK2*: YALI0F01320g, *ALK3:* YALI0A20130g, *ALK4*: YALI0B13816g, *ALK5:* YALI0B13838g, *ALK6:* YALI0B01848g, *ALK7:* YALI0A15488g, *ALK8*: YALI0C12122g, *ALK9:* YALI0B06248g, *ALK10*: YALI0B20702g, *ALK11:* YALI0C10054g, *ALK12:* YALI0A20130g)
(ii) die Erhöhung der Aktivität der NADPH-Cytochrom P450-Reduktase (*CPR1*: YALI0D04422g)

Nur für die Produktion von Dicarbonsäuren:
(iii) die Erhöhung der Aktivität relevanter (Fett-)Alkohol-Dehydrogenasen (*FADH:* YALI0F09603g, *ADH1:* YALI0D25630g, *ADH2*: YALI0E17787g, *ADH3:* YALI0A16379g, *ADH4:* YALI0E15818g, *ADH5:* YALI0D02167g, *ADH6:* YALI0A15147g, *ADH7*: YALI0E07766g)
(iv) die Erhöhung der Aktivität relevanter (Fett-)Alkohol-Oxidasen (*FAO1*: YALI0B14014g)
(v) die Erhöhung der Aktivität relevanter (Fett-)Aldehyd-Dehydrogenasen *(FALDH1:* YALI0A17875g, *FALDH2*: YALI0E15400g, *FALDH3*: YALI0B01298g, *FALDH4*: YALI0F23793g)

Im Rahmen unserer Untersuchungen wurde festgestellt, dass die Zellen im Verlauf der Kultivierung sehr große Fettkörperchen (*Lipid bodies*) bilden. Es ist davon auszugehen, dass die Fettsäuren (oder intrazellulär vorliegende Derivate dieser) mit Glycerol verestert und intrazellulär in den Fettkörperchen gespeichert werden. Dies verringert die letztendlich erzielte Produktausbeute.

Vorzugsweise führen gentechnische Veränderungen zu einer Ausbildung kleinerer Fettkörperchen und somit zu einer gesteigerten Produktausbeute, insbesondere sind diese:
(i) die Verringerung der Aktivität der Phosphatidsäure-Dephosphohydrolase (*PAH1*: YALI0D27016p)
(ii) die Verringerung der Aktivität der Phospholipid-Diacylglycerol-Acyltransferase (*LRO1*: YALI0E16797g)
(iii) die Verringerung der Aktivität der Diacylglycerol-Acyltransferase (*DGA1*: YALI0E32769g)
(iv) die Verringerung der Aktivität der Glycerol-3-phosphat-Acyltransferase (*SCT1*: YALI0C00209g)
(v) die Erhöhung der Aktivität der Glycerol-3-phosphat-Dehydrogenase (*GUT2*: YALI0B13970g)
(vi) die Erhöhung der Aktivität der Triacylglycerol-Lipasen (*TGL3*/*TGL4*: YALI0F10010g, YALI0D16379g, YALI0D17534g)

Insbesondere die Verringerung der Aktivität der Phosphatidsäure-Dephosphohydrolase, der Diacylglycerol-Acyltransferase und/oder der Glycerol-3-phosphat-Acyltransferase führen zu einer gesteigerten Produktausbeute, d.h. Herstellung von ω-Hydroxyfettsäuren bzw. Dicarbonsäuren.

Weiterhin werden der Transport der Substrate in die Zelle bzw. die jeweiligen Organellen (Peroxisomen, Endoplasmatisches Retikulum) sowie der Transport der Produkte (ω-Hydroxyfettsäuren und Dicarbonsäuren) aus der Zelle bzw. aus den jeweiligen Organellen gesteigert. Dies kann beispielsweise durch die Erhöhung der Aktivität spezifischer Transporter gewährleistet werden.

Nach einer besonders bevorzugten Ausgestaltung der vorliegenden Erfindung führt die Kombination mehrerer vorgenannter gentechnischer Veränderungen zu einer gesteigerten Produktausbeute, einem gesteigerten Ertrag und/oder einer gesteigerten Raum-Zeit-Ausbeute.

Als Ausgangsstamm für die Konstruktion eines erfindungsgemäßen Stammes kann ein Wildtypisolat der Hefe *Y. lipolytica* verwendet werden, vorzugsweise der Stamm H222. Der Stamm H222 wurde am 29.04.2013 bei der DSMZ (Deutsche Sammlung für Mikroorganismen und Zellkulturen GmbH, D-38142 Braunschweig) unter der Nummer DSM 27185 gemäß Budapester Vertrag über die internationale Anerkennung der Hinterlegung von Mikroorganismen für die Zwecke von Patentverfahren hinterlegt.

Zur Verwendung eines Stammes für die weitere gentechnische Bearbeitung ist ein Selektionsmarker notwendig. Dieser Selektionsmarker kann z.B. in Form der Uracilauxotrophie in an sich bekannter Weise in den Stamm eingebracht werden. Alternativ kann auf bereits bekannte uracilauxotrophe Stämme zurückgegriffen, vorzugsweise auf den Stamm H222-S4 (Mauersberger S, Wang HJ, Gaillardin C, Barth G & Nicaud JM (2001) J Bacteriol 183: 5102-5109.).

Eingebracht werden zusätzliche Gene oder Konstrukte, die vorhandene Gene ersetzen, vorzugsweise mittels üblicher Verfahren, die im Stand der Technik bekannt sind. Um eine effiziente Transkription der eingebrachten Gene zu gewährleisten, ist es bevorzugt, für den Organismus funktionierende Promotoren mit dem offenen Leserahmen der Gene zu verknüpfen. Hierbei können sowohl konstitutive Promotoren als auch von den Kultivierungsbedingungen abhängige Promotoren genutzt werden.

Bevorzugt sind die eingebrachten Gene, die für die genannten Enzyme kodieren, funktionell unter die Kontrolle eines konstitutiven Promotors gestellt, wobei die Promotoren identisch oder untereinander unterschiedlich sein können.

Vorzugsweise werden konstitutive Promotoren genutzt. Besonders bevorzugt wird der Promotor des Translationselongationsfaktors 1 alpha (pTEF1) verwendet.

Alternativ bevorzugt werden die eingebrachten Gene unter die Kontrolle eines, von den Kultivierungsbedingungen abhängigen Promotors gestellt. Vorzugsweise aktivieren Aktivatoren bzw. Induktoren den von den Kultivierungsbedingungen abhängigen Promotor (im Sinne der Anmeldung auch Operator genannt), indem sie direkt mit einem regulierbaren Promotor interagieren bzw. in dem sie an ein Repressorprotein binden, das sich daraufhin vom Promotor löst. Durch das Kontaktieren des erfindungsgemäßen Hefestamms mit einem Aktivator und/oder Induktor werden die o.g. Enzyme in dem erfindungsgemäßen Hefestamm synthetisiert und stehen somit für die biokatalytische Umsetzung eines hydrophoben Substrates, insbesondere eines entsprechenden *n*-Alkans oder einer Fettsäure, zur Verfügung.

In einer Ausgestaltung der Erfindung unterscheiden sich die von den Kultivierungsbedingungen abhängigen Promotoren voneinander, sodass die Promotoren Primärsignal-spezifisch aktivierbar sind. Vorteilhaft kann somit die Anwesenheit unterschiedlicher Aktivatoren und/oder Induktoren in dem Hefestamm zur Expression ausgewählter Gene führen, wodurch der Stress für eine rekombinante Hefezelle minimiert wird.

Die entsprechenden Nukleinsäuresequenzen für Promotoren (bspw. pTEF1, pPOX2, pPOT1, pXPR1, pICL1), die für ein erfindungsgemäßes Verfahren eingesetzt werden können, sind dem Fachmann bestens bekannt oder können bekannten Datenbanken entnommen werden. Vorteilhaft können für ein erfindungsgemäßes Verfahren neben künstlich in Organismen eingebrachte Promotoren ebenfalls natürliche Promotoren eingesetzt werden.

Ebenso ist es bevorzugt, den offenen Leserahmen der Gene mit einem Terminator zu versehen. Vorzugsweise wird der Terminator des Gens der Isocitratlyase (ICL1t) verwendet.

Offenbart wird auch ein Verfahren zur biokatalytischen Herstellung von ω-Hydroxyfettsäuren, vorzugsweise gemäß der allgemeinen Struktur: HOOC-(CH₂)ₙ-CH₂OH, HOOC-(CₙH₂ₙ₋₂)-CH₂OH, COOC-(CₙH₂ₙ₋₄)-CH₂OH oder HOOC-(CₙH₂ₙ₋₆)-CH₂OH, mit n im Bereich von 6 bis 18, vorzugsweise im Bereich von 8 bis 16, aus einem hydrophoben Substrat, das die folgenden Schritte umfasst:
a) Bereitstellung von Hefezellen mindestens eines Hefestamms der Art *Y. lipolytica,* der
   (i) eine Verringerung der Aktivitäten der Acyl-CoA-Oxidasen,
   (ii) eine reduzierte Aktivität relevanter (Fett-)Alkohol-Dehydrogenasen und
   (iii) eine reduzierte Aktivität relevanter (Fett-)Alkohol-Oxidasen
   aufweist, in einem geeigneten Kultivierungsmedium,
b) Kontaktieren der Hefezellen mit dem hydrophoben Substrat, wobei das hydrophobe Substrat mit mindestens einem der Enzyme der Hefezellen, ausgewählt aus der Gruppe,
   (i) Cytochrom P450-Genprodukte,
   (ii) NADPH-Cytochrom P450-Reduktase und
   (iii) (Fett-)Aldehyd-Dehydrogenasen zu dem Reaktionsprodukt ω-Hydroxyfettsäure umgesetzt wird
c) Isolierung der ω-Hydroxyfettsäure.

Besonders bevorzugt ist die ω-Hydroxyfettsäure eine lineare ω-Hydroxyfettsäure gemäß der allgemeinen Struktur: HOOC-(CH₂)ₙ-CH₂OH mit n im Bereich von 8 bis 16.

Nach einer bevorzugten Ausgestaltung des offenbarten Verfahrens zur biokatalytischen Herstellung von ω-Hydroxyfettsäuren kann vorgesehen sein, dass die Umsetzung zu dem Reaktionsprodukt ω-Hydroxyfettsäure zusätzlich mit in den verwendeten Hefezellen verbleibenden (Fett-)Alkohol-Oxidasen, (Fett-)Alkohol-Dehydrogenasen erfolgt.

Bevorzugt sind die Hefezellen mindestens eines Hefestamms in dem erfindungsgemäßen Verfahren zur biokatalytischen Herstellung von ω-Hydroxyfettsäuren der Art *Y. lipolytica* ausgewählt aus dem erfindungsgemäßen Hefestamm zur mikrobiellen Herstellung von ω-Hydroxyfettsäuren.

Überraschend hat sich gezeigt, dass nach der biokatalytischen Umsetzung des hydrophoben Substrates zu dem gewünschten Reaktionsprodukt ω-Hydroxyfettsäuren diese in den erfindungsgemäßen Hefezellen eines Hefestamms der Art *Y. lipolytica* nicht weiter verstoffwechselt (d.h. im Stoffwechsel des Ganzzellkatalysators abgebaut) werden und sich in der wässrigen Komponente anreichern. Vorteilhaft reichert sich die ω-Hydroxyfettsäure in Folge des Ausschleusens aus den Hefezellen in dem Kultivierungsmedium an.

Bevorzugt erfolgt das Kontaktieren des Hefestamms mit einem hydrophoben Substrat durch direkte Zugabe zur flüssigen Komponente in Form als Flüssigkeit und/oder Feststoff. Bei direkter Zugabe zum Hefestamm kann zudem eine organische Phase als Substratreservoir Anwendung finden, wodurch die Prozesslaufzeit optimiert werden kann.

Im Sinne der vorliegenden Erfindung wird unter einem hydrophoben Substrat ein an sich wasserunlöslicher Stoff verstanden, aufweisend ein gegebenenfalls verzweigtes und/oder ungesättigtes Kohlenwasserstoffgrundgerüst mit 8 bis 20 Kohlenstoffatomen. Bevorzugt ist das hydrophobe Substrat ausgewählt aus der Gruppe der *n*-Alkane der allgemeinen Struktur: H₃C-(CH₂)ₙ-CH₃ (bspw. Dodekan, Pentadekan, Hexadekan) oder Alkene der allgemeinen Struktur H₃C-(CₙH₂ₙ₋₂)-CH₃, H₃C-(CₙH₂ₙ₋₄)-CH₃ oder H₃C-(CₙH₂ₙ₋₆)-CH₃, wobei n eine ganze Zahl im Bereich von 6 bis 18 ist, der gegebenenfalls verzweigten gesättigten Fettsäuren der allgemeinen Struktur: HOOC-(CH₂)ₙ-CH₃, wobei n eine ganze Zahl im Bereich von 6 bis 18 ist (bspw. Dodekansäure, Hexadekansäure, Pentadekansäure, Phytansäure), der ungesättigten Fettsäuren der allgemeinen Struktur: HOOC-(CₙH₂ₙ₋₂)-CH₃, COOC-(CₙH₂ₙ₋₄)-CH₃ oder HOOC-(CₙH₂ₙ₋₆)-CH₃, wobei n eine ganze Zahl im Bereich von 6 bis 18 ist (bspw. Ölsäure, Linolsäure, Arachidonsäure), und Fettsäurealkylester der allgemeinen Struktur: ROOC-(CH₂)ₙ-CH₃, mit n im Bereich von 6 bis 18 und wobei R ein gegebenenfalls verzweigter C₁₋₄-Alkylrest ist.

Insbesondere bevorzugt aber nicht ausschließlich dienen vorgenannte *n*-Alkane, Alkene oder gesättigte bzw. ungesättigte Fettsäuren mit einer Kettenlänge (Anzahl der Kohlenstoffatome der längsten Kette) von acht bis achtzehn Kohlenstoffatomen, d.h. n im Bereich von 6 bis 16 als hydrophobes Substrat für die Biokonversion.

Nach einer alternativ bevorzugten Ausgestaltung der vorliegenden Erfindung dienen für die Biokonversion vorzugsweise aber nicht ausschließlich *n*-Alkane und Fettsäuren mit einer Kettenlänge von acht bis achtzehn Kohlenstoffatomen, d.h. n im Bereich von 6 bis 16, als hydrophobes Substrat.

Nach einer bevorzugten Ausgestaltung des erfindungsgemäßen Verfahrens beträgt die Konzentration des hydrophoben Substrats zu Beginn der Herstellung 1 bis 50 Gramm pro Liter Kulturmedium (g l⁻¹), besonders bevorzugt sind 5 bis 40 g l⁻¹.

Ferner offenbart ist ein Verfahren zur Herstellung von Dicarbonsäuren, vorzugsweise gemäß der allgemeinen Struktur: HOOC-(CH₂)ₙ-COOH, HOOC-(CₙH₂ₙ₋₂)-COOH, COOC-(CₙH₂ₙ₋₄)-COOH oder HOOC-(CₙH₂ₙ₋₆)-COOH, mit n im Bereich von 6 bis 18 vorzugsweise im Bereich von 8 bis 16, aus einem hydrophoben Substrat, insbesondere ausgewählt aus *n*-Alkanen und Fettsäuren, das die folgenden Schritte umfasst:
a) Bereitstellung von Hefezellen mindestens eines Hefestamms der Art *Y. lipolytica,* der
   (i) eine Verringerung der Aktivitäten der Acyl-CoA-Oxidasen,
   (ii) eine erhöhte Aktivität relevanter (Fett-)Alkohol-Dehydrogenasen und
   (iii) eine erhöhte Aktivität relevanter (Fett-)Alkohol-Oxidasen
   aufweist, in einem geeigneten Kultivierungsmedium,
b) Kontaktieren der Hefezellen mit dem hydrophoben Substrat, wobei das hydrophobe Substrat mit mindestens einem der Enzyme der Hefezellen, ausgewählt aus der Gruppe,
   (i) der Cytochrom P450-Genprodukte,
   (ii) der NADPH-Cytochrom P450-Reduktase,
   (iii) der (Fett-)Alkohol-Dehydrogenasen nach a),
   (iv) der (Fett-)Alkohol-Oxidasen nach a) und
   (v) (Fett-)Aldehyd-Dehydrogenase,
   zu dem Reaktionsprodukt Dicarbonsäure umgesetzt wird,
c) Isolierung der Dicarbonsäure.

Besonders bevorzugt ist die Dicarbonsäure eine lineare Dicarbonsäure gemäß der allgemeinen Struktur: HOOC-(CH₂)ₙ-CH₂OH mit n im Bereich von 8 bis 16.

Bevorzugt sind in den Hefezellen für das offenbarte Verfahren zur Herstellung von ω-Hydroxyfettsäuren oder Dicarbonsäuren zusätzlich die Aktivitäten einzelner oder aller Cytochrom P450-Genprodukte, insbesondere von *ALK1*: YALI0E25982g, *ALK2*: YALI0F01320g, *ALK3:* YALI0A20130g, *ALK4*: YALI0B13816g, *ALK5:* YALI0B13838g, *ALK6:* YALI0B01848g, *ALK7:* YALI0A15488g, *ALK8*: YALI0C12122g, *ALK9:* YALI0B06248g, *ALK10*: YALI0B20702g, *ALK11:* YALI0C10054g und/oder *ALK12*: YALI0A20130g erhöht. Vorteilhaft führt die Erhöhung einzelner oder aller Cytochrom P450-Genprodukte zu einer gesteigerten Bildung von ω-Hydroxyfettsäuren oder Dicarbonsäuren aus dem entsprechenden hydrophoben Substrat, insbesondere *n*-Alkanen und Fettsäuren.

Es kann vorteilhaft vorgesehen sein, dass in den Hefezellen für das erfindungsgemäße Verfahren zur Herstellung von ω-Hydroxyfettsäuren oder Dicarbonsäuren zusätzlich die Aktivität der NADPH-Cytochrom P450-Reduktase (*CPR1:* YALI0D04422g) erhöht ist, was zu einer gesteigerten Bildung von ω-Hydroxyfettsäuren oder Dicarbonsäuren aus dem entsprechenden hydrophoben Substrat, insbesondere *n*-Alkanen und Fettsäuren, führt.

Nach einer bevorzugten Ausgestaltung des offenbarten Verfahrens zur Herstellung von Dicarbonsäuren sind in den Hefezellen zusätzlich die Aktivitäten relevanter (Fetts-)Alkohol-Dehydrogenasen (namentlich *FADH:* YALI0F09603g, *ADH1:* YALI0D25630g, *ADH2*: YALI0E17787g, *ADH3:* YALI0A16379g, *ADH4:* YALI0E15818g, *ADH5:* YALI0D02167g, *ADH6:* YALI0A15147g, *ADH7*: YALI0E07766g) erhöht, was vorteilhaft zu einer gesteigerten Bildung von Dicarbonsäuren aus dem entsprechenden hydrophoben Substrat, insbesondere n-Alkanen und Fettsäuren führt.

Bevorzugt sind in den Hefezellen des offenbarten Verfahrens zur Herstellung von Dicarbonsäuren zusätzlich die Aktivitäten relevanter (Fett-)Alkohol-Oxidasen (*FAO1*: YALI0B14014g) erhöht, was zu einer gesteigerten Bildung von Dicarbonsäuren aus dem entsprechenden hydrophoben Substrat, insbesondere *n*-Alkanen und Fettsäuren führt
Nach einer bevorzugten Ausgestaltung des offenbarten Verfahrens zur Herstellung von Dicarbonsäuren, sind in den Hefezellen zusätzlich die Aktivitäten relevanter (Fett-)Aldehyd-Dehydrogenasen *(FALDH1:* YALI0A17875g, *FALDH2:* YALI0E15400g, *FALDH3:* YALI0B01298g, *FALDH4:* YALI0F23793g) erhöht, was zu einer gesteigerten Bildung von Dicarbonsäuren aus dem entsprechenden hydrophoben Substrat, insbesondere *n*-Alkanen und Fettsäuren führt.

Bevorzugt liegen die Ausbeuten von ω-Hydroxyfettsäuren bzw. Dicarbonsäuren in dem erfindungsgemäßen Verfahren für den Zeitraum von einem Tag (d.h. 24 h) im Bereich von 1 bis 20 g l⁻¹, besonders bevorzugt im Bereich von 4 bis 15 g l⁻¹.

Nach einer bevorzugten Ausgestaltung erfolgt die Herstellung von ω-Hydroxyfettsäuren und Dicarbonsäuren in einem Bioreaktor (Fermenter), in dem ein erfindungsgemäßer Hefestamm unter geeigneten Wachstumsbedingungen in einem geeigneten Kultivierungsmedium fermentiert wird.

Ferner offenbart ist Verfahren zur Herstellung von ω-Hydroxyfettsäuren und Dicarbonsäuren, die dadurch gekennzeichnet sind, dass ein erfindungsgemäßer Mikroorganismus in an sich bekannter Art und Weise in einem Bioreakor (Fermenter) kultiviert wird.

Bevorzugt erfolgt die Isolierung des Reaktionsproduktes ω-Hydroxyfettsäuren und Dicarbonsäuren durch Extraktion der wässrigen Komponente mit einem organischen Lösungsmittel ausgewählt aus der Gruppe der Phthalsäureester, besonders bevorzugt Ethylacetat, 1,2-Cyclo-hexandicarbonsäurediisononylester und Mesamoll®, und/oder der aliphatischen verzweigten und/oder linearen Kohlenwasserstoffe, vorzugsweise mit 5 bis 16 Kohlenstoffatomen, wie z.B. n-Pentan, Cyclopentan, n-Hexan, Cyclohexan, n-Heptan, n-Octan, Cyclooctan, n-Dekan, n-Dodekan oder n-Hexadekan. Bevorzugt dienen die genannten organischen Lösungsmittel in einem einphasigen, wässrigen System zur Extraktion nach dem Umsatz des hydrophoben Substrates. Alternativ dienen die genannten organischen Lösungsmittel in einem zweiphasigen System in Form einer zweiten Phase neben dem Kultivierungsmedium als Reservoir für das hydrophobe Substrat und/oder zur Abtrennung des Reaktionsprodukts.

Die Isolierung der Dicarbonsäuren und/oder ω-Hydroxyfettsäuren durch Extraktion der wässrigen Komponente mit einem organischen Lösungsmittel erfolgt vorzugsweise nach der Abtrennung der Hefezellen in Form von Biomasse von dem Kultivierungsmedium, wobei die Abtrennung der Hefezellen in Form von Biomasse von dem Kultivierungsmedium bevorzugt durch Zentrifugation oder Filtration erfolgt.

Bevorzugt erfolgt die Extraktion von den Reaktionsprodukten ω-Hydroxyfettsäuren und Dicarbonsäuren mit einem organischen Lösungsmittel bei einem pH-Wert zwischen 0 und 8, besonders bevorzugt zwischen 1 und 7, ganz besonders bevorzugt zwischen 1 und 6.

Gegebenenfalls erfolgt zur Reinigung der Dicarbonsäuren und/oder ω-Hydroxyfettsäuren im Anschluss an die Extraktion eine Destillation, wobei bevorzugt das organische Lösungsmittel abgetrennt wird. Bevorzugt erfolgt die Abtrennung des organischen Lösungsmittels durch Verdampfung bei einem Druck zwischen 0,1 und 1000 mbar, besonders bevorzugt zwischen 0,1 und 750 mbar, ganz besonders bevorzugt zwischen 1 und 400 mbar.

Bevorzugt ist in einem Hefestamm für das offenbarte Verfahren zur Herstellung von ω-Hydroxyfettsäuren bzw. Dicarbonsäuren zusätzlich die Aktivität der Phosphatidsäure-Dephosphohydrolase (*PAH1*: YALI0D27016p) verringert. Vorteilhaft führt dies zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren.

Nach einer bevorzugten Ausgestaltung des offenbartenn Verfahrens zur Herstellung von ω-Hydroxyfettsäuren bzw. Dicarbonsäuren ist in dem eingesetzten Hefestamm zusätzlich die Aktivität der Glycerol-3-phosphat-Acyltransferase (*SCT1*: YALI0C00209g) verringert, was vorteilhaft zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren (und anderen hydrophoben Substraten) führt.

Es kann ferner auch vorgesehen sein, dass in dem offenbarten Verfahren zur mikrobiellen Herstellung von Dicarbonsäuren bzw. ω-Hydroxyfettsäuren in den Hefezellen des eingesetzten Hefestamms zusätzlich die Aktivität der Phospholipid-Diacylglycerol-Acyltransferase (*LRO1*: YALI0E16797g) verringert ist, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Nach einer bevorzugten Ausgestaltung ist in dem erfindungsgemäßen Hefestamm zur mikrobiellen Herstellung von ω-Hydroxyfettsäuren bzw. Dicarbonsäuren zusätzlich die Aktivität der Diacylglycerol-Acyltransferase (*DGA1*: YALI0E32769g) verringert, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Nach einer bevorzugten Ausgestaltung des offenbarten Verfahrens zur Herstellung von ω-Hydroxyfettsäuren bzw. Dicarbonsäuren ist in den Hefezellen des entsprechend eingesetzten Hefestamms zusätzlich die Aktivität der Glycerol-3-phosphat-Dehydrogenase (*GUT2*: YALI0B13970g) erhöht, was zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Besonders vorteilhaft wurde nun gefunden, dass die zusätzlich Erhöhung der Aktivitäten relevanter Triacylglycerol-Lipasen (*TGL3*/*TGL4*: YALI0F10010g, YALI0D16379g, YALI0D17534g) in den verwendeten Hefezellen zur mikrobiellen Herstellung von ω-Hydroxyfettsäuren bzw. Dicarbonsäuren, zu einer Reduzierung der Größe der Fettkörperchen und somit zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Bevorzugt ist in den Hefezellen des für das offenbarten Verfahren zur Herstellung von ω-Hydroxyfettsäuren bzw. Dicarbonsäuren verwendeten Hefestamms zusätzlich der Transport der hydrophoben Substrate in die Hefezelle bzw. die jeweiligen Organellen (Peroxisomen, Endoplasmatisches Retikulum), z.B. durch die erhöhte Aktivität spezifischer Membrantransporter, gesteigert ist, was zu einer gesteigerten Bildung von ω-Hydroxyfettsäuren oder Dicarbonsäuren aus dem entsprechenden hydrophoben Substrat, insbesondere *n*-Alkanen und Fettsäuren führt.

Optional kann vorgesehen sein, dass in den Hefezellen zur mikrobiellen Herstellung von ω-Hydroxyfettsäuren bzw. Dicarbonsäuren zusätzlich der Transport von ω-Hydroxyfettsäuren und Dicarbonsäuren aus der Hefezelle bzw. aus dem Endoplasmatisches Retikulum ins Cytoplasma, z.B. durch die erhöhte Aktivität spezifischer Membrantransporter, gesteigert ist, was zu einer gesteigerten Bildung von ω-Hydroxyfettsäuren oder Dicarbonsäuren aus dem entsprechenden hydrophoben Substrat, insbesondere *n*-Alkanen und Fettsäuren und Anreicherung in dem Kulturmedium führt.

Besonders vorteilhaft hat sich die die Verringerung der Aktivität des Citrat- und Oxalacetat-Transporters (*YHM2*: YALI0B10736g) und/oder die Verringerung der Aktivität des Citrat-Transport-Proteins (*CTP1*: YALI0F26323g) in den Hefezellen für das erfindungsgemäße Verfahren zur mikrobiellen Herstellung von ω-Hydroxyfettsäuren bzw. Dicarbonsäuren herausgestellt, da dadurch eine geringere Mengen an organischen Säuren produziert und/oder ins Kulturmedium sekretiert wird.

Bevorzugt ist das Kultivierungsmedium ein Minimalmedium. Unter einem Minimalmedium wird im Sinne der vorliegenden Erfindung ein wässriges Kulturmedium verstanden, welches alle zum Wachstum der Hefezellen benötigten Substrate (bspw. C-Quelle, Mineralsalze, Spurenelemente, Vitamine) in gelöster Form enthält, welches aber zugleich keine komplexen Inhaltsstoffe bzw. komplexen Biomolekülen (bspw. Hefeextrakt, Pepton, Trypton usw.) aufweist. Dem Fachmann ist aus dem Stand der Technik eine Vielzahl von Minimalmedien zur Kultivierung von Hefezellen bekannt.

Neben dem angebotenen hydrophoben Substrat für die Biokonversion ist während der Kultivierung eine weitere Kohlenstoffquelle (C-Quelle; Substrat für die Energiegewinnung), auf deren Grundlage die Zelle Energie gewinnen kann, bereitzustellen.

Vorzugsweise sind dem Kultivierungsmedium daher als C-Quelle Mono- bzw. Disaccharide, bspw. Glukose, D-Fructose, Xylose, Maltose, Saccharose und/oder mehrwertige Alkohole, wie bspw. Glycerol, Rohglycerol und Mannit zugesetzt, besonders bevorzugt ist die zugesetzte Kohlenstoffquelle Glucose.

Insbesondere dient als Stickstoffquelle (N-Quelle) für die Kultivierung von Hefestämmen in dem erfindungsgemäßen Verfahren Ammonium/Ammoniak oder Aminosäuren wie Glutamin/Glutamat.

Bei der Kultivierung eines erfindungsgemäßen Stammes sind Kultivierungsbedingungen, unter denen die Fettkörperchen vergleichsweise gering und die Umsatzrate der angebotenen Substrate vergleichsweise groß ausgeprägt sind, zu bevorzugen.
Eine Limitierung des Wachstums der Zellen verhindert, dass zu große Mengen des eingesetzten Substrates in den Zellen gespeichert werden, was die Produktausbeute verringert, und dass eine Sauerstofflimitierung eintritt, die die Produktivität vermindert.

Eine mögliche Form der Limitierung ist die Stickstofflimitierung. Diese Limitierung führt in der Hefe *Y. lipolytica* zu einem Wachstumsstopp nach Aufbrauchen der Stickstoffquelle und zur Bildung von Citronensäure und Iso-Citronensäure. Vorzugsweise sollte der Stickstoffgehalt im Medium während der Produktionsphase, also nach dem Kontaktieren der Hefezellen mit dem hydrophoben Substrat, kleiner als 2 g l⁻¹, vorzugsweise kleiner als 1 g l⁻¹, hoch vorzugswiese kleiner 0,5 g l⁻¹, höchst vorzugsweise 0 g l⁻¹ sein. Die Produktionsphase ist dadurch gekennzeichnet, dass sie sich einer Phase starken Wachstums (Wachstumsphase) anschließt und nur noch (im Vergleich zur Wachstumsrate) geringes Wachstum, jedoch erhöhte Produktbildung aufweist.

Vorzugsweise erfolgt die Kultivierung im Fermenter unter kontrollierten Prozessparametern, wobei besonders bevorzugt der pH-Wert im Kultivierungsmedium (während einer bestimmten Wachstumsphase) konstant gehalten wird. Insbesondere bevorzugt erfolgt die Fermentation unter Limitationsbedingungen.

Der pH-Wert im Kultivierungsmedium sollte, nach dem Kontaktieren, also während der Wachstumsphase dem physiologisch bevorzugten von 5,5 entsprechen und während der Produktionsphase, also nach dem Kontaktieren der Hefezellen mit dem hydrophoben Substrat, vorzugsweise 5,5 - 9,0, besonders bevorzugt 7,0 - 9,0 und insbesondere bevorzugt 8,0 betragen.

Bevorzugt erfolgt die Kultivierung der Hefezellen unter physiologischen Bedingungen bei einer Temperatur zwischen 0 und 60 °C, bevorzugt zwischen 10 und 50 °C, besonders bevorzugt zwischen 20 und 40 °C, wobei der pH-Wert des Kultivierungsmediums zwischen 2,0 und 9,0, bevorzugt zwischen 5,5 und 9,0, besonders bevorzugt zwischen 7,0 und 9,0, insbesondere bevorzugt um die 8,0 liegt.

Vorzugsweise werden im offenbarten Verfahren die genannten Limitationsbedingungen miteinander kombiniert.

Offenbart wird auch ein auch ein Kit zur biokatalytischen Herstellung von Dicarbonsäuren oder ω-Hydroxyfettsäuren enthaltend:
a) mindestens einen Typ an Hefezellen, bevorzugt ein Typ erfindungsgemäß modifizierter Hefezellen des Hefestamms der Art *Y. lipolytica* in einer wässrigen Komponente und/oder
b) mindestens einen Typ kryokonservierter Hefezellen, bevorzugt eines Typs erfindungsgemäß modifizierter Hefezellen des Hefestamms der Art *Y. lipolytica.*

Die Biomasse in Form von Hefezellen für das offenbarte Verfahren zur biokatalytischen Herstellung von Dicarbonsäuren oder ω-Hydroxyfettsäuren kann durch eine dem Fachmann bekannte Weise der Vorkultivierung der entsprechend einzusetzenden Hefezellen (Anzucht auf Vollmedium und/oder Minimalmedium) erhalten werden, beispielsweise durch Kultivierung in Vollmedium, wie YPD-Medium (DSM-Medium No. 393), vorzugsweise jedoch durch Kultivierung in einem Medium, das die Erzeugung hoher Zelldichten, beispielsweise durch Kultivierung in Minimalmedium (bspw. 5 % (w/v) Glucose, 20 g l⁻¹ CaCO₃, 1 g l⁻¹ KH₂PO₄, 0,16 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid) insbesondere größer als 1 × 10⁹ Zellen pro ml, zulässt. Die Anzucht dieser Vorkultur der Hefezellen des erfindungsgemäßen Kits erfolgt vorzugsweise in laborüblichen Schüttelkolben, zur Erzeugung größerer Mengen an Biomasse ist aber auch die Anzucht unter kontrollierten Bedingungen im Fermenter möglich.

Ein weiterer Aspekt der Erfindung betrifft auch die Verwendung erfindungsgemäß modifizierter Hefezellen des Hefestamms der Art *Y. lipolytica,* ein erfindungsgemäßes Verfahren oder eines erfindungsgemäßes Kits.

Bevorzugte Weiterbildungen der Erfindung ergeben sich aus den Kombinationen der Ansprüche.

Nachfolgend soll die Erfindung anhand einiger Ausführungsbeispiele und zugehöriger Figuren eingehender erläutert werden. Die folgenden Beispiele dienen der weiteren Erläuterung der Erfindung, ohne die Erfindung auf diese zu beschränken.

Dabei zeigt:
- Fig. 1:: die Stoffwechselwege zum Abbau hydrophober Substrate durch *Y*. *lipolytica.*
- Fig. 2:: die Akkumulation von Dicarbonsäuren und ω-Hydroxyfettsäuren in *Y*. *lipolytica* H222ΔP, H222ΔPΔA und H222ΔPΔF unter Verwendung verschiedener hydrophober Substrate.
- Fig. 3:: die Biokonversion vom Pentadekansäure zu ω-Hydroxypentadekansäure durch *Y. lipolytica* H222ΔPΔF.
- Fig. 4:: die Ausbildung von Fettkörperchen bei *Y. lipolytica* H222ΔPΔF, H222ΔPΔFΔD, H222ΔPΔFΔH und H222ΔPΔFΔS.
- Fig. 5:: die Akkumulation von Dodekandisäure und ω-Hydroxydodekansäure bei der Kultivierung verschiedener Stämme der Hefe *Y. lipolytica* unter Verwendung von Dodekan als Substrat.
- Fig. 6:: die (Fett-)Alkohol-Dehydrogenase und -Oxidase-Enzymaktivität bei der Kultivierung verschiedener Stämme der Hefe *Y. lipolytica* unter Verwendung von Dodekan als Substrat.
- Fig. 7:: die Biokonversion vom Dodekan zu ω-Hydroxydodekansäure und Dodekandisäure durch *Y. lipolytica* H222ΔPΔF.

### Erläuterungen zu den genetischen Veränderungen:

| | |
|---|---|
| ΔP | Deletion der Acyl-CoA-Oxidase-Gene (*POX1-6*) |
| ΔF | Deletion des (Fett-)Alkohol-Oxidase-Gens (*FAO1*) |
| ΔA | Deletion der (Fett-)Alkohol-Dehydrogenase-Gene (*FADH*, *ADH1-7*) |
| ΔD | Deletion des Diacylglycerol-Acyltransferase-Gens (*DGA1*) |
| ΔH | Deletion des Phosphatidsäure-Dephosphohydrolase-Gens (*PAH1*) |
| ΔS | Deletion der Glycerol-3-phosphat-Acyltransferase-Gens (*SCT1*) |
| oC | Überexpression des NADPH-Cytochrom P450-Reduktase-Gens *CPR1* |
| oF | Überexpression der (Fett-)Alkohol-OxidaseGens *FAO1* |

### Erläuterungen zum Sequenzprotokoll

SEQ ID NO. 1: DNA-Sequenz des Plasmids pJET1.2/blunt (Fermentas).
SEQ ID NO. 2: DNA-Sequenz des Plasmids pUCBM21 (Boehringer Ingelheim).
SEQ ID NO. 3: DNA-Sequenz des artifiziellen Plasmids pUC-Lys2-DK2.
SEQ ID NO. 5: DNA-Sequenz des artifiziellen Plasmids pINTB_HMG1.

Die Fig. 1 zeigt, dass *n*-Alkane in die Zelle aufgenommen und dann im Rahmen der primären Alkanoxidation zur korrespondierenden Fettsäure mit gleicher Kettenlänge umgewandelt werden. Die Fettsäuren werden dann im Rahmen der β-Oxidation zu Acetyl-CoA (bzw. Propionyl-CoA) abgebaut. Die diterminale Oxidation von Fettsäuren (ω-Oxidation) kann parallel stattfinden. Die Namen der gebildeten chemischen Substanzen sind ebenso angegeben wie die Bezeichnungen der beteiligten Enzyme und der korrespondierenden Gene. *ALK1*: *YALI0E25982g, ALK2*: *YALI0F01320g, ALK3*: *YALI0A20130g, ALK4*: *YALI0B13816g, ALK5*: *YALI0B13838g, ALK6*: *YALI0B01848g, ALK7*: *YALI0A15488g, ALK8*: *YALI0C12122g, ALK9*: *YALI0B06248g, ALK10*: *YALI0B20702g, ALK11*: *YALI0C10054g, ALK12*: *YALI0A20130g, CPR1*: *YALI0D04422g, FADH*: *YALI0F09603g, ADH1*: *YALI0D25630g, ADH2*: *YALI0E17787g, ADH3*: *YALI0A16379g, ADH4*: *YALI0E15818g, ADH5*: *YALI0D02167g, ADH6*: *YALI0A15147g, ADH7*: *YALI0E07766g, ADH8*: *YALI0C12595g, FAO1*: *YALI0B14014g, FALDH1*: *YALI0A17875g, FALDH2*: *YALI0E15400g, FALDH3*: *YALI0B01298g, FALDH4*: *YALI0F23793g, FAA1*: *YALI0D17864g, POX1*: *YALI0E32835g, POX2*: *YALI0F10857g, POX3*: *YALI0D24750g, POX4*: *YALI0E27654g, POX5*: *YALI0C23859g, POX6*: *YALI0E06567g, MFE2*: *YALI0E15378g, POT1*: *YALI0E18568g.*

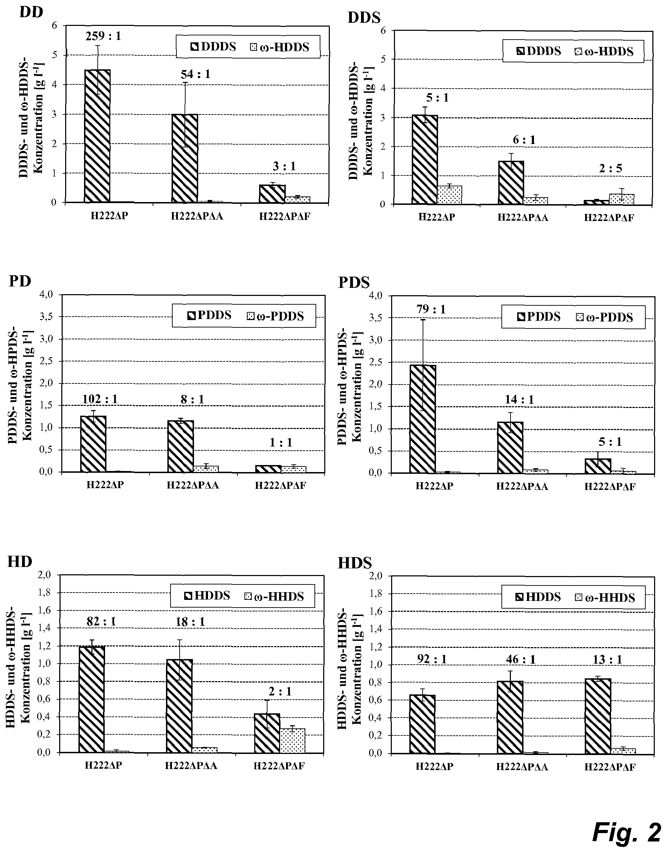

In Fig. 2 wurden *Y. lipolytica* H222ΔP, H222ΔPΔA und H222ΔPΔF in Minimalmedium mit Glycerol und verschiedenen *n*-Alkanen und Fettsäuren (DD: Dodekan; DDS: Dodekansäure; PD: Pentadekan; PDS: Pentadekansäure; HD: Hexadekan; HDS: Hexadekansäure) im Schüttelkolben kultiviert (3 % (v/v) Glycerol + 0,5 % (v/v) Glycerol nach 48 h, 1 % (v/v) *n*-Alkan oder 1 % (v/v) Fettsäure, 20 g l⁻¹ CaCO₃, 17,3 g l⁻¹ KH₂PO₄, 1,35 g I⁻¹ K₂HPO₄ x 3 H₂O, 3 g I⁻¹ (NH₄)₂SO₄, 0,7 g I⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x ² H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Mengen der gebildeten Dicarbonsäuren und ω-Hydroxyfettsäuren (DDDS: Dodekandisäure; ω-HDDS: ω-Hydroxydodekansäure; PDDS: Pentadekandisäure; ω-HPDS: ω-Hydroxypentadekansäure; HDDS: Hexadekandisäure; ω-HHDS: ω-Hydroxyhexadekansäure) wurden nach 96 h Kultivierung mittels Gaschromatographie bestimmt.

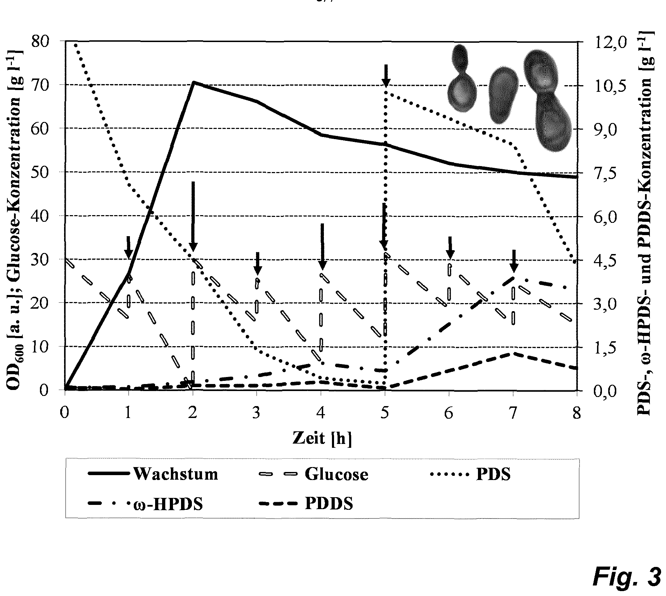

Bei Fig. 3 wurde *Y. lipolytica* H222ΔPΔF in Minimalmedium mit Glucose und Pentadekansäure (PDS) als Kohlenstoffquellen im Schüttelkolben kultiviert (3 % (v/v) Glucose, 1 % (w/v) PDS, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Glucosekonzentration wurde durch regelmäßiges Nachfüttern (Pfeile) zwischen 2 und 3 % (w/v) gehalten. Nach 5 Tagen wurde 1 % (w/v) Pentadekansäure nachgefüttert. Die gebildeten Pentadekansäurederivate, ω-Hydroxypentadekansäure (w HPDS) und Pentadekandisäure (PDDS), wurden mittels Gaschromatographie quantifiziert. Die Zellen wurden gegen Ende der Kultivierung mikroskopiert, wobei die Fettkörperchen mit Nilrot angefärbt wurden.

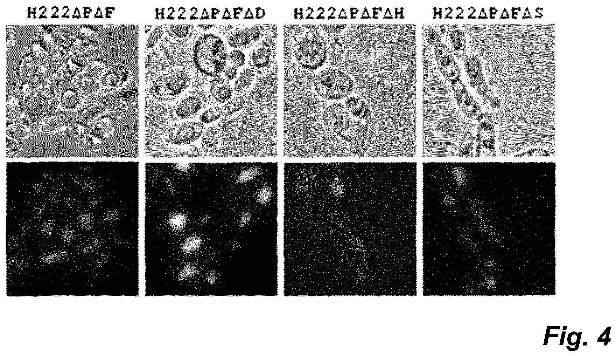

Fig. 4 zeigt die Kultivierung von *Y. lipolytica* H222ΔPΔF, H222ΔPΔFΔD, H222ΔPΔFΔH und H222ΔPΔFΔS in Minimalmedium mit Glucose und Pentadekansäure (PDS) als Kohlenstoffquellen (3% (v/v) Glucose, 1 % (w/v) PDS, 17,3 g I⁻¹ KH₂PO₄, 1,35 g l⁻¹ K₂HPO₄ x 3 H₂O, 3 g I⁻¹ (NH₄)₂SO₄, 0,7 g I⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Glucosekonzentration wurde durch regelmäßiges Nachfüttern zwischen 2 und 3% (w/v) gehalten. Nach 3 Tagen wurde 1% (w/v) Pentadekansäure nachgefüttert. Die Zellen wurden nach 5 Tagen mikroskopiert, wobei die Fettkörperchen mit Nilrot angefärbt wurden (oben: lichtmikroskopische Aufnahme; unten: fluoreszenzmikroskopische Aufnahme).

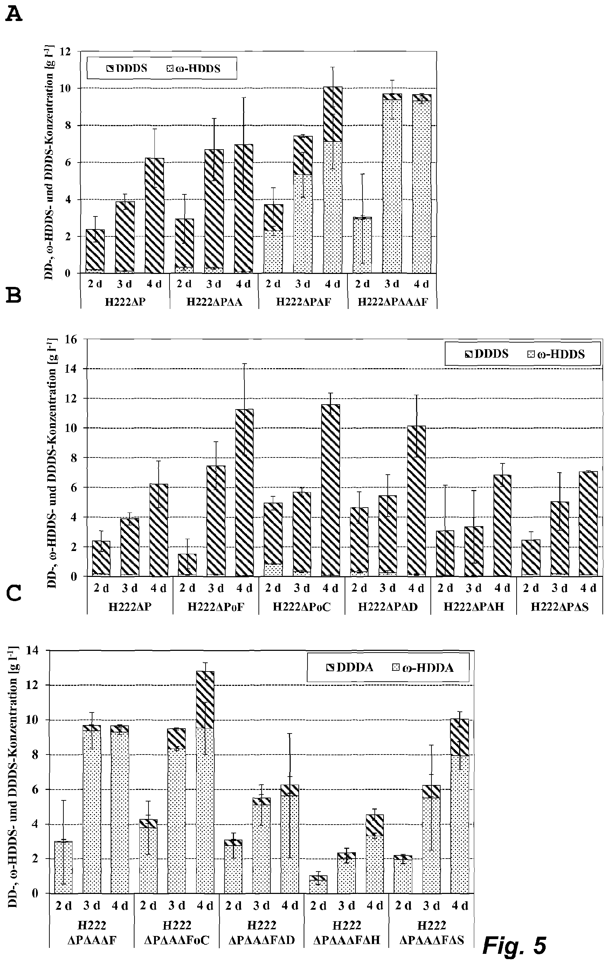

In Fig. 5 wurden *Y. lipolytica* H222ΔP, H222ΔPΔA, H222ΔPΔF, H222ΔPΔAΔF, H222ΔPoF, H222ΔPoC, H222ΔPΔD, H222ΔPΔH, H222ΔPΔS, H222ΔPΔAΔFoC, H222ΔPΔAΔFΔD, H222ΔPΔAΔFΔH und H222ΔPΔAΔFΔS im Fermenter kultiviert. Als Kultivierungmedium diente hierbei Minimalmedium mit Glucose (5 % (w/v) Glucose, 1 g l⁻¹ KH₂PO₄, 0,16 g l⁻¹ K2HPO4 x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 24 h bei pH 5,5 (automatisierte Titration von HCI und NaOH) inkubiert. Dann wurden 15 g l⁻¹ DD zugegeben und der pH wurde auf 8,0 eingestellt. Alle 24 h wurde Glucose zugegeben, um eine Endkonzentration von 5 % (w/v) einzustellen. Die Mengen der gebildeten Dodekandisäure (DDDS) und ω-Hydroxydodekansäure (ω-HDDS) wurden nach 2 d, 3 d und 4 d Kultivierung mittels Gaschromatographie bestimmt.

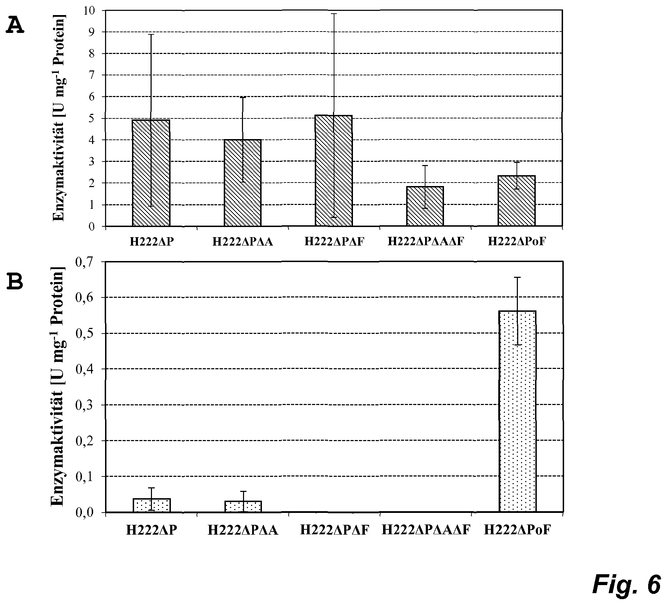

Fig. 6 zeigt die Enzymaktivitäten der (Fett-)Alkohol-Dehydrogenasen und -Oxidase in Zelllysaten der Stämme *Yarrowia lipolytica* H222ΔP, H222ΔPΔA, H222ΔPΔF, H222ΔPΔAΔF und H222ΔPoF. Die Zellen wurden wie in Beispiel 8 beschrieben kultiviert und nach drei Tagen geerntet und aufgeschlossen.

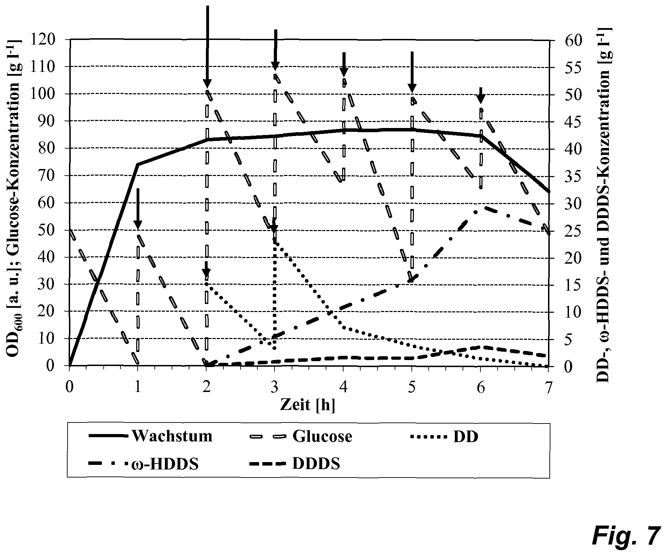

Bei Fig. 7 wurde *Y. lipolytica* H222ΔPΔF in Minimalmedium mit Glucose und Dodekan (DD) als Kohlenstoffquellen im Fermenter kultiviert (5 % (w/v) Glucose, 2 g l⁻¹ KH₂PO₄, 0,32 g l⁻¹ K₂HPO₄ x 3 H₂O, 6 g l⁻¹ (NH₄)₂SO₄, 1,4 g l⁻¹ MgSO₄ x 7 H₂O, 1 g l⁻¹ NaCl, 0,8 g l⁻¹ Ca(NO₃)₂ x 4 H₂O, 1 mg l⁻¹ H₃BO₃, 0,08 mg l⁻¹ CuSO₄ x 5 H₂O, 0,2 mg l⁻¹ KI, 0,8 mg l⁻¹ MnSO₄ x 4 H₂O, 0,4 mg l⁻¹ Na₂MoO₄ x 2 H₂O, 0,8 mg l⁻¹ ZnSO₄ x 7 H₂O, 12 mg l⁻¹ FeCl₃ x 6 H₂O, 0,6 mg l⁻¹ Thiaminhydrochlorid). Die Glucosekonzentration wurde alle 24 h durch regelmäßiges Nachfüttern (Pfeile) auf 5-10 % (w/v) eingestellt. Nach 2 d wurde der pH auf 8,0 eingestellt und es wurden nach 2 d und 3 d 15 g l⁻¹ Dodekan zugegeben. Alle 24 h wurde Glucose zugegeben, um eine Endkonzentration von 5-10 % (w/v) einzustellen.

### Beispiel 1: Verhinderung des Abbaus von Fettsäuren durch Blockierung der β-Oxidation

Für die Konstruktion der Deletionskassetten für die POX-Gene wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y. lipolytica* H222.
Durch Verwendung von Überhang-Primern wurde am Ende des Promotor- sowie am Anfang des Terminator-Bereichs eine I-*Sce*l-Restriktionsschnittstelle eingefügt. Promotor- und Terminator-Fragment wurden durch Overlap-PCR mit dem Primern pXXX_fw und XXXt_rv miteinander fusioniert. Das Overlap-Fragment wurde anschließend in den Vektor pJET1.2/blunt (Fermentas; SEQ ID NO 1) ligiert. Das resultierende Plasmid wurde mit I-Scel geschnitten und die IoxP-URA3-IoxP-Kassette, die aus dem pJMP113-Plasmid (Fickers P, Le Dall MT, Gaillardin C, Thonart P & Nicaud JM (2003) New disruption cassettes for rapid gene disruption and marker rescue in the yeast Yarrowia lipolytica. J Microbiol Methods 55: 727-737.) unter Verwendung des Restiktionsenzyms I-Scel gewonnen wurde, wurde eingefügt.

Die jeweilige Deletionskassette wurde durch PCR oder Restriktion gewonnen und in *Y. lipolytica* H222-S4, der aus *Y*. *lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker mithilfe des Cre-Iox-Rekombinationssystems zurückgewonnen (Fickers P, Le Dall MT, Gaillardin C, Thonart P & Nicaud JM (2003) New disruption cassettes for rapid gene disruption and marker rescue in the yeast Yarrowia lipolytica. J Microbiol Methods 55: 727-737.). Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette.
Der Stamm, der die Deletionen der Gene *POX1, POX2, POX3, POX4, POX5* und *POX6* trug, wurde H222ΔP genannt und bildet die Grundlage für die Konstruktion von Stämmen, die zur biotechnologischen Produktion ω-Hydroxyfettsäuren und Dicarbonsäuren genutzt werden können.

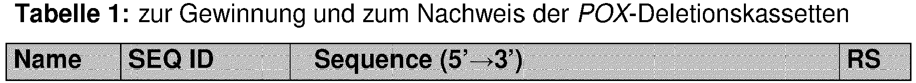

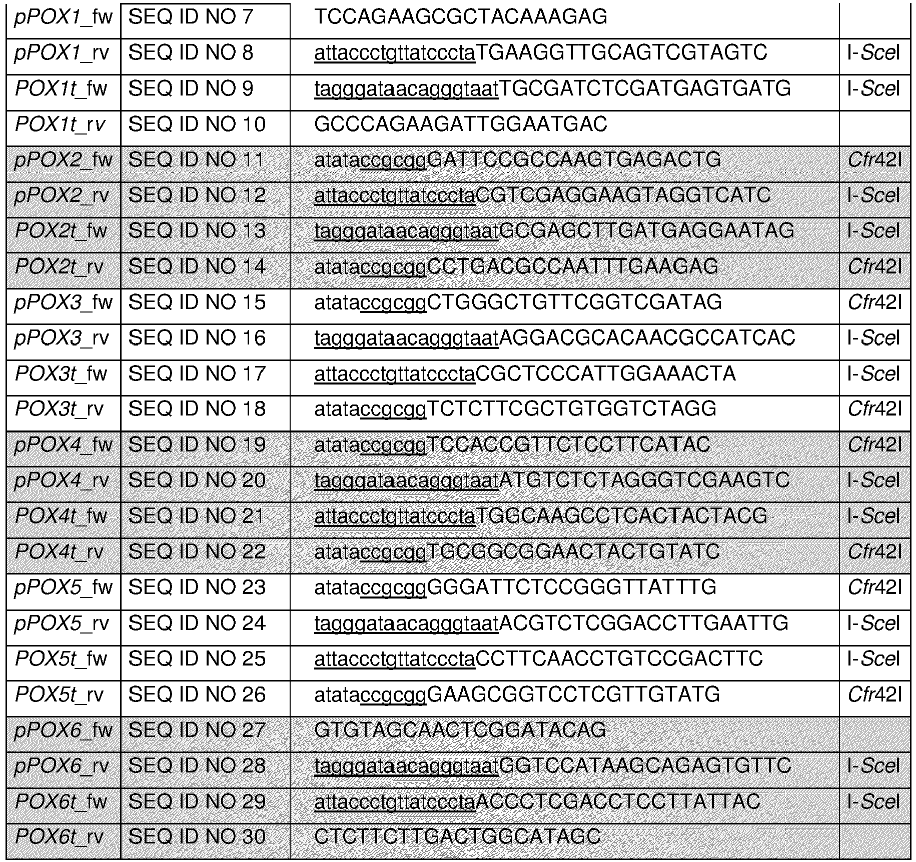

### Beispiel 2: Verhinderung der Oxidation der ω-Hydroxyfettsäuren zum Fettsäure-Aldehyd, bzw. zur Dicarbonsäure im Rahmen der ω-Oxidation

Für die Konstruktion der Deletionskassetten für die (Fett-)Alkohol-Dehydrogenase- und -Oxidase-Gene wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y. lipolytica* H222.

Für die Konstruktion der Deletionskassetten für die Gene *ADH1-ADH6* und *FAO1* wurde durch Verwendung von Überhang-Primern am Ende des Promotor- sowie am Anfang des Terminator-Bereichs eine *Bam*HI-Restriktionsschnittstelle eingefügt. Zusätzlich wurde am Anfang des Promotor-Bereichs eine *Hin*dIII-Restriktionsschnittstelle und am Ende des Terminator-Bereichs eine *Nde*l-Restriktionsschnittstelle (*ADH1*: *Not*I*, FAO1*: *EcoRI)* angefügt. Die Fragmente wurden in den Vektor pJET1.2/blunt (Fermentas; SEQ ID NO 1) oder pUCBM21 (Boehringer Ingelheim; SEQ ID NO 2) ligiert. Die so erhaltenen Plasmide wurden mit *Bam*HI linearisiert und der URA-Blaster (TcR-URA3-TcR-Kassette), der aus dem Plasmid pUC-Lys2-DK2(SEQ ID NO 3) durch Restriktion mit *Bam*HI und *Bg*/II gewonnen wurde, wurde eingefügt.

Für die Konstruktion des ADH7-Deletionsvektors wurde das vollständige Gen (inklusive des Promotor- und Terminator-Bereichs) mittels PCR amplifiziert, wobei die Primer pADH7_fw und ADH7t_rv verwendet wurden. Das erhaltene Fragment wurde in den Vektor pJET1.2/blunt (Fermentas; SEQ ID NO 1) ligiert, der offene Leserahmen des *ADH7-Gens* wurde durch Restriktion mit SanDI und *Nsi*I entfernt und der URA-Blaster, der aus dem Plasmid pUC-Lys2-DK2 durch Restriktion mit SanDI und *Nsi*I gewonnen wurde, wurde eingefügt.

Die jeweilige Deletionskassette wurde durch PCR oder Restriktion gewonnen und in *Y. lipolytica* H222-S4, der aus *Y. lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker per FOA-Selektion (Boeke JD, LaCroute F & Fink GR (1984) Mol Gen Genet 197: 345-346.) zurückgewonnen. Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette.
Der Stamm, der sowohl die Deletionen der Gene *POX1, POX2, POX3, POX4, POX5* und *POX6 als auch FADH, AHD1, AHD2, AHD3, AHD4, AHD5, AHD6* und *AHD7* trug, wurde H222ΔPΔA genannt.

Der Stamm, der sowohl die Deletionen der Gene *POX1, POX2, POX3, POX4, POX5* und *POX6* als auch *FAO1* trug, wurde H222ΔPΔF genannt.

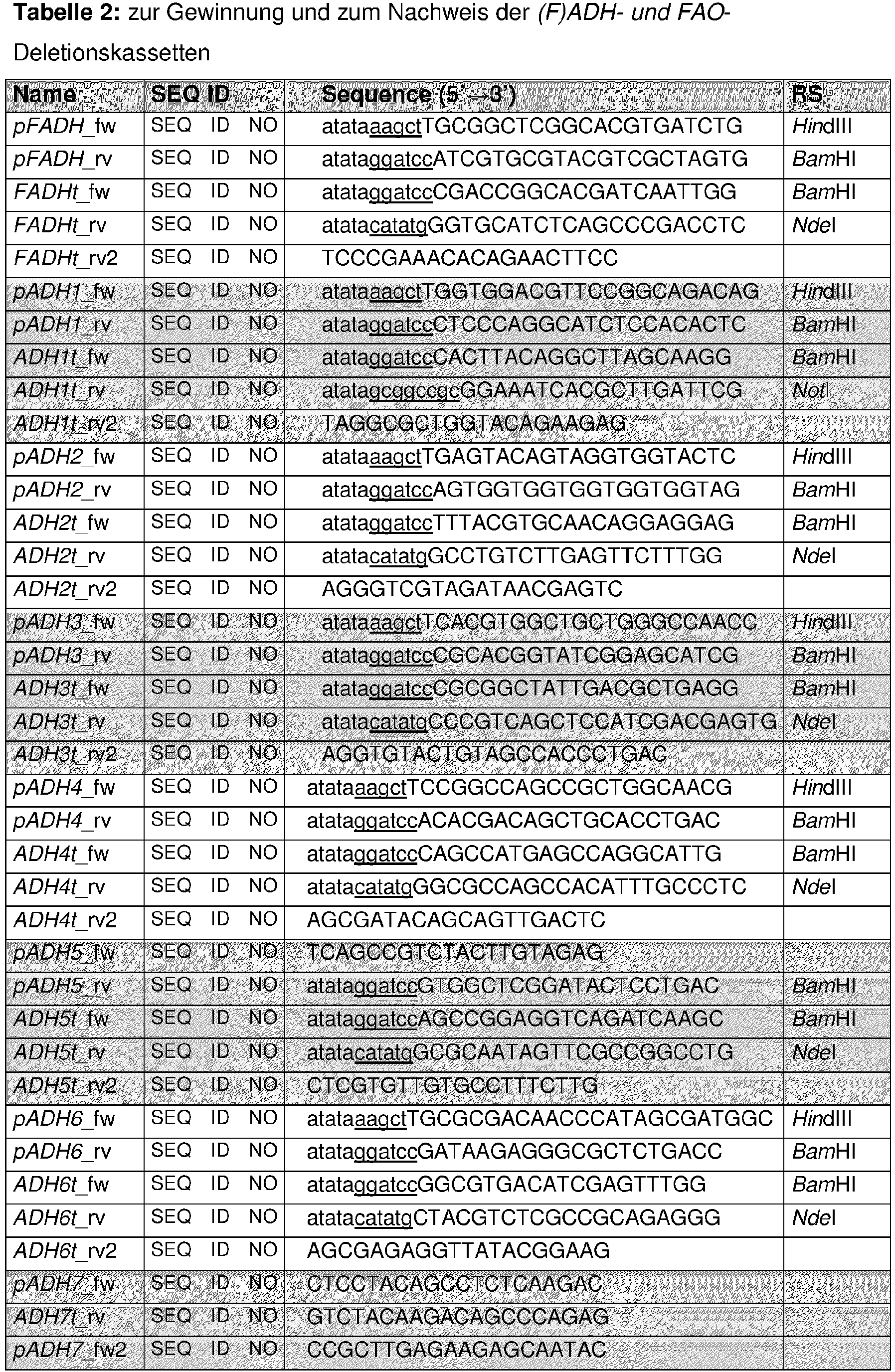

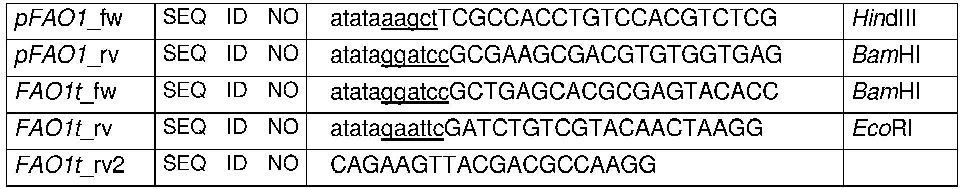

### Beispiel 3: Vergleichende Kultivierung verschiedener Produktionsstämme im Schüttelkolben

Um zu überprüfen, ob die erzeugten Ausgangsstämme *Yarrowia lipolytica* H222ΔP, H222ΔPΔA und H222ΔPΔF zur Produktion größerer Mengen an ω-Hydroxyfettsäuren und Dicarbonsäuren befähigt sind, wurden diese in Minimalmedium mit Glycerol und verschiedenen *n*-Alkanen (Dodekan, Pentadekan, Hexadekan) und Fettsäuren (Dodekansäure, Pentadekansäure, Hexadekansäure) kultiviert (3 % (v/v) Glycerol + 0,5 % (v/v) Glycerol nach 48 h, 1 % (v/v) *n*-Alkan oder 1 % (v/v) Fettsäure, 20 g l⁻¹ CaCO₃, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Mengen der gebildeten ω-Hydroxyfettsäuren (ω-Hydroxydodekansäure, ω-Hydroxypentadekansäure, ω-Hydroxyhexadekansäure) und Dicarbonsäuren (Dodekandisäure, Pentadekandisäure, Hexadekandisäure) wurden nach 96 h Kultivierung mittels Gaschromatographie bestimmt (Fig. 2).

*Y. lipolytica* H222ΔP bildete hierbei relativ große Mengen an Dicarbonsäuren und kann somit als Ausgangsstamm zur Konstruktion eines entsprechenden Produktionsstamms für Dicarbonsäuren genutzt werden.

Sowohl *Y. lipolytica* H222ΔPΔA als auch H222ΔPΔF bildeten gesteigerte Mengen an ω-Hydroxyfettsäuren und können somit als Ausgangsstämme zur Konstruktion entsprechender Produktionsstämme für ω-Hydroxyfettsäuren genutzt werden. *Y*. *lipolytica* H222ΔPΔF ist hierbei gegenüber H222ΔPΔA zu bevorzugen.

Sowohl *n*-Alkane als auch Fettsäuren können von allen Stämmen zur ω-Hydroxyfettsäure, bzw. Dicarbonsäure mit gleicher Kettenlänge umgesetzt werden.

### Beispiel 4: Nutzung verschiedener Kultivierungsbedingungen und -medien

Verschiedene Stämme (z.B. *Yarrowia lipolytica* H222ΔP und H222ΔPΔF) wurden in unterschiedlichen Medien unter verschiedenen Bedingungen kultiviert, wobei sich herausstellte, dass die verwendeten Medien und Bedingungen unterschiedlich gut geeignet sind.

Die Hefen wurden vergleichend in Minimalmedium mit Glucose (3 % (w/v) Glucose, 1 % (w/v) Pentadekansäure, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid) und Glycerol (3 % (v/v) Glycerol, 1 % (w/v) Pentadekansäure, 17,3 g l⁻¹ KH₂PO₄, 1,35 g l⁻¹ K2HPO4 x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid) als Kohlenstoffquelle kultiviert, wobei nach 48 h 1 % (w/v) Glucose, bzw. 1 % (v/v) Glycerol nachgefüttert wurden. Nach 96 h bildeten alle untersuchten Stämme (z.B. *Y. lipolytica* H222ΔP, H222ΔPΔA und H222ΔPΔF) bei Wachstum in Minimalmedium mit Glucose größere Mengen an ω-Hydroxypentadekansäure, bzw. Pentadekandisäure als bei Wachstum in Minimalmedium mit Glycerol.

Die Hefen wurden weiterhin in Vollmedium mit Glucose und Pentadekansäure kultiviert (1 % (w/v) Hefeextrakt, 2 % (w/v) Pepton, 2 % (w/v) Glucose und 1 % (w/v) Pentadekansäure in 1 % Tween 80), wobei 1 % (w/v) Glucose nachgefüttert wurden, wenn diese aufgebraucht war. Nach 4 Tagen wurden 1 % (w/v) Pentadekansäure in 1 % Tween 80 nachgefüttert. Unter diesen Bedingungen wurde nahezu keine ω-Hydroxypentadekansäure, bzw. Pentadekandisäure gebildet (je < 0,1 g l⁻¹).

Die Hefen wurden in Minimalmedium mit Glucose und Pentadekansäure kultiviert (3 % (v/v) Glucose, 1 % (w/v) Pentadekansäure in 1 % Tween 80, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid), wobei 1 % (w/v) Glucose nachgefüttert wurden, wenn diese aufgebraucht war. Nach 4 Tagen wurden 1 % (w/v) Pentadekansäure in 1 % Tween 80 nachgefüttert. Unter diesen Kultivierungsbedingungen bildete der Stamm *Y. lipolytica* H222ΔPΔF nach 10 Tagen rund 0,7 g l⁻¹ ω-Hydroxypentadekansäure und 2,4 g l⁻¹ Pentadekandisäure.

Die Hefen wurden in Minimalmedium mit Glucose und Pentadekansäure kultiviert (3 % (v/v) Glucose, 1 % (w/v) Pentadekansäure in 1 % Tween 80, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid), wobei die Glucose so nachgefüttert wurde, dass deren Konzentration im Medium zwischen 1 und 3 % (w/v) lag. Nach 5 Tagen wurden 1 % (w/v) Pentadekansäure in 1 % Tween 80 nachgefüttert. Unter diesen Kultivierungsbedingungen bildete der Stamm *Y. lipolytica* H222ΔPΔF nach 7 Tagen rund 3,9 g l⁻¹ ω-Hydroxypentadekansäure und 1,3 g l⁻¹ Pentadekandisäure.

Zusammenfassend ist festzustellen, dass Minimalmedium für die Kultivierung besser geeignet ist als Vollmedium. Weiterhin ist Glucose als Kohlenstoffquelle für die Energiebereitstellung besser geeignet als Glycerol. Die Glucose sollte im Verlauf der Kultivierung derart nachgefüttert werden, dass die Glucose nicht aufgebraucht wird (d. h. dass deren Konzentration nicht auf 0 g l⁻¹ absinkt).

### Beispiel 5: Reduktion der Bildung vom Fettkörperchen

Für die Konstruktion der Deletionskassetten für die Gene *DGA1, PAH1* und *SCT1* wurde der jeweilige Promotor- und Terminator-Bereich durch PCR amplifiziert, wobei die Primer pXXX_fw/pXXX_rv und XXXt_fw/XXXt_rv genutzt wurden (XXX steht für das zu deletierende Gen). Als Template diente hierbei genomische DNA von *Y*. *lipolytica* H222.

Für die Konstruktion der Deletionskassetten für die Gene *DGA1, PAH1* und *SCT1* wurde durch Verwendung von Überhang-Primern am Ende des Promotor- sowie am Anfang des Terminator-Bereichs eine *Bam*HI-Restriktionsschnittstelle eingefügt. Zusätzlich wurde am Anfang des Promotor-Bereichs eine *Hin*dIII-Restriktionsschnittstelle und am Ende des Terminator-Bereichs eine *Eco*RI-Restriktionsschnittstelle angefügt. Die Fragmente wurden in den Vektor pUCBM21 (Boehringer Ingelheim; SEQ ID NO 2) ligiert. Die so erhaltenen Plasmide wurden mit *Bam*HI linearisiert und der URA-Blaster (TcR-URA3-TcR-Kassette), der aus dem Plasmid pUC-Lys2-DK2(SEQ ID NO 3) durch Restriktion mit *Bam*HI und *Bg*/II gewonnen wurde, wurde eingefügt.

Die jeweilige Deletionskassette wurde durch PCR oder Restriktion gewonnen und in *Y. lipolytica* H222-S4, der aus *Y. lipolytica* H222 hergestellt werden kann (Mauersberger, S., H. J. Wang, et al. (2001), J Bacteriol 183(17): 5102-5109), nach Barth und Gaillardin (Barth G & Gaillardin C (1996) Yarrowia lipolytica. Springer-Verlag, Berlin, Heidelberg, New York.) transformiert.

Vor einer erneuten Transformation wurde der Marker per FOA-Selektion (Boeke JD, LaCroute F & Fink GR (1984) Mol Gen Genet 197: 345-346.) zurückgewonnen.

Die erfolgreiche Deletion eines Gens wurde durch PCR bestätigt, wobei die Primer pXXX_fw und XXXt_rv sowie pXXX_fw und XXXt_rv2 genutzt wurden. XXXt_rv2 bindet im Bereich des zu deletierenden Gens, jedoch außerhalb der Deletionskassette.

Der Stamm, der sowohl die Deletionen der Gene *POX1, POX2, POX3, POX4, POX5* und *POX6* als auch *DGA1* trug, wurde H222ΔPΔD genannt.

Der Stamm, der sowohl die Deletionen der Gene *POX1, POX2, POX3, POX4, POX5* und *POX6* als auch *PAH1* trug, wurde H222ΔPΔH genannt.

Der Stamm, der sowohl die Deletionen der Gene *POX1, POX2, POX3, POX4, POX5* und *POX6* als auch *SCT1* trug, wurde H222ΔPΔS genannt.

Der Stamm, der sowohl die Deletionen der Gene *POX1, POX2, POX3, POX4, POX5* und *POX6* als auch *FAO1* und *DGA1* trug, wurde H222ΔPΔFΔD genannt.

Der Stamm, der sowohl die Deletionen der Gene *POX1, POX2, POX3, POX4, POX5* und *POX6* als auch *FAO1* und *PAH1* trug, wurde H222ΔPΔFΔH genannt.

Der Stamm, der sowohl die Deletionen der Gene *POX1, POX2, POX3, POX4, POX5* und *POX6* als auch *FAO1* und *SCT1* trug, wurde H222ΔPΔFΔS genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *DGA1* trug, wurde H222ΔPΔAΔFΔD genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *PAH1* trug, wurde H222ΔPΔAΔFΔH genannt.

Der Stamm, der sowohl die Deletionen der Gene *POX1, POX2, POX3, POX4, POX5* und *POX6* als auch *FADH, ADH1, ADH2, ADH3, ADH4, ADH5, ADH6, ADH7, FAO1* und *SCT1* trug, wurde H222ΔPΔAΔFΔS genannt.

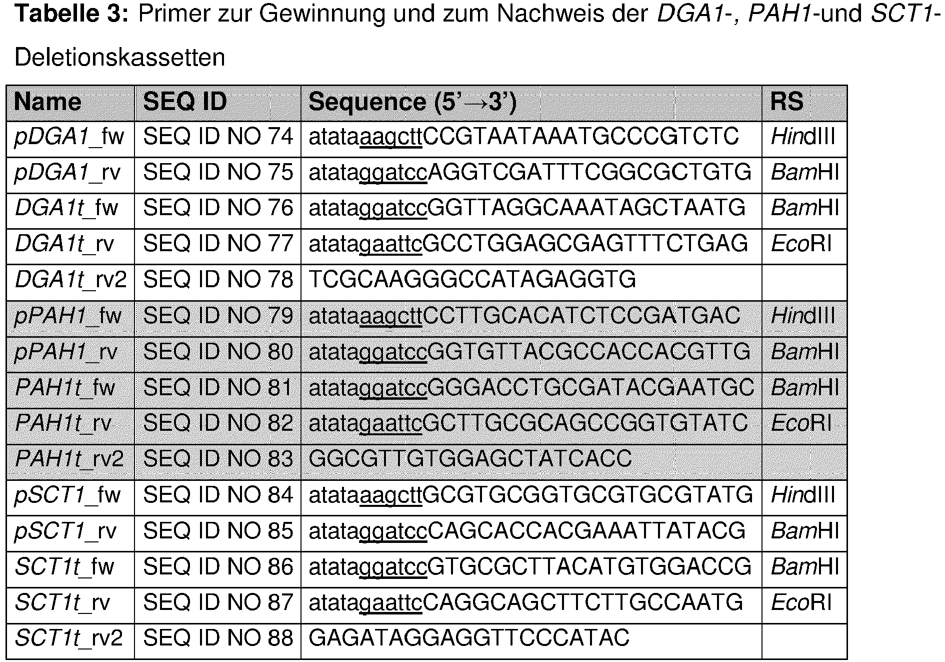

Die Stämme *Y. lipolytica* H222ΔPΔFΔD,H222ΔPΔFΔH und H222ΔPΔFΔS sowie deren Ausgangsstamm *Y. lipolytica* H222ΔPΔF wurden in Minimalmedium mit Glucose und Pentadekansäure kultiviert (3 % (v/v) Glucose, 1 % (w/v) Pentadekansäure, 17,3 g l⁻¹ KH₂PO₄, 1,35 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiamin-Hydrochlorid). Alle 24 h wurde die Glucose-Konzentration auf 3 % (w/v) eingestellt und nach 3 Tagen wurde 1 % (w/v) Pentadekansäure nachgefüttert. Die Mengen an ω-Hydroxypentadekansäure und Pentadekandisäure wurden nach 8 Tagen Kultivierung mittels Gaschromatographie bestimmt. Die Zellen wurden mikroskopisch untersucht, wobei die Fettkörperchen mit Nilrot angefärbt wurden und somit fluoreszenzmikroskopisch detektierbar sind (Fig. 4).

Hierbei ist zu erkennen, dass die Größe der Fettkörperchen durch die Deletionen von *PAH1* und *SCT1* unter den genannten Kultivierungsbedingungen deutlich reduziert war. Dies konnte, wie bereits gezeigt, nicht für die bereits beschriebene Deletion von *DGA1* (Thevenieau F (2006) Institut National Agronomique Paris-Grignon, PhD thesis.; Athenstaedt K (2011) Biochim Biophys Acta 1811: 587-596.)festgestellt werden.

Die Verfügbarkeit von Stämmen mit verkleinerten Fettkörperchen bildet die Grundlage für die Entwicklung von Stämmen für die effiziente Produktion von ω-Hydroxyfettsäuren und Dicarbonsäuren, da die Veresterung der angebotenen Substrate mit Glycerol und deren Einlagerung in den Fettkörperchen die Umsatzrate verringert.

### Beispiel 6: Erhöhte Expression von FAO1 in Yarrowia lipolytica H222ΔP

Für die Konstruktion eines Vektors zu Überexpression von *FAO1* wurde ein Teil des konstitutiven Promotors des Translationselongationsfaktor 1 alpha-Gens (*TEF1: YALI0C09141g*) mittels PCR gewonnen, wobei die Primer pTef_SpeI_fw3 und pTef_FAOo_ol_rv genutzt wurden. Als Template diente hierbei das Plasmid pINTB_HMG1 (SEQ ID NO 5).

Weiterhin wurde das *FAO1*-Gen(YALI0B14014g) mittels PCR unter Verwendung der Primer pTef_FAOo_ol_fw und FAO1o_SphI_rv amplifiziert (Template: genomische DNA von *Y. lipolytica* H222).

Durch die Primer pTef_FAOo_ol_rv und pTef_FAOo_ol_fw wurde ein Überhangbereich angefügt, mit dessen Hilfe beide PCR-Fragmente mittels Overlap-PCR unter Verwendung der Primer pTef_SpeI_fw3 und FAO1o_SphI_rv verbunden wurden. Das Overlap-Fragment wurde anschließend in den Vektor pJET1.2/blunt (Fermentas; SEQ ID NO 1) ligiert, aus diesem unter Verwendung der Restriktionsenzyme *Spe*I und *Sph*I herausgeschnitten und letztendlich in das mit *Spe*I und *Sph*I geschnittene Rückgrat des Plasmids pINTB_HMG1 (SEQ ID NO 5) ligiert, wobei des Plasmid pINTB-FAO1 entstand.

Weiterhin wurde eine Integrationplattform mittels PCR unter Verwendung der Primer INT_AscI_fw_KpnI und INT_AscI_rv_KpnI gewonnen, mit KpnI geschnitten und in das durch Restriktionsverdau mit Kpnl gewonnene Rückgrat des Vektors pINTB-FAO1 ligiert, wobei das Plasmid pINTC-FAO1 entstand.

Das Plasmid pINTC-FAO1 wurde letztendlich mit Ascl linearisiert und in den gewünschten Y. lipolytica-Empfängerstamm transformiert.

Die erfolgreiche Integration des FAO1-Gens wurde durch PCR bestätigt, wobei die Primer INT_AscI_fw_out und INT_AscI_rv_out genutzt wurden, die in der genomischen DNA außerhalb des integrierten Konstruktes binden.

Der Stamm, der sowohl die Deletionen der Gene POX1, POX2, POX3, POX4, POX5 und POX6 als auch die zusätzliche Kopie von FAO1 unter Kontrolle des TEF1-Promotors trug, wurde H222ΔPoF genannt und diente als Ausgangsstamm zu Konstruktion weiterer Stämme für die Produktion von Dicarbonsäuren.

**Tabelle 4: Primer für die Überexpression von FAO1**

| **Name** | **SEQ ID** | **Sequence (5'→3')** | **RS** |
|---|---|---|---|
| pTef_SpeI_fw3 | SEQ ID NO 89 | CTACGCTTGTTCAGACTTTG | |
| pTef_FAOo_ol_rv | SEQ ID NO 90 | | |
| pTef_FAOo_ol_fw | SEQ ID NO 91 | | |
| FAO1o_SphI_rv | SEQ ID NO 92 | gcatgcTTAGATTCGAGGTCGGAGAT | *Sph*I |
| INT_AscI_fw_KpnI | SEQ ID NO 93 | ggtacCACGCACGGATAGTTTATCCA | *Kpn*I |
| INT_AscI_rv_KpnI | SEQ ID NO 94 | | *Kpn*I |
| INT_AscI_fw_out | SEQ ID NO 95 | CCTCCAACGTGACTTTC | |
| INT_AscI_rv_out | SEQ ID NO 96 | AGAGACCTCCCACAAAG | |

### Beispiel 7: Erhöhte Expression von CPR1 in Yarrowia lipolytica H222ΔP und H222ΔPΔAΔF

Für die Konstruktion eines Vektors zu Überexpression von *FAO1* wurde ein Teil des konstitutiven Promotors des Translationselongationsfaktor 1 alpha-Gens (*TEF1*: *YALI0C09141g*) mittels PCR gewonnen, wobei die Primer pTef_SpeI_fw3 und pTEF_CPR1_ol_rv genutzt wurden. Als Template diente hierbei das Plasmid pINTB_HMG1 (SEQ ID NO 5).

Weiterhin wurde das *CPR1*-Gen(YALI0D04422g) mittels PCR unter Verwendung der Primer pTEF_CPR1_ol_fw und CPR1_SphI_rv amplifiziert (Template: genomische DNA von *Y. lipolytica* H222).

Durch die Primer pTEF_CPR1_ol_rv und pTEF_CPR1_ol_fw wurde ein Überhangbereich angefügt, mit dessen Hilfe beide PCR-Fragmente mittels Overlap-PCR unter Verwendung der Primer pTef_SpeI_fw3 und CPR1_SphI_rv verbunden wurden. Das Overlap-Fragment wurde anschließend in den Vektor pJET1.2/blunt (Fermentas; SEQ ID NO 1) ligiert, aus diesem unter Verwendung der Restriktionsenzyme *Spe*I und *Sph*I herausgeschnitten und letztendlich in das mit *Spe*I und *Sph*I geschnittene Rückgrat des Plasmids pINTB_HMG1 (SEQ ID NO 5) ligiert, wobei das Plasmid pINTB-CPR1 entstand.

Das resultierende Plasmid wurde letztendlich mit *Not*I linearisiert und in die gewünschten *Y*. *lipolytica*-Empfängerstämme transformiert.

Die erfolgreiche Integration des *CPR1*-Gens wurde durch PCR bestätigt, wobei die Primer INTB_out_fw und INTB_out_rv genutzt wurden, die in der genomischen DNA außerhalb des integrierten Konstruktes binden.

Der Stamm, der sowohl die Deletionen der Gene *POX1, POX2, POX3, POX4, POX5* und *POX6* als auch die zusätzliche Kopie von *CPR1* unter Kontrolle des *TEF*1-Promotors trug, wurde H222ΔPoC genannt und diente als Ausgangsstamm zu Konstruktion weiterer Stämme für die Produktion von Dicarbonsäuren.

Der Stamm, der sowohl die Deletionen der Gene *POX1, POX2, POX3, POX4, POX5, POX6, FADH, ADH1, ADH2, ADH3, ADH4, ADH5, ADH6, ADH7, FAO1* als auch die zusätzliche Kopie von *CPR1* unter Kontrolle des *TEF*1-Promotors trug, wurde H222ΔPΔAΔFoC genannt.

**Tabelle 5: Primer für die Überexpression von CPR1**

| **Name** | **SEQ ID** | **Sequence (5'→3')** | **RS** | |
|---|---|---|---|---|
| pTef_SpeI_fw3 | SEQ ID NO 89 | CTACGCTTGTTCAGACTTTG | | |
| pTEF_CPR1_ol_rv | SEQ ID NO 97 | | | |
| pTEF_CPR1_ol_fw | SEQ ID NO 98 | | | |
| CPR1_SphI_rv | SEQ ID NO 99 | | *Sph*I | |
| INTB_out_fw | SEQ ID NO | CTCAAGATACGGCATTGG | | |
| INTB_out_rv | SEQ ID NO | TCCTTGGCTAGACGAATG | | |

### Beispiel 8: Vergleichende Kultivierung verschiedener Produktionsstämme im Fermenter

Um zu überprüfen, inwieweit sich verschiedene gentechnische Modifikationen auf die Produktion von ω-Hydroxyfettsäuren und/oder Dicarbonsäuren auswirken, wurden die Stämme *Yarrowia lipolytica* H222ΔP, H222ΔPΔA, H222ΔPΔF, H222ΔPΔAΔF, H222ΔPoF, H222ΔPoC, H222ΔPΔD, H222ΔPΔH, H222ΔPΔS, H222ΔPΔAΔFoC, H222ΔPΔAΔFΔD, H222ΔPΔAΔFΔH und H222ΔPΔAΔFΔS, deren Konstruktion in den Beispielen 1,2,5,6 und 7 beschrieben wurde, im Fermenter kultiviert. Als Kultivierungmedium diente hierbei Minimalmedium mit Glucose (5 % (w/v) Glucose, 1 g l⁻¹ KH₂PO₄, 0,16 g l⁻¹ K₂HPO₄ x 3 H₂O, 3 g l⁻¹ (NH₄)₂SO₄, 0,7 g l⁻¹ MgSO₄ x 7 H₂O, 0,5 g l⁻¹ NaCl, 0,4 g l⁻¹ Ca(NO₃)₂ x 4 H₂O, 0,5 mg l⁻¹ H₃BO₃, 0,04 mg l⁻¹ CuSO₄ x 5 H₂O, 0,1 mg l⁻¹ KI, 0,4 mg l⁻¹ MnSO₄ x 4 H₂O, 0,2 mg l⁻¹ Na₂MoO₄ x 2 H₂O, 0,4 mg l⁻¹ ZnSO₄ x 7 H₂O, 6 mg l⁻¹ FeCl₃ x 6 H₂O, 0,3 mg l⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 24 h bei pH 5,5 (automatisierte Titration von HCl und NaOH) inkubiert. Dann wurden 15 g l⁻¹ DD zugegeben und der pH wurde auf 8,0 eingestellt. Alle 24 h wurde Glucose zugegeben, um eine Endkonzentration von 5 % (w/v) einzustellen.

*Y. lipolytica* H222ΔP bildete hierbei relativ große Mengen an Dicarbonsäuren und kann somit als Ausgangsstamm zur Konstruktion eines entsprechenden Produktionsstamms für Dicarbonsäuren genutzt werden (Fig. 5A).

*Y. lipolytica* H222ΔPΔA als auch H222ΔPΔF bildeten gesteigerte Mengen an ω-Hydroxyfettsäuren und können somit als Ausgangsstämme zur Konstruktion entsprechender Produktionsstämme für ω-Hydroxyfettsäuren genutzt werden. *Y*. *lipolytica* H222ΔPΔF ist hierbei gegenüber H222ΔPΔA zu bevorzugen(Fig. 5A).

*Y. lipolytica* H222ΔPΔAΔF bildete nur geringe Mengen Dicarbonsäuren und ist somit der bevorzugte Ausgangsstamm zur Konstruktion von Produktionsstämmen für ω-Hydroxyfettsäuren (Fig. 5A).

Die Überexpressionen von *FAO1* und *CPR1* in dem Stamm *Y. lipolytica* H222ΔP (resultierende Stämme: *Y. lipolytica* H222ΔPoF und H222ΔPoC) führte jeweils zu einer gesteigerten Produktion von Dicarbonsäuren (Fig. 5B).

Die Deletion von *DGA1* in dem Stamm *Y. lipolytica* H222ΔP (resultierender Stamm: *Y. lipolytica* H222ΔPΔD) führte zu einer gesteigerten Produktion von Dicarbonsäuren, wohingegen die Deletionen von *PAH1* und *SCT1* (resultierende Stämme: *Y. lipolytica* H222ΔPΔH und H222ΔPΔS) die Produktion von Dicarbonsäuren nicht signifikant steigerte (Fig. 5B).

Die Überexpression von *CPR1* in dem Stamm *Y. lipolytica* H222ΔPΔAΔF (resultierender Stamm: *Y. lipolytica* H222ΔPΔAΔFoC) führte zu einer gesteigerten Produktion von Dicarbonsäuren, nicht jedoch von ω-Hydroxyfettsäuren (Fig. 5C).

Die Deletionen von *DGA1, PAH1* und *SCT1* in dem Stamm *Y. lipolytica* H222ΔPΔAΔF (resultierende Stämme: *Y. lipolytica* H222ΔPΔAΔFΔD, H222ΔPΔAΔFΔH und H222ΔPΔAΔFΔS) führte nicht zu einer gesteigerten Produktion von ω-Hydroxyfettsäuren (Fig. 5B).

### Beispiel 9: Enzymtest zur Bestimmung der (Fett-)Alkohol-Dehydrogenase und - Oxidase-Aktivität

Die Enzymaktivitäten der (Fett-)Alkohol-Dehydrogenasen und -Oxidase wurden in Zelllysaten der Stämme *Yarrowia lipolytica* H222ΔP, H222ΔPΔA, H222ΔPΔF, H222ΔPΔAΔF und H222ΔPoF bestimmt. Die Zellen wurden wie in Beispiel 8 beschrieben kultiviert und nach drei Tagen geerntet und aufgeschlossen.

Die (Fett-)Alkohol-Dehydrogenase- und -Oxidase-Enzymaktivitäten wurden wie bei Matatiele (2005) beschrieben durchgeführt (Matatiele PR (2005) PhD thesis, University of the Free State, Republic of South Africa).

Das (Fett-)Alkohol-Dehydrogenase-Assay (50 mM Tris-HCI pH 8,5, 1,3 mM Dodekan-1-ol in DMSO, 2 mM NAD⁺, 2 mM NAP⁺, 1,5 % (v/v) Zellextrakt) wurde mit einem wurde mit einem aufzeichnenden Spektrophotometer bei 30 °C und λ = 340 nm eingemessen und die Enzymaktivität wurde mithilfe des bestimmten Extinktions-Anstiegs berechnet (ε_{NAD(P)H} = 6,3 mM⁻¹ cm⁻¹).

Das (Fett-)Alkohol-Oxidase-Assay (50 mM Glycin-NaOH pH 9,0, 0,35 mM Dodekan-1-ol in DMSO, 0,013 % (w/v) Peroxidase (150 U/mg), 0,044 % (w/v) ABTS, 6 mM Natriumazid, 1-5 % (v/v) Zellextrakt) wurde mit einem wurde mit einem aufzeichnenden Spektrophotometer bei λ = 405 nm eingemessen und die Enzymaktivität wurde mithilfe des bestimmten Extinktions-Anstiegs berechnet (ε_{ABTSox} = 18,4 mM⁻¹ cm⁻¹).
Überraschenderweise wurde zwischen den Stämmen keinen deutlicher Unterschied der (Fett-)Alkohol-Dehydrogenase festgestellt (Fig. 6A). Dies könnte mit verbleibender Alkohol-Dehydrogenase-Aktivität begründet werden. Weiterhin ist es möglich, dass die gemessene Umsetzung Dodekan-1-ol auch durch Cytochrom P450 katalysiert und letztendlich die Aktivität von Aldehyd-Dehydrogenasen bestimmt wird.

Wie erwartet war die (Fett-)Alkohol-Oxidase-Aktivität nur in den Stämmen *Y*. *lipolytica* H222ΔP und H222ΔPΔA nachweisbar und nicht in den *FAO1-*Deletionsstämmen *Y. lipolytica* H222ΔPΔF und H222ΔPΔAΔF (Fig. 6B). Die gemessene (Fett-)Alkohol-Oxidase-Aktivität war durch die Überexpression von *FAO1* (Stamm: *Y. lipolytica* H222ΔPoF) mehr als zehnfach erhöht.

### Beispiel 9: Kultivierung Y. lipolytica H222ΔPΔF im Fermenter

Um zu überprüfen, ob die Produktionsstämme in der Lage sind, auch größere Mengen an Dicarbonsäuren und/oder ω-Hydroxyfettsäuren zu produzieren und im Kulturmadium anzuhäufen, wurde der Stamm *Y. lipolytica* H222ΔPΔF für 7 d im Fermenter kultiviert. Als Kultivierungmedium diente hierbei Minimalmedium mit Glucose und einer erhöhten Menge an Mineralsalzen, Spurenelementen und Vitaminen (5 % (w/v) Glucose, 2 g l⁻¹ KH₂PO₄, 0,32 g l⁻¹ K₂HPO₄ x 3 H₂O, 6 g l⁻¹ (NH₄)₂SO₄, 1,4 g l⁻¹ MgSO₄ x 7 H₂O, 1 g l⁻¹ NaCl, 0,8 g l⁻¹ Ca(NO₃)₂ x 4 H₂O, 1 mg l⁻¹ H₃BO₃, 0,08 mg l⁻¹ CuSO₄ x 5 H₂O, 0,2 mg l⁻¹ KI, 0,8 mg l⁻¹ MnSO₄ x 4 H₂O, 0,4 mg l⁻¹ Na₂MoO₄ x 2 H₂O, 0,8 mg l⁻¹ ZnSO₄ x 7 H₂O, 12 mg l⁻¹ FeCl₃ x 6 H₂O, 0,6 mg l⁻¹ Thiaminhydrochlorid). Die Kultivierung erfolgte im Fermenter bei 28 °C. Die Sauerstoffsättigung wurde auf 55 % eingestellt. Die Zellen wurden mit einer OD₆₀₀ von 1 inokuliert und für 2 d bei pH 5,5 (automatisierte Titration von HCl und NaOH) inkubiert. Nach 2 d wurde der pH auf 8,0 eingestellt und es wurden nach 2 d und 3 d 15 g l⁻¹ Dodekan zugegeben. Alle 24 h wurde Glucose zugegeben, um eine Endkonzentration von 5-10 % (w/v) einzustellen.

Nach 6 d Kultiviernung (d.h. nach 4 d in der Produktionsphase) bildete *Y. lipolytica* H222ΔPΔF 29,5 g l⁻¹ ω-Hydroxydodekansäure und 3,5 g l⁻¹ Dodekandisäure (Fig. 7).

## Patentansprüche

1. Ein Hefestamm der Art *Yarrowia lipolytica,* aufweisend eine Verringerung der Aktivität von Fao1p, kodiert von Gen *FA01: YALIOB14014g,* wobei der Hefestamm weiterhin eine Verringerung der Aktivitäten der Acyl-CoA-Oxidasen Gene POX1, POX2, POX3, POX4, POX5 und POX6 aufweist, wobei die Aktivitäten der Fao1p, POX1, POX2, POX3, POX4, POX5 und POX6 Proteine durch
(i) partielle oder komplette Deletion der kodierenden Gene für Fao1p, POX1, POX2, POX3, POX4, POX5 und POX6 oder
(ii) Expression von nicht-funktionalen Genen für Fao1p, POX1, POX2, POX3, POX4, POX5 und POX6 oder
(iii) Inhibierung oder Verminderung der Aktivität der exprimierten Gene für Fao1p, POX1, POX2, POX3, POX4, POX5 und POX6 durch Insertion, Substitution oder Punktmutation in dem für das Protein kodierenden Gen
verringert werden.

2. Hefestamm nach Anspruch 1, **dadurch gekennzeichnet, dass** die Gene für Fao1p, *POX1, POX2, POX3, POX4, POX5* und *POX6* deletiert sind.

3. Hefestamm nach einem der Ansprüche 1 oder 2 **dadurch gekennzeichnet, dass** die Gene für die Fett-Alkohol-Dehydrogenasen FADH, ADH1, ADH2, ADH3, ADH4, ADH5, ADH6 und/oder ADH7 deletiert sind.

4. Hefestamm nach einem der Ansprüche 1 to 3, **dadurch gekennzeichnet, dass** zusätzlich die Aktivitäten einzelner oder aller Cytochrom P450-Genprodukte *ALK1, ALK2, ALK3, ALK4, ALK5, ALK6, ALK7, ALK8, ALK9, ALK10, ALK11* und/oder *ALK12* pro Zelle erhöht sind, wobei die erhöhte Aktivität der Proteine durch
(i) Einbringen des Gens des Cytochrom P450-Genprodukts in mehrfacher, vorzugsweise 8 bis 12-facher, Kopie in das Genom des Organismus oder
(ii) Austausch des natürlichen Promoters des Gens des Cytochrom P450- Genprodukts durch einen stärkeren als den natürlichen Promoter, vorzugsweise einen Promoter, der mindestens 50% der Aktivität des Promoters des Translationselongationsfaktor 1 alpha-Gens *TEF1* besitzt, höchstvorzugsweise einen Promoter, der mindestens die Aktivität des *TEF1*-Promotors besitzt,
erfolgt.

5. Hefestamm nach einem der Ansprüche 1 bis 4, **dadurch gekennzeichnet, dass** zusätzlich die Aktivität der NADPH-Cytochrom P450-Reduktase *CPR1* erhöht ist, wobei die erhöhte Aktivität der NADPH-Cytochrom P450-Reduktase durch
(i) Einbringen des Gens der NADPH-Cytochrom P450-Reduktase *CPR1* in mehrfacher, vorzugsweise 8 bis 12-facher, Kopie in das Genom des Organismus oder
(ii) Austausch des natürlichen Promoters des Gens der NADPH-Cytochrom P450-Reduktase *CPR1* durch einen stärkeren als den natürlichen Promoter, vorzugsweise einen Promoter, der mindestens 50% der Aktivität des Promoters des Translationselongationsfaktor 1 alpha-Gens *TEF1* besitzt, höchstvorzugswelse einen Promoter, der mindestens die Aktivität des *TEF1*-Promotors besitzt,
erfolgt.

6. Hefestamm nach einem der Ansprüche 1 bis 5, **dadurch gekennzeichnet, dass** zusätzlich die Aktivität der Phosphatidsäure-Dephosphohydrolase *PAH1* verringert ist, wobei die Aktivität der Phosphatidsäure-Dephosphohydrolase *PAH1* durch
(i) die Inhibierung oder Verminderung der Expression der kodierenden Gene der Phosphatidsäure-Dephosphohydrolase *PAH1* oder
(ii) partielle oder komplette Deletion der kodierenden Gene der Phosphatidsäure-Dephosphohydrolase *PAH1* oder
(iii) Expression von nicht-funktionalen Genen der Phosphatidsäure-Dephosphohydrolase *PAH1* oder
(iv) Inhibierung oder Verminderung der Aktivität der exprimierten Gene der Phosphatidsäure-Dephosphohydrolase *PAH1*
verringert wird.

7. Hefestamm nach einem der Ansprüche 1 bis 6, **dadurch gekennzeichnet, dass** zusätzlich die Aktivität der Glycerol-3-phosphat-Acyltransferase *SCT1* verringert ist, wobei die Aktivität der Glycerol-3-phosphat-Acyltransferase *SCT1* durch
(i) die Inhibierung oder Verminderung der Expression der kodierenden Gene der Glycerol-3-phosphat-Acyltransferase Gen SCT1 oder
(ii) partielle oder komplette Deletion der kodierenden Gene der Glycerol-3-phosphat-Acyltransferase Gen SCT1 oder
(iii) Expression von nicht-funktionalen Genen der Glycerol-3-phosphat-Acyltransferase Gen SCT1 oder
(iv) Inhibierung oder Verminderung der Aktivität der exprimierten Gene der Glycerol-3-phosphat-Acyltransferase Gen SCT1
verringert wird.

8. Verfahren zur biokatalytischen Herstellung von ω-Hydroxyfettsäuren aus einem hydrophoben Substrat, das die folgenden Schritte umfasst:
a) Bereitstellung von Hefezellen mindestens eines Hefestamms der Art *Yarrowia lipolytica* nach einem der Ansprüche 1 bis 7
in einem geeigneten Kultivierungsmedium,
b) Kontaktieren der Hefezellen mit dem hydrophoben Substrat, wobei das hydrophobe Substrat mit mindestens einem der Enzyme der Hefezellen, ausgewählt aus der Gruppe,
(i) Cytochrom P450-Genprodukte,
(ii) NADPH-Cytochrom P450-Reduktase,
(iii) Fett-Aldehyd-Dehydrogenasen
zu dem Reaktionsprodukt ω-Hydroxyfettsäure umgesetzt wird,
c) Isolierung der ω-Hydroxyfettsäure.

9. Verfahren nach Anspruch 8, dadurchgekennzeichnet, dass die Umsetzung zu dem Reaktionsprodukt ω-Hydroxyfettsäure zusätzlich mit verbleibenden Fett-Alkohol-Oxidasen, Fett-Alkohol-Dehydrogenasen erfolgt.

10. Verfahren nach einem der Ansprüche 8 oder 9, **dadurch gekennzeichnet, dass** das hydrophobe Substrat ausgewählt ist aus der Gruppe der *n*-Alkane der allgemeinen Struktur: H₃C-(CH₂)ₙ-CH₃, der Alkene der allgemeinen Struktur H₃C-(CₙH₂ₙ-₂)-CH₃, H₃C-(CₙH₂ₙ-₄)-CH₃ oder H₃C-(CₙH₂ₙ-₆)-CH₃, Fettsauren der allgemeinen Struktur: HOOC-(CH₂)n-CH₃, HOOC-(CₙH₂ₙ-₂)-CH₃, HOOC-(CₙH₂ₙ-₄)-CH₃ oder HOOC-(CₙH₂ₙ-₆)-CH₃, und Fettsäurealkylester der allgemeinen Struktur: ROOC-(CH₂)ₙ-CH₃, mit n im Bereich von 6 bis 18.

11. Verfahren nach einem der vorgenannten Ansprüche 8 bis 10, **dadurch gekennzeichnet, dass** zusatzlich zu dem hydrophoben Substrat Glucose als Kohlenstoffquelle verwendet wird.

12. Verfahren nach einem der vorgenannten Ansprüche 8 bis 10, **dadurch gekennzeichnet, dass** das hydrophobe Substrat ein *n*-Alkan, ein Alken oder eine Fettsäure mit n im Bereich 8 bis 18 ist.

13. Kit zur biokatalytischen Herstellung von Dicarbonsäuren oder ω-Hydroxyfettsäuren enthaltend:
- mindestens einen Typ an Hefezellen des Hefestamms der Art *Yarrowia lipolytica* nach einem der Ansprüche 1 bis 7 in einer wässrigen Komponente und/oder
- mindestens einen Typ kryokonservierter Hefezellen des Hefestamms der Art *Yarrowia lipolytica* nach einem der Ansprüche 1 bis 7.

14. Verwendung eines Hefestamms nach einem der Ansprüche 1 bis 7, eines Verfahrens nach einem der Ansprüche 8 bis12 oder eines Kits nach Anspruch 13 zur mikrobiellen Herstellung von ω-Hydroxyfettsäuren gemäß der allgemeinen Struktur: HOOC-(CH₂)n-OH, HOOC-(CₙH₂ₙ-₂)-CH₂OH, HOOC-(CₙH₂ₙ-₄)-CH₂OH oder HOOC-(CₙH₂ₙ-₆)-CH₂OH, mit n im Bereich von 6 bis 18, aus einem hydrophoben Substrat.

## Claims

1. Yeast strain of the species *Yarrowia lipolytica* having a reduction in the activity of Faolp encoded by the gene *FA01: YALIOB14014g,* wherein the yeast strain has furthermore a reduction in the activities of the acyl-CoA oxidases of the genes POX1, POX2, POX3, POX4, POX5 and POX6, wherein the activities of the Fao1p, POX1, POX2, POX3, POX4, POX5 and POX6 proteins are reduced by
(i) partial or complete deletion of the coding genes for Faolp, POX1, POX2, POX3, POX4, POX5 and POX6 or
(ii) expression of non-functional genes for Faolp, POX1, POX2, POX3, POX4, POX5 and POX6 or
(iii) inhibition or reduction of the activity of the expressed genes for Faolp, POX1, POX2, POX3, POX4, POX5 and POX6 by insertion, substitution or point mutation in the gene encoding the protein.

2. Yeast strain according to Claim 1, **characterized in that** the genes for Faolp, *POX1, POX2, POX3, POX4,* POX5 and *POX6* have been deleted.

3. Yeast strain according to either of Claims 1 and 2, **characterized in that** the genes for the FADH, ADH1, ADH2, ADH3, ADH4, ADH5, ADH6 and/or ADH7 fatty alcohol dehydrogenases have been deleted.

4. Yeast strain according to any of Claims 1 to 3, **characterized in that** additionally the activities of some or all cytochrome P450 gene products of *ALK1, ALK2, ALK3, ALK4, ALK5, ALK6, ALK7, ALK8, ALK9, ALK10, ALK11* and/or *ALK12* per cell have been increased, wherein the increased activity of the proteins is achieved by
(i) introduction of the gene of the cytochrome P450 gene product in multiple copies, preferably 8 to 12 copies, into the genome of the organism or
(ii) exchanging of the natural promoter of the gene of the cytochrome P450 gene product for a promoter stronger than the natural one, preferably a promoter having at least 50% of the activity of the promoter of the translation elongation factor 1 alpha gene *TEF1,* most preferably a promoter having at least the activity of the *TEF1* promoter.

5. Yeast strain according to any of Claims 1 to 4, **characterized in that** additionally the activity of the *CPR1* NADPH-cytochrome P450 reductase is increased, wherein the increased activity of the NADPH-cytochrome P450 reductase is achieved by
(i) introduction of the gene of the *CPR1* NADPH-cytochrome P450 reductase in multiple copies, preferably 8 to 12 copies, into the genome of the organism or
(ii) exchanging of the natural promoter of the gene of the *CPR1* NADPH-cytochrome P450 reductase for a promoter stronger than the natural one, preferably a promoter having at least 50% of the activity of the promoter of the translation elongation factor 1 alpha gene *TEF1,* most preferably a promoter having at least the activity of the *TEF1* promoter.

6. Yeast strain according to any of Claims 1 to 5, **characterized in that** additionally the activity of the *PAH1* phosphatidic acid dephosphohydrolase is reduced, wherein the activity of the *PAH1* phosphatidic acid dephosphohydrolase is reduced by
(i) inhibition or reduction of the expression of the coding genes of the *PAH1* phosphatidic acid dephosphohydrolase or
(ii) partial or complete deletion of the coding genes of the *PAH1* phosphatidic acid dephosphohydrolase or
(iii) expression of non-functional genes of the *PAH1* phosphatidic acid dephosphohydrolase or
(iv) inhibition or reduction of the activity of the expressed genes of the *PAH1* phosphatidic acid dephosphohydrolase.

7. Yeast strain according to any of Claims 1 to 6, **characterized in that** additionally the activity of the *SCT1* glycerol-3-phosphate acyltransferase is reduced, wherein the activity of the *SCT1* glycerol-3-phosphate acyltransferase is reduced by
(i) inhibition or reduction of the expression of the coding genes of the SCT1 glycerol-3-phosphate acyltransferase gene or
(ii) partial or complete deletion of the coding genes of the SCT1 glycerol-3-phosphate acyltransferase gene or
(iii) expression of non-functional genes of the SCT1 glycerol-3-phosphate acyltransferase gene or
(iv) inhibition or reduction of the activity of the expressed genes of the SCT1 glycerol-3-phosphate acyltransferase gene.

8. Process for biocatalytically producing ω-hydroxy fatty acids from a hydrophobic substrate, comprising the following steps:
a) providing yeast cells of at least one yeast strain of the species *Yarrowia lipolytica* according to any of Claims 1 to 7 in a suitable cultivation medium,
b) contacting the yeast cells with the hydrophobic substrate, wherein the hydrophobic substrate is converted with at least one of the enzymes of the yeast cells, selected from the group
(i) cytochrome P450 gene products,
(ii) NADPH-cytochrome P450 reductase,
(iii) fatty aldehyde dehydrogenases,
to form the reaction product ω-hydroxy fatty acid,
c) isolating the ω-hydroxy fatty acid.

9. Process according to Claim 8, **characterized in that** the conversion to form the reaction product ω-hydroxy fatty acid is additionally achieved with remaining fatty alcohol oxidases and fatty alcohol dehydrogenases.

10. Process according to either of Claims 8 and 9, **characterized in that** the hydrophobic substrate is selected from the group of n-alkanes of the general structure H₃C-(CH₂)ₙ-CH₃, of alkenes of the general structure H₃C-(CₙH₂ₙ₋₂)-CH₃, H₃C-(CₙH₂ₙ₋₄)-CH₃ or H₃C-(CₙH₂ₙ₋₆)-CH₃, fatty acids of the general structure HOOC-(CH₂)ₙ-CH₃, HOOC-(CₙH₂ₙ₋₂)-CH₃, HOOC- (CₙH₂ₙ₋₄)-CH₃ or HOOC-(CₙH₂ₙ₋₆)-CH₃, and fatty acid alkyl esters of the general structure ROOC-(CH₂)ₙ-CH₃, where n ranges from 6 to 18.

11. Process according to any of aforementioned Claims 8 to 10, **characterized in that** glucose is used as a carbon source in addition to the hydrophobic substrate.

12. Process according to any of aforementioned Claims 8 to 10, **characterized in that** the hydrophobic substrate is an n-alkane, an alkene or a fatty acid where n ranges from 8 to 18.

13. Kit for biocatalytically producing dicarboxylic acids or ω-hydroxy fatty acids, containing:
- at least one type of yeast cells of the yeast strain of the species *Yarrowia lipolytica* according to any of Claims 1 to 7 in an aqueous component and/or
- at least one type of cryopreserved yeast cells of the yeast strain of the species *Yarrowia lipolytica* according to any of Claims 1 to 7.

14. Use of a yeast strain according to any of Claims 1 to 7, of a process according to any of Claims 8 to 12 or of a kit according to Claim 13 for microbial production of ω-hydroxy fatty acids as per the general structure HOOC-(CH₂)ₙ-OH, HOOC-(CₙH₂ₙ₋₂)-CH₂OH, HOOC- (CₙH₂ₙ₋₄)-CH₂OH or HOOC-(CₙH₂ₙ₋₆)-CH₂OH, where n ranges from 6 to 18, from a hydrophobic substrate.

## Revendications

1. Souche de levure de l'espèce *Yarrowia lipolytica* présentant une diminution de l'activité de Fao1p, codée par le gène *FA01 : YALIOB14014g,* la souche de levure présentant en outre une diminution de l'activité des gènes codant pour les acyl-CoA-oxydases POX1, POX2, POX3, POX4, POX5 et POX6, les activités des protéines Fao1p, POX1, POX2, POX3, POX4, POX5 et POX6 étant diminuée par
(i) délétion partielle ou complète des gènes codant pour Fao1p, POX1, POX2, POX3, POX4, POX5 et POX6 ou
(ii) expression des gènes non fonctionnels pour Fao1p POX1, POX2, POX3, POX4, POX5 et POX6 ou
(iii) inhibition ou diminution de l'activité des gènes exprimés pour Fao1p POX1, POX2, POX3, POX4, POX5 et POX6 par insertion, substitution ou mutation ponctuelle dans le gène codant pour la protéine.

2. Souche de levure selon la revendication 1, **caractérisée en ce que** les gènes pour Fao1p, *POX1, POX2, POX3, POX4, POX5* et *POX6* sont délétés.

3. Souche de levure selon l'une des revendications 1 ou 2, **caractérisée en ce que** les gènes codant pour les acide gras-déshydrogénases FADH, ADH1, ADH2, ADH3, ADH4, ADH5, ADH6 et/ou ADH7 sont délétés.

4. Souche de levure selon l'une des revendications 1 à 3, **caractérisée en ce qu'**en outre l'activité de certains ou de la totalité des produits géniques du cytochrome P450 *ALK1, ALK2, ALK3, ALK4, ALK5, ALK6, ALK7, ALK8, ALK9, ALK10, ALK11* et/ou *ALK12* par cellule sont augmentés, l'activité augmentée des protéines étant réalisée par
(i) introduction du gène du produit génique du cytochrome P450 en multicopies, de préférence en 8 à 12 copies, dans le génome de l'organisme, ou
(ii) échange du promoteur naturel du gène du produit génique du cytochrome P450 par un promoteur plus fort que le promoteur naturel, de préférence par un promoteur qui possède au moins 50 % de l'activité du promoteur du gène alpha du facteur d'élongation de traduction 1 *TEF1,* tout spécialement par un promoteur qui possède au moins l'activité du promoteur *TEF1.*

5. Souche de levure selon l'une des revendications 1 à 4, **caractérisée en ce qu'**en outre l'activité de la NADPH-cytochrome P450-réductase *CPR1* est augmentée, l'activité augmentée de la NADPH-cytochrome P450-réductase étant réalisée par
(i) introduction du gène de la NDPH-cytochrome P450-réductase *CPR1* en multicopies, de préférence en 8 à 12 copies, dans le génome de l'organisme, ou
(ii) échange du produit naturel du gène de la NADPH-cytochrome P450-réductase *CPR1* par un promoteur plus fort que le promoteur naturel, de préférence par un promoteur qui possède au moins 50 % de l'activité du promoteur du gène alpha du facteur d'élongation de traduction 1 *TEF1,* tout spécialement par un promoteur qui possède au moins l'activité du promoteur *TEF1;*

6. Souche de levure selon l'une des revendication 1 à 5, **caractérisée en ce qu'**en outre l'activité de l'acide phosphatidique-déphosphohydrolase *PAH1* est diminuée, l'activité de l'acide phosphatidique-déphosphohydrolase *PAH1* étant diminuée par
(i) l'inhibition ou la diminution de l'expression des gènes codants de l'acide phosphatidique-déphosphohydrolase *PAH1* ou
(ii) délétion partielle ou complète des gènes codants de l'acide phosphatidique-déphosphohydrolase *PAH1* ou
(iii) expression de gènes non fonctionnels de l'acide phosphatidique-déphosphohydrolase *PAH1* ou
(iv) inhibition ou diminution de l'activité des gènes exprimés de l'acide phosphatidique-déphosphohydrolase *PAH1.*

7. Souche de levure selon l'une des revendications 1 à 6, **caractérisée en ce qu'**en outre l'activité de la glycérol-3-phosphate-acyltransférase *SCT1* est diminuée, l'activité de la glycérol-3-phosphate-acyltransférase *SCT1* étant diminuée par
(i) l'inhibition ou la diminution de l'expression des gènes codants du gène de la glycérol-3-phosphate-acyltransférase SCT1 ou
(ii) délétion partielle ou complète des gènes codants du gène de la glycérol-3-phosphate-acyltransférase SCT1 ou
(iii) expression de gènes non fonctionnels du gène de la glycérol-3-phosphate-acyltransférase SCT1 ou
(iv) inhibition ou diminution de l'activité des gènes exprimés du gène de la glycérol-3-phosphate-acyltransférase SCT1.

8. Procédé de fabrication biocatalytique d'acides gras ω-hydroxylés à partir d'un substrat hydrophobe, qui comprend les étapes suivantes :
a) fourniture de cellules de levure d'au moins une souche de levure de l'espèce *Yarrowia lipolytica* selon l'une des revendications 1 à 7, dans un milieu de culture approprié,
b) mise en contact des cellules de levure avec un substrat hydrophobe, le substrat hydrophobe étant mis à réagir avec au moins l'une des enzymes des cellules de levure choisies dans le groupe
(i) produits géniques du cytochrome P450,
(ii) NADPH-cytochrome P450-réductase,
(iii) aldéhydes gras-déshydrogénases pour obtenir le produit de réaction acide gras ω-hydroxylé,
c) isolement de l'acide gras ω-hydroxylé.

9. Procédé selon la revendication 8, **caractérisé en ce que** la réaction conduisant au produit de réaction acide gras ω-hydroxylé est réalisée en outre avec des acide gras-oxydases, des alcool gras-déshydrogénase restantes.

10. Procédé selon l'une des revendications 8 ou 9, **caractérisé en ce que** le substrat hydrophobe est choisi dans le groupe des n-alcanes de structure générale : H₃C-(CH₂)ₙ-CH₃, des alcènes de structure générale : H₃C-(CₙH₂ₙ₋₂) -CH₃, H₃C-(CₙH₂ₙ₋₄) -CH₃ ou H₃C-(CₙH₂ₙ₋₆)-CH₃, des acides gras de formule générale : HOOC-(CH₂)ₙ-CH₃, HOOC-CₙH₂ₙ₋₂) -CH₃, HOOC- (CₙH₂ₙ₋₄) -CH₃ ou HOOC- (CₙH₂ₙ₋₆) -CH₃ ou les esters alkyliques d'acides gras de structure : ROOC-(CH₂)ₙ-CH₃, avec n compris dans la plage de 6 à 18.

11. Procédé selon l'une des revendications précédentes 8 à 10, **caractérisé en ce qu'**on utilise, en plus du substrat hydrophobe, du glucose comme source de carbone.

12. Procédé selon l'une des revendications précédentes 8 à 10, **caractérisé en ce que** le substrat hydrophobe est un n-alcane, un alcène ou un acide gras, n étant compris dans la plage de 8 à 18.

13. Kit pour la fabrication biocatalytique d'acides dicarboxyliques ou d'acides gras ω-hydroxylés contenant
- au moins un type de cellules de levure de la souche de levure de l'espèce *Yarrowia lipolytica* selon l'une des revendications 1 à 7 dans un composant aqueux et/ou
- au moins un type de cellules de levure cryoconservées de la souche de levure de l'espèce *Yarrowia lipolytica* selon l'une des revendications 1 à 7.

14. Utilisation d'une souche de levure selon l'une des revendications 1 à 7, d'un procédé selon l'une des revendications 8 à 12 ou d'un kit selon la revendication 13 pour la fabrication d'acides gras ω-hydroxylés ayant la structure générale : HOOC-(CH₂)ₙ-OH, HOOC-(CₙH₂ₙ₋₂) -CH₂OH, HOOC-(CₙH₂ₙ₋₄) -CH₂OH ou HOOC-(CnH₂ₙ₋₆)-CH₂OH, n étant compris dans la plage de 6 à 18, à partir d'un substrat hydrophobe.
